# EUROPEAN PATENT APPLICATION

(11) **EP 4 324 473 A2**
(43) Date of publication of application: **21.02.2024**
(21) Application number: 23206998.9
(22) Date of filing: 04.11.2015
(51) Int. Cl.: A61K 38/02

(54) **MULTIPARAMETRIC NUCLEIC ACID OPTIMIZATION**

(30) Priority: 10.11.2014 US 201462077886 P
(62) Divisional of application: 15859969.6
(71) Applicant: ModernaTX, Inc., Cambridge, MA 02139 (US)
(72) Inventor: REYNDERS III, John van Wicheren, Newton, MA, 02465 (US); CHAKRABORTY, Tirtha, Medford, MA, 02155 (US); HOGE, Stephen G., Brookline, MA, 02446 (US); MCFADYEN, Iain James, Arlington, MA, 02474 (US)
(74) Representative: Hoffmann Eitle

(57) **Abstract**

The present disclosure provides multiparametric codon optimization methods to improve at least a property in a candidate nucleic acid sequence, for example the translation efficacy of a therapeutic mRNA.

## Description

### FIELD OF THE INVENTION

The present disclosure is related to multiparametric methods for designing nucleic acids (e.g., mRNAs) with desired properties, and in particular, synthetic mRNAs with optimized translational efficacy.

### BACKGROUND

Due to the degeneracy of the genetic code, there are numerous different nucleotide sequences that can all encode the same protein. Each amino acid is encoded by up to six synonymous codons; the choice between these codons influences gene expression. In addition, the frequency with which different organisms use codons for expressing a polypeptide sequence differs (codon usage).

Redesigning a naturally occurring gene sequence by choosing different codons without necessarily altering the encoded amino acid sequence often dramatically increases protein expression levels (Gustafsson et al., 2004, "Codon bias and heterologous protein expression," Journal/Trends Biotechnol 22, 346-53). Variables such as codon adaptation index (CAI), mRNA secondary structures, cis-regulatory sequences, GC content and many other similar variables have been shown to somewhat correlate with protein expression levels (Villalobos et al., 2006, "Gene Designer: a synthetic biology tool for constructing artificial DNA segments," Journal/BMC Bioinformatics 7, 285).

Codon optimization is often suggested as a primary consideration for generating high-expressing mRNA constructs suitable for gene therapy and genetic vaccines. Although protein expression can be increased using these approaches, mRNAs contain numerous layers of information that overlap the amino acid code, making conventional codon optimization techniques unsuitable for mRNA optimization in most cases. See, e.g., Mauro & Chappell (2014) Trends in Molecular Medicine 20(11): 604-613. There are potentially serious consequences associated with using codon optimization for nucleic acid therapeutics, e.g., mRNA therapeutics, such as disrupting the normal patterns of tRNA usage, affecting protein structure and function in the target tissue; or producing novel peptides (e.g., truncations) with unknown biological activities.

Currently, there is are no reliable strategies for selecting the codons in synthetic genes to be used as therapeutic agents, in particular synthetic mRNAs, nor is there currently a reliable algorithm with which to assess the likely level of protein expressed in a certain target tissue or cell after the administration of a synthetic mRNA. There is thus a need in the art for both of these.

### BRIEF SUMMARY

The present disclosure provides a multiparametric method for optimizing a candidate nucleic acid sequence (e.g., a wild type nucleic acid sequence, a mutant nucleic acid sequence, a chimeric nucleic sequence, etc. which can be, for example, an mRNA), the method comprising at least one optimization method selected from:
(i) modifying at least one subsequence in the candidate nucleic acid sequence (e.g., an mRNA) to generate a ramp subsequence;
(ii) substituting at least one codon in the candidate nucleic acid sequence with an alternative codon to increase or decrease uridine content to generate a uridine-modified sequence;
(iii) substituting at least one codon in the candidate nucleic acid sequence or the uridine-modified sequence with a fast recharging codon;
(iv) substituting at least one codon in the candidate nucleic acid sequence with an alternative codon having a higher codon frequency in the synonymous codon set;
(v) substituting at least one natural nucleobase in the candidate nucleic acid sequence with an alternative synthetic nucleobase;
(vi) substituting at least one internucleoside linkage in the candidate nucleic acid sequence with a non-natural internucleoside linkage; and,
(vii) combinations thereof.

After such optimization, the resulting optimized nucleic acid sequence has at least one optimized property with respect to the candidate nucleic acid sequence.

In some aspects, the optimized nucleic acid sequence comprises at least one ramp subsequence. In some aspects, a ramp subsequence comprises at least 1, 2, 3, 4, 5, 6, 7, 8, 9, 10, 11, 12, 13, 14, 15, 16, 17, 18, 19 or 20 consecutive codons. In some aspects, the ramp subsequence is located at least 1, 2, 3, 4, 5, 6, 7, 8, 9, 10, 11, 12, 13, 14, 15, 16, 17, 18, 19 or 20 codons from the 5' end of the optimized nucleic acid sequence. In other aspects, the ramp subsequence is a speed-up ramp subsequence. In some aspects, the ramp subsequence is a speed-down ramp subsequence. In some aspects, the optimized nucleic acid sequence comprises at least two ramp subsequences. In other aspects, both ramp subsequences are speed-up ramp subsequences. In some aspects, both ramp subsequences are speed-down ramp subsequences. In some aspects, the optimized nucleic acid sequence comprises a ramp subsequence which is a speed-up ramp subsequence and a ramp subsequence which is a speed-down ramp subsequence.

In some aspects, two ramp subsequences are at least 10, 20, 30, 40, 50, 60, 70, 80, 90 or 100 codons apart in the optimized nucleic acid sequence. In certain aspects, the translation speed of the speed-up ramp subsequence is at least 10% higher than the translation speed of the corresponding subsequence in the candidate nucleic acid sequence. In some aspects, the translation speed of the speed-down ramp subsequence is at least 10% lower than the translation speed of the corresponding subsequence in the candidate nucleic acid sequence. In some aspects, the ramp subsequence is a homologous ramp subsequence. In other aspects, the ramp subsequence is a heterologous ramp subsequence.

In some aspects, the ramp subsequence has a GC content (absolute or relative) at least about 5%, about 10%, about 15%, about 20%, about 25%, about 30%, about 35%, about 40%, about 45%, about 50%, about 55%, about 60%, about 65%, about 70%, about 75%, about 80%, about 85%, about 90%, about 95%, or about 100% higher or lower than the GC content (absolute or relative) of the corresponding subsequence in the candidate nucleic acid sequence. In some aspects, the ramp subsequence has a uridine (U) content (absolute or relative) at least about 5%, about 10%, about 15%, about 20%, about 25%, about 30%, about 35%, about 40%, about 45%, about 50%, about 55%, about 60%, about 65%, about 70%, about 75%, about 80%, about 85%, about 90%, about 95%, or about 100% higher or lower than the uridine (U) content (absolute or relative) of the corresponding subsequence in the candidate nucleic acid sequence.

In some aspects, the protein sequence encoded by the ramp subsequence has an alpha-helical, beta-sheet, or random coil secondary structure. In some aspects, the protein sequence encoded by the ramp subsequence comprises an amino acid sequence with: alpha-helix and beta strand secondary structure; alpha-helix and random coil secondary structure; beta strand and random coil secondary structure; or, alpha-helix, beta strand, and random coil secondary structure.

In some aspects, the codons in the optimized nucleic acid sequences are selected from an optimized codon set. In some aspects, the optimized codon set is a limited codon set. In some aspects, the limited codon set comprises 61, 60, 59, 58, 57, 56, 55, 54, 53, 52, 51, 50, 49, 48, 47, 46, 45, 44, 43, 42, 41, 40, 39, 38, 37, 36, 35, 34, 33, 32, 31, 30, 30, 29, 28, 27, 26, 25, 24, 23, 22, 21, or 20 codons. In some aspects, at least one amino acid selected from the group consisting of Ala, Arg, Asn, Asp, Cys, Gln, Glu, Gly, His, Ile, Leu, Lys, Phe, Pro, Ser, Thr, Tyr, and Val is encoded by a single codon in the limited codon set.

In some aspects, the limited codon set consists of 20 codons, and each codon encodes one of 20 amino acids. In some aspects, the limited codon set comprises at least one codon selected from the group consisting of GCT, GCC, GCA, and GCG; at least a codon selected from the group consisting of CGT, CGC, CGA, CGG, AGA, and AGG; at least a codon selected from AAT or ACC; at least a codon selected from GAT or GAC; at least a codon selected from TGT or TGC; at least a codon selected from CAA or CAG; at least a codon selected from GAA or GAG; at least a codon selected from the group consisting of GGT, GGC, GGA, and GGG; at least a codon selected from CAT or CAC; at least a codon selected from the group consisting of ATT, ATC, and ATA; at least a codon selected from the group consisting of TTA, TTG, CTT, CTC, CTA, and CTG; at least a codon selected from AAA or AAG; an ATG codon; at least a codon selected from TTT or TTC; at least a codon selected from the group consisting of CCT, CCC, CCA, and CCG; at least a codon selected from the group consisting of TCT, TCC, TCA, TCG, AGT, and AGC; at least a codon selected from the group consisting of ACT, ACC, ACA, and ACG; a TGG codon; at least a codon selected from TAT or TAC; and, at least a codon selected from the group consisting of GTT, GTC, GTA, and GTG.

In some aspects, the limited codon set comprises at least one codon selected from the group consisting of GCU, GCC, GCA, and GCG; at least a codon selected from the group consisting of CGU, CGC, CGA, CGG, AGA, and AGG; at least a codon selected from AAU or ACC; at least a codon selected from GAU or GAC; at least a codon selected from UGU or UGC; at least a codon selected from CAA or CAG; at least a codon selected from GAA or GAG; at least a codon selected from the group consisting of GGU, GGC, GGA, and GGG; at least a codon selected from CAU or CAC; at least a codon selected from the group consisting of AUU, AUC, and AUA; at least a codon selected from the group consisting of UUA, UUG, CUU, CUC, CUA, and CUG; at least a codon selected from AAA or AAG; an AUG codon; at least a codon selected from UUU or UUC; at least a codon selected from the group consisting of CCU, CCC, CCA, and CCG; at least a codon selected from the group consisting of UCU, UCC, UCA, UCG, AGU, and AGC; at least a codon selected from the group consisting of ACU, ACC, ACA, and ACG; a UGG codon; at least a codon selected from UAU or UAC; and, at least a codon selected from the group consisting of GUU, GUC, GUA, and GUG.

In some aspects, the limited codon set is:
(a) TTC, TTG, CTG, ATC, ATG, GTG, AGC, CCC, ACC, GCC, TAC, CAC, CAG, AAC, AAG, GAG, TGC, TGG, AGG, GGC;
(b) TTT, CTA, ATA, ATG, GTA, TCG, CCG, ACG, GCG, TAT, CAT, CAA, AAT, AAA, GAT, GAA, TGT, TGG, CGT, GGT;
(c) TTC, CTV, ATM, ATG, GTV, AGC, CCV, ACV, GCV, TAC, CAC, CAR, AAC, AAR, GAC, GAR, TGC, TGG, CGV, GGV; or,
(d) TTC, CTV, ATM, ATG, GTV, AGC, CCV, ACV, GCV, TAC, CAC, CAR, AAC, AAR, GAC, GAR, TGC, TGG, AGR, GGV.

In other aspects, the limited codon set is:
(a) UUC, UUG, CUG, AUC, AUG, GUG, AGC, CCC, ACC, GCC, UAC, CAC, CAG, AAC, AAG, GAG, UGC, UGG, AGG, GGC;
(b) UUU, CUA, AUA, AUG, GUA, UCG, CCG, ACG, GCG, UAU, CAU, CAA, AAU, AAA, GAU, GAA, UGU, UGG, CGU, GGU;
(c) UUC, CUV, AUM, AUG, GUV, AGC, CCV, ACV, GCV, UAC, CAC, CAR, AAC, AAR, GAC, GAR, UGC, UGG, CGV, GGV; or,
(d) UUC, CUV, AUM, AUG, GUV, AGC, CCV, ACV, GCV, UAC, CAC, CAR, AAC, AAR, GAC, GAR, UGC, UGG, AGR, GGV.

In some aspects, the optimized codon set comprises at least one codon encoding an unnatural amino acid. In other aspects, the optimized codon set comprises at least one codon consisting of more than 3 nucleobases. In some aspect, the at least one codon consisting of more than 3 nucleobases consists of 4 or 5 nucleobases.

In some aspects, the optimized codon set comprises at least one codon comprising an unnatural nucleobase.

In some aspects, the uridine-modified sequence induces a lower Toll-Like Receptor (TLR) response when compared to the candidate nucleic acid sequence. In some aspects, the TLR response is mediated by TLR3, TLR7, TLR8, or TLR9. In some aspects, the TLR response is at least 10%, at least 20%, at least 30%, at least 40%, at least 50%, at least 60%, at least 70%, at least 80%, at least 90 or at least 100% lower than the TLR response caused by the candidate nucleic acid sequence.

In some aspects, the uridine content (absolute or relative) of the uridine-modified sequence is higher than the uridine content (absolute or relative) of the candidate nucleic acid sequence. In some aspects, the uridine content (absolute or relative) of the uridine-modified sequence is lower than the uridine content (absolute or relative) of the candidate nucleic acid sequence. In some aspects, the uridine-modified sequence contains at least 5%, 10%, 15%, 20%, 25%, 30%, 35%, 40%, 45% or 50% more uridine that the candidate nucleic acid sequence. In other aspects, the uridine-modified sequence contains at least 5%, 10%, 15%, 20%, 25%, 30%, 35%, 40%, 45% or 50% less uridine than the candidate nucleic acid sequence. In some aspects, the uridine content of the uridine-modified sequence is less than 50%, 49%, 48%, 47%, 46%, 45%, 44%, 43%, 42%, 41%, 40%, 39%, 38%, 37%, 36%, 35%, 34%, 33%, 32%, 31%, 30%, 29%, 28%, 27%, 26%, 25%, 24%, 23%, 22%, 21%, 20%, 19%, 18%, 17%, 16%, 15%, 14%, 13%, 12%, 11%, 10%, 9%, 8%, 7%, 6%, 5%, 4%, 3%, 2% or 1%.

In some aspects, the candidate nucleic acid sequence comprises at least one uridine cluster, wherein said uridine cluster is a subsequence of the candidate nucleic acid sequence, and wherein the percentage of total uridine nucleobases in said subsequence is above or below a predetermined threshold. In some aspects, the length of the subsequence is about 10, 15, 20, 25, 30, 35, 40, 45, 50, 55, 60, 65, 70, 75, 80, 85, 90, 95, or 100 nucleobases. In some aspects, the candidate nucleic acid sequence comprises at least one uridine cluster, wherein said uridine cluster is a subsequence of the candidate nucleic acid sequence, and wherein the percentage of uridine nucleobases in said subsequence as measured using a sliding window is above a predetermined threshold. In some aspects, the length of the sliding window is 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, or 40 nucleobases. In some aspects, the threshold is 1%, 2%, 3%, 4%, 5%, 6%, 7%, 8%, 9%, 10%, 11%, 12%, 13%, 14%, 15%, 16%, 17%, 18%, 19%, 20%, 21%, 22%, 23%, 24% or 25% uridine content.

In some aspects, the candidate nucleic acid sequence comprises at least two uridine clusters. In some aspects, the uridine-modified sequence contains less uridine-rich clusters than the candidate nucleic acid sequence. In some aspects, the uridine-modified sequence contains more uridine-rich clusters than the candidate nucleic acid sequence. In some aspects, the uridine-modified sequence contains uridine-rich clusters with are shorter in length than corresponding uridine-rich clusters in the candidate nucleic acid sequence. In other aspects, the uridine-modified sequence contains uridine-rich clusters which are longer in length that corresponding uridine-rich cluster in the candidate nucleic acid sequence.

In some aspects, the optimized nucleic acid sequence comprises an overall increase in Guanine/Cytosine (G/C) content (absolute or relative) relative to the G/C content (absolute or relative) of the candidate nucleic acid sequence. In other aspects, the overall increase in G/C content (absolute or relative) is by at least 5%, 10%, 15%, 20%, 25%, 30%, 35%, 40%, 45% or 50% relative to the G/C content (absolute or relative) of the candidate nucleic acid sequence. In other aspects, the optimized nucleic acid sequence comprises an overall decrease in Guanine/Cytosine (G/C) content (absolute or relative) relative to the G/C content (absolute or relative) of the candidate nucleic acid sequence. In some aspects, the overall decrease in G/C content (absolute or relative) is by at least about 5%, about 10%, about 15%, about 20%, about 25%, about 30%, about 35%, about 40%, about 45% , about 50%, about 55%, about 60%, about 65%, about 70%, or about 75% relative to the G/C content (absolute or relative) of the candidate nucleic acid sequence.

In some aspects, the optimized nucleic acid sequence comprises a local increase in Guanine/Cytosine (G/C) content (absolute or relative) in a subsequence (G/C modified subsequence) relative to the G/C content (absolute or relative) of the corresponding subsequence in the candidate nucleic acid sequence. In other aspects, the local increase in G/C content (absolute or relative) is by at least about 5%, about 10%, about 15%, about 20%, about 25%, about 30%, about 35%, about 40%, about 45%, about 50%, about 55%, about 60%, about 65%, about 70%, or about 75% relative to the G/C content (absolute or relative) of the candidate nucleic acid sequence. In some aspects, the optimized nucleic acid sequence comprises a local decrease in Guanine/Cytosine (G/C) content (absolute or relative) in a subsequence relative to the G/C content (absolute or relative) of the corresponding subsequence of the candidate nucleic acid sequence. In some aspects, the local decrease in G/C content (absolute or relative) is by at least about 5%, about 10%, about 15%, about 20%, about 25%, about 30%, about 35%, about 40%, about 45%, about 50%, about 55%, about 60%, about 65%, about 70% or about 75% relative to the G/C content (absolute or relative) of the candidate nucleic acid sequence. In some aspects, the length of the subsequence is at least 5, 10, 15, 20, 25, 30, 35, 40, 45, 50, 55, 60, 65, 70, 75, 80, 85, 90, 95, or 100 nucleobases.

In some aspects, the subsequence is located within:
(a) at least 5, 10, 15, 20, 25, 30, 35, 40, 45, 50, 55, 60, 65, 70, 75, 80, 85, 90, 95 or 100 nucleobases from the 5' end of the candidate nucleic acid sequence; or,
(b) a distance from the 5' end of the candidate nucleic acid sequence which is at least about 5%, about 10%, about 15%, about 20%, about 25%, about 30%, about 35%, about 40%, about 45%, about 50%, about 55%, about 60%, about 65%, about 70%, about 75%, about 80%, about 85%, about 90%, or about 95% of the length of the candidate nucleic acid sequence.

In some aspects, the subsequence is located within:
(a) at least 5, 10, 15, 20, 25, 30, 35, 40, 45, 50, 55, 60, 65, 70, 75, 80, 85, 90, 95 or 100 nucleobases from the 3' end of the candidate nucleic acid sequence; or,
(b) a distance from the 3' end of the candidate nucleic acid sequence which is at least about 5%, about 10%, about 15%, about 20%, about 25%, about 30%, about 35%, about 40%, about 45%, about 50%, about 55%, about 60%, about 65%, about 70%, about 75%, about 80%, about 85%, about 90%, or about 95% of the length of the candidate nucleic acid sequence.

In some aspects, the optimized nucleic acid sequence comprises more than one G/C content-modified subsequence wherein the G/C content (absolute or relative) of each G/C content-modified subsequence is increased or decreased with respect to the G/C content (absolute or relative) in a corresponding subsequence of the candidate nucleic acid sequence. In some aspects, the optimized nucleic acid sequence comprises at least 2, 3, 4, 5, 6, 7, 8, 9, 10, 11, 12, 13, 14, 15, 16, 17, 18, 19, 20, 21, 22, 23, 24 or 25 G/C content-modified subsequences. In some aspects, the distance between two G/C content-modified subsequences is at least 5, 10, 15, 20, 25, 30, 35, 40, 45, 50, 55, 60, 65, 70, 75, 80, 85, 90, 95 or 100 nucleobases. In some aspects, the G/C content (absolute or relative) of each G/C content-modified subsequence in the optimized nucleic acid sequence is increased with respect to the G/C content (absolute or relative) in a corresponding subsequence of the candidate nucleic acid sequence. In some aspects, the G/C content (absolute or relative) of each G/C content-modified subsequence in the optimized nucleic acid sequence is decreased with respect to the G/C content (absolute or relative) in a corresponding subsequence of the candidate nucleic acid sequence.

In some aspects, at least about 5%, at least about 10%, at least about 15%, at least about 20%, at least about 25%, at least about 30%, at least about 35%, at least about 40%, at least about 45%, at least about 50%, at least about 55%, at least about 60%, at least about 65%, at least about 70%, at least about 75%, at least about 80%, at least about 85%, at least about 90%, at least about 95%, at least about 99%, or 100% of the codons in the candidate nucleic acid sequence are substituted with alternative codons, each alternative codon having a codon frequency higher than the codon frequency of the substituted codon in the synonymous codon set.

In some aspects, at least one codon in the candidate nucleic acid sequence is substituted with an alternative codon having a codon frequency higher than the codon frequency of the substituted codon in the synonymous codon set, and at least one codon in the candidate nucleic acid sequence is substituted with an alternative codon having a codon frequency lower than the codon frequency of the substituted codon in the synonymous codon set.

In some aspects, at least about 5%, at least about 10%, at least about 15%, at least about 20%, at least about 25%, at least about 30%, at least about 35%, at least about 40%, at least about 45%, at least about 50%, at least about 55%, at least about 60%, at least about 65%, at least about 70%, or at least about 75% of the codons in the candidate nucleic acid sequence are substituted with alternative codons, each alternative codon having a codon frequency higher than the codon frequency of the substituted codon in the synonymous codon set. In some aspects, at least one alternative codon having a higher codon frequency has the highest codon frequency in the synonymous codon set. In other aspects, all alternative codons having a higher codon frequency have the highest codon frequency in the synonymous codon set.

In some aspects, at least one alternative codon having a lower codon frequency has the lowest codon frequency in the synonymous codon set. In some aspects, all alternative codons having a lower codon frequency have the lowest codon frequency in the synonymous codon set. In some specific aspects, at least one alternative codon has a second highest, the third highest, the fourth highest, the fifth highest or the sixth highest frequency in the synonymous codon set. In some specific aspects, at least one alternative codon has the second lowest, the third lowest, the fourth lowest, the fifth lowest, or the sixth lowest frequency in the synonymous codon set.

In some aspects, at least about 5%, at least about 10%, at least about 15%, at least about 20%, at least about 25%, at least about 30%, at least about 35%, at least about 40%, at least about 45%, at least about 50%, at least about 55%, at least about 60%, at least about 65%, at least about 70%, at least about 75%, at least about 80%, at least about 85%, at least about 90%, at least about 95%, at least about 99%, or 100% of the codons in the candidate nucleic acid sequence are substituted with alternative codons having faster recharging rates.

In some aspects, at least one codon in the candidate nucleic acid sequence is substituted with an alternative codon having a faster recharging rate, and at least one codon in the candidate nucleic acid sequence is substituted with an alternative codon having a slower recharging rate. In other aspects, at least about 5%, at least about 10%, at least about 15%, at least about 20%, at least about 25%, at least about 30%, at least about 35%, at least about 40%, at least about 45%, at least about 50%, at least about 55%, at least about 60%, at least about 65%, at least about 70%, or at least about 75% of the codons in the candidate nucleic acid sequence are substituted with alternative codons, each codon having a having a slower recharging rate.

In some aspects, at least one alternative codon having a faster recharging rate has the fastest recharging rate. In some aspects, all alternative codons having a faster recharging rate have the fastest recharging rate. In some aspects, at least one alternative codon having a slower recharging rate has the slowest recharging rate. In some aspects, all alternative codons having a slower recharging rate have the slowest recharging rate.

In some aspects, the multiparametric nucleic acid optimization method comprises one optimization method selected from the group consisting of (i) modifying at least one subsequence in the candidate nucleic acid sequence (e.g., an mRNA) to generate a ramp subsequence; (ii) substituting at least one codon in the candidate nucleic acid sequence with an alternative codon to increase or decrease uridine content to generate a uridine-modified sequence; (iii) substituting at least one codon in the candidate nucleic acid sequence or the uridine-modified sequence with a fast recharging codon; (iv) substituting at least one codon in the candidate nucleic acid sequence with an alternative codon having a higher codon frequency in the synonymous codon set; (v) substituting at least one natural nucleobase in the candidate nucleic acid sequence with an alternative synthetic nucleobase; and (vi) substituting at least one internucleoside linkage in the candidate nucleic acid sequence with a non-natural internucleoside linkage.

In other aspects, the multiparametric nucleic acid optimization method comprises two optimization methods selected from the group consisting of (i) modifying at least one subsequence in the candidate nucleic acid sequence (e.g., an mRNA) to generate a ramp subsequence; (ii) substituting at least one codon in the candidate nucleic acid sequence with an alternative codon to increase or decrease uridine content to generate a uridine-modified sequence; (iii) substituting at least one codon in the candidate nucleic acid sequence or the uridine-modified sequence with a fast recharging codon; (iv) substituting at least one codon in the candidate nucleic acid sequence with an alternative codon having a higher codon frequency in the synonymous codon set; (v) substituting at least one natural nucleobase in the candidate nucleic acid sequence with an alternative synthetic nucleobase; and (vi) substituting at least one internucleoside linkage in the candidate nucleic acid sequence with a non-natural internucleoside linkage.

In other aspects, the multiparametric nucleic acid optimization method comprises three optimization methods selected from the group consisting of (i) modifying at least one subsequence in the candidate nucleic acid sequence (e.g., an mRNA) to generate a ramp subsequence; (ii) substituting at least one codon in the candidate nucleic acid sequence with an alternative codon to increase or decrease uridine content to generate a uridine-modified sequence; (iii) substituting at least one codon in the candidate nucleic acid sequence or the uridine-modified sequence with a fast recharging codon; (iv) substituting at least one codon in the candidate nucleic acid sequence with an alternative codon having a higher codon frequency in the synonymous codon set; (v) substituting at least one natural nucleobase in the candidate nucleic acid sequence with an alternative synthetic nucleobase; and (vi) substituting at least one internucleoside linkage in the candidate nucleic acid sequence with a non-natural internucleoside linkage.

In some aspects, the multiparametric nucleic acid optimization method comprises four optimization methods selected from the group consisting of (i) modifying at least one subsequence in the candidate nucleic acid sequence (e.g., an mRNA) to generate a ramp subsequence; (ii) substituting at least one codon in the candidate nucleic acid sequence with an alternative codon to increase or decrease uridine content to generate a uridine-modified sequence; (iii) substituting at least one codon in the candidate nucleic acid sequence or the uridine-modified sequence with a fast recharging codon; (iv) substituting at least one codon in the candidate nucleic acid sequence with an alternative codon having a higher codon frequency in the synonymous codon set; (v) substituting at least one natural nucleobase in the candidate nucleic acid sequence with an alternative synthetic nucleobase; and (vi) substituting at least one internucleoside linkage in the candidate nucleic acid sequence with a non-natural internucleoside linkage.

In some aspects, the multiparametric nucleic acid optimization method comprises five optimization methods selected from the group consisting of (i) modifying at least one subsequence in the candidate nucleic acid sequence (e.g., an mRNA) to generate a ramp subsequence; (ii) substituting at least one codon in the candidate nucleic acid sequence with an alternative codon to increase or decrease uridine content to generate a uridine-modified sequence; (iii) substituting at least one codon in the candidate nucleic acid sequence or the uridine-modified sequence with a fast recharging codon; (iv) substituting at least one codon in the candidate nucleic acid sequence with an alternative codon having a higher codon frequency in the synonymous codon set; (v) substituting at least one natural nucleobase in the candidate nucleic acid sequence with an alternative synthetic nucleobase; and (vi) substituting at least one internucleoside linkage in the candidate nucleic acid sequence with a non-natural internucleoside linkage.

In some aspects, the multiparametric nucleic acid optimization method comprises six optimization methods selected from the group consisting of (i) modifying at least one subsequence in the candidate nucleic acid sequence (e.g., an mRNA) to generate a ramp subsequence; (ii) substituting at least one codon in the candidate nucleic acid sequence with an alternative codon to increase or decrease uridine content to generate a uridine-modified sequence; (iii) substituting at least one codon in the candidate nucleic acid sequence or the uridine-modified sequence with a fast recharging codon; (iv) substituting at least one codon in the candidate nucleic acid sequence with an alternative codon having a higher codon frequency in the synonymous codon set; (v) substituting at least one natural nucleobase in the candidate nucleic acid sequence with an alternative synthetic nucleobase; and (vi) substituting at least one internucleoside linkage in the candidate nucleic acid sequence with a non-natural internucleoside linkage.

In particular aspects, the multiparametric nucleic acid optimization method comprises 7, 8, 9, 10, 11, 12, 13, 14, 15, 16, 17, 18, 19 or 20 optimization methods. In some aspects, the multiparametric optimization method comprises more than 20 optimization methods. In some aspects, the optimization methods are executed sequentially. In some aspects, the optimization methods are executed concurrently. In some aspects, the optimization methods are executed recursively.

In some aspects, at least 5%, at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 85%, at least 90%, at least 95%, at least 96%, at least 97%, at least 98%, at least 99% or 100% of the codons in the candidate nucleic acid sequence are replaced.

The disclosure also provides a method for expressing a protein in a target tissue or cell or an *in vitro* translation system, the method comprising:
(a) obtaining an optimized gene sequence (e.g., an optimized mRNA sequence) for expression in a mammal in vivo, in particular, in a human, for example, systemically or in a target tissue or target cell, using a multiparametric optimization method disclosed herein;
(b) synthesizing a nucleic acid molecule comprising the optimized gene sequence (e.g., a synthetic mRNA);
(c) introducing the nucleic acid molecule into the target tissue or cell or combining it with the *in vitro* translation system.

In some aspects of the methods disclosed herein, at least one property is optimized in the optimized nucleic acid sequence with respect to the candidate nucleic acid sequence resulting, for example, in (i) an increase in transcription efficacy; (ii) an increase in translation efficacy; (iii) an increase in nucleic acid (DNA or RNA) in vivo half-life; (iv) an increase in nucleic acid (DNA or RNA) in vitro half-life; (v) a decrease in nucleic acid (DNA or RNA) in vivo half-life; (vi) a decrease in nucleic acid (DNA or RNA) in vitro half-life; (vii) an increase in expressed protein yield; (viii) an increase in expressed protein quality; (ix) an increase in nucleic acid (DNA or RNA) structural stability; (x) an increase in viability of cells expressing the optimized nucleic acid sequence; or (xi) combinations thereof.

The present disclosure also provides a computer implemented multiparametric codon optimization method comprising:
(a) inputting at least one candidate nucleic acid sequence (e.g., an mRNA);
(b) applying a multiparametric codon optimization method disclosed herein to the candidate nucleic acid sequence; and,
(c) outputting at least one optimized nucleic acid sequence.

In some aspects of the computer implemented method disclosed herein, at least one optimized nucleic acid sequence (e.g., an mRNA) outputted in step (c) is used an inputting sequence in step (a). In some aspect, method is executed recursively for at least 2, 3, 4, 5, 6, 7, 8, 9 or 10 cycles. In other aspects, the method is executed recursively for at least 10, at least 20, at least 30, at least 40, at least 50, at least 60, at least 70, at least 80, at least 90, or at least 100 cycles. In some aspects, the method is executed recursively for at least 200, at least 300, at least 400, at least 500, at least 600, at least 700, at least 800, at least 900, or at least 1000 cycles. In some aspects, the method is executed recursively for at least 2000, at least 3000, at least 4000, at least 5000, at least 6000, at least 7000, at least 8000, at least 9000, or at least 10000 cycles. In some aspects, the method further comprises submitting electronically the optimized nucleic acid sequence to an automated nucleic acid synthesizer.

In some aspects, a library of candidate nucleic acid sequences (e.g., mRNAs) is used as input in step (a). In some aspects, the output of step (c) is a library of optimized nucleic acid sequences. In some aspects, the multiparametric codon optimization method of step (b) is implemented as a swarm algorithm. In other aspects, the multiparametric codon optimization method of step (b) is implemented as a multi-swarm algorithm. In some aspects, the multiparametric codon optimization method of step (b) is implemented as a Bayesian optimization algorithm. In some aspects, the multiparametric codon optimization method of step (b) is implemented as a combinatorial optimization algorithm. In some aspects, the multiparametric codon optimization method of step (b) is implemented as a genetic algorithm. In some aspects, the genetic algorithm is an implementation in parallel of a genetic algorithm. In some aspects, the parallel implementation of the genetic algorithms is a coarse-grained parallel genetic algorithm. In some aspects, the parallel implementation of the genetic algorithms is a fine-grained parallel genetic algorithm. In some aspects, the genetic algorithm comprises adaptive parameters.

The present disclosure also provides an isolated nucleic acid molecule or a complement thereof (e.g., an mRNA) encoding a protein optimized according to any of the multiparametric codon optimization methods disclosed herein. In some aspects, the isolated nucleic acid molecule is DNA. In other aspects, the isolated nucleic acid molecule is RNA. In some aspects, the RNA is mRNA. In some aspects, mRNA is a therapeutic mRNA. In some aspects, the mRNA is a synthetic mRNA. In some aspects, the isolated nucleic acid molecule comprises at least one nucleotide analogue. In some aspects, the at least one nucleotide analogue is selected from the group consisting of a 2'-O-methoxyethyl-RNA (2'-MOE-RNA) monomer, a 2'-fluoro-DNA monomer, a 2'-O-alkyl-RNA monomer, a 2'-amino-DNA monomer, a locked nucleic acid (LNA) monomer, a cEt monomer, a cMOE monomer, a 5'-Me-LNA monomer, a 2'-(3-hydroxy)propyl-RNA monomer, an arabino nucleic acid (ANA) monomer, a 2'-fluoro-ANA monomer, an anhydrohexitol nucleic acid (HNA) monomer, an intercalating nucleic acid (INA) monomer, and a combination of two or more of said nucleotide analogues. In some aspects, the isolated nucleic acid molecule comprises at least one backbone modification. In some aspects, at least one backbone modification is a phosphorothioate internucleotide linkage. In some aspects, all of the internucleotide linkages are phosphorothioate internucleotide linkages.

In some aspects, the isolated nucleic acid molecule (e.g., an mRNA) comprises pseudouridine, 5-methoxyuridine, 2-thiouridine, 4-thiouridine, N1-methylpseudouridine, 5-aza-uridine, 2-thio-5-aza-uridine, 4-thio-pseudouridine, 2-thio-pseudouridine, 5-hydroxyuridine, 4-methoxy-pseudouridine, 4-methoxy-2-thio-pseudouridine, 3-methyluridine, 5-carboxymethyl-uridine, 1-carboxymethyl-pseudouridine, 5-propynyl-uridine, 1-propynyl-pseudouridine, 2-methoxy-4-thio-uridine, 5-taurinomethyluridine, 1-taurinomethyl-pseudouridine, 5-taurinomethyl-2-thio-uridine, 1-taurinomethyl-4-thiouridine, 5-methyl-uridine, 2-methoxyuridine, 1-methyl-pseudouridine, 4-thio-1-methyl-pseudouridine, 2-thio-1-methyl-pseudouridine, 1-methyl-1-deaza-pseudouridine, 2-thio-1-methyl-1-deaza-pseudouridine, or 2-thio-dihydrouridine.

In some aspects, the isolated nucleic acid molecule (e.g., an mRNA) comprises of 2-aminopurine, 2,6-diaminopurine, 7-deaza-adenine, 7-deaza-8-aza-adenine, 7-deaza-2-aminopurine, 7-deaza-8-aza-2-aminopurine, 7-deaza-2,6-diaminopurine, 7-deaza-8-aza-2,6-diaminopurine, 1-methyladenosine, N6-methyladenosine, N6-isopentenyladenosine, N6-(cis-hydroxyisopentenyl)adenosine, 2-methylthio-N6-(cis-hydroxyisopentenyl)adenosine, N6-glycinylcarbamoyladenosine, N6-threonylcarbamoyladenosine, 2-methylthio-N6-threonyl carbamoyladenosine, N6,N6-dimethyladenosine, or 7-methyladenine.

In some aspects, the isolated nucleic acid molecule (e.g., an mRNA) comprises inosine, 1-methyl-inosine, wyosine, wybutosine, 7-deaza-guanosine, 7-deaza-8-aza-guanosine, 6-thio-guanosine, 6-thio-7-deaza-guanosine, 6-thio-7-deaza-8-aza-guanosine, 7-methyl-guanosine, 6-thio-7-methyl-guanosine, 7-methylinosine, 6-methoxy-guanosine, 1-methylguanosine, N2-methylguanosine, N2,N2-dimethylguanosine, 8-oxo-guanosine, 7-methyl-8-oxo-guanosine, or 1-methyl-6-thio-guanosine.

In some aspects, the isolated nucleic acid molecule (e.g., an mRNA) comprises 5-methylcytidine, 5-aza-cytidine, pseudoisocytidine, 3-methyl-cytidine, N4-acetylcytidine, 5-formylcytidine, N4-methylcytidine, 5-hydroxymethylcytidine, 1-methyl-pseudoisocytidine, pyrrolo-cytidine, pyrrolo-pseudoisocytidine, 2-thio-cytidine, 2-thio-5-methyl-cytidine, 4-thio-pseudoisocytidine, 4-thio-1-methyl-pseudoisocytidine, 4-thio-1-methyl-1-deaza-pseudoisocytidine, 1-methyl-1-deaza-pseudoisocytidine, zebularine, 5-aza-zebularine, 5-methyl-zebularine, 5-aza-2-thio-zebularine, 2-thio-zebularine, 2-methoxy-cytidine, 2-methoxy-5-methyl-cytidine, 4-methoxy-pseudoisocytidine, or 4-methoxy-1-methyl-pseudoisocytidine.

In some aspects, at least one uridine has been replaced with pseudouridine, 5-methoxyuridine, 2-thiouridine, 4-thiouridine, N1-methylpseudouridine, or 5-aza-uridine.

In other aspects, at least one uridine has been replaced with 2-thio-5-aza-uridine, 4-thio-pseudouridine, 2-thio-pseudouridine, 5-hydroxyuridine, 4-methoxy-pseudouridine, or 4-methoxy-2-thio-pseudouridine.

In other aspects, at least one uridine has been replaced with 3-methyluridine, 5-carboxymethyl-uridine, 1-carboxymethyl-pseudouridine, 5-propynyl-uridine, 1-propynyl-pseudouridine, or 2-methoxy-4-thio-uridine.

In other aspects, at least one uridine has been replaced with 5-taurinomethyluridine, 1-taurinomethyl-pseudouridine, 5-taurinomethyl-2-thio-uridine, 1-taurinomethyl-4-thiouridine, 5-methyl-uridine, or 2-methoxyuridine.

In other aspects, at least one uridine has been replaced with 1-methyl-pseudouridine, 4-thio-1 -methyl-pseudouridine, 2-thio-1 -methyl-pseudouridine, 1-methyl-1-deaza-pseudouridine, 2-thio-1-methyl-1-deaza-pseudouridine, or 2-thio-dihydrouridine.

In other aspects, at least one adenosine has been replaced with 2-aminopurine, 2,6-diaminopurine, 7-deaza-adenine, 7-deaza-8-aza-adenine, 7-deaza-2-aminopurine, or 7-deaza-8-aza-2-aminopurine.

In other aspects, at least one adenosine has been replaced with 7-deaza-2,6-diaminopurine, 7-deaza-8-aza-2,6-diaminopurine, 1-methyladenosine, N6-methyladenosine, N6-isopentenyladenosine, or N6-(cis-hydroxyisopentenyl)adenosine.

In other aspects, at least one adenosine has been replaced with 2-methylthio-N6-(cis-hydroxyisopentenyl) adenosine, N6-glycinylcarbamoyladenosine, N6-threonylcarbamoyladenosine, 2-methylthio-N6-threonyl carbamoyladenosine, N6,N6-dimethyladenosine, or 7-methyladenine.

In other aspects, at least one guanosine has been replaced with inosine, 1-methyl-inosine, wyosine, wybutosine, 7-deaza-guanosine, 7-deaza-8-aza-guanosine, or 6-thio-guanosine.

In other aspects, at least one guanosine has been replaced with 6-thio-7-deaza-guanosine, 6-thio-7-deaza-8-aza-guanosine, 7-methyl-guanosine, 6-thio-7-methyl-guanosine, 7-methylinosine, or 6-methoxy-guanosine.

In other aspects, at least one guanosine has been replaced with 1-methylguanosine, N2-methylguanosine, N2,N2-dimethylguanosine, 8-oxo-guanosine, 7-methyl-8-oxo-guanosine, or 1-methyl-6-thio-guanosine.

In other aspects, at least one cytidine has been replaced with 5-methylcytidine, 5-aza-cytidine, pseudoisocytidine, 3-methyl-cytidine, N4-acetylcytidine, or 5-formylcytidine.

In other aspects, at least one cytidine has been replaced with N4-methylcytidine, 5-hydroxymethylcytidine, 1-methyl-pseudoisocytidine, pyrrolo-cytidine, pyrrolo-pseudoisocytidine, or 2-thio-cytidine.

In other aspects, at least one cytidine has been replaced with 2-thio-5-methyl-cytidine, 4-thio-pseudoisocytidine, 4-thio-1-methyl-pseudoisocytidine, 4-thio-1-methyl-1-deaza-pseudoisocytidine, 1-methyl-1-deaza-pseudoisocytidine, or zebularine.

In other aspects, at least one cytidine has been replaced with 5-aza-zebularine, 5-methyl-zebularine, 5-aza-2-thio-zebularine, 2-thio-zebularine, 2-methoxy-cytidine, or 2-methoxy-5-methyl-cytidine.

In some aspects, at least one cytidine has been replaced with replaced with 5-methylcytidine, 5-aza-cytidine, pseudoisocytidine, 3-methyl-cytidine, N4-acetylcytidine, or 5-formylcytidine. In some aspects, at least 25%, at least 50%, at least 75% or at least 100% of cytidines have been replaced with replaced with 5-methylcytidine, 5-aza-cytidine, pseudoisocytidine, 3-methyl-cytidine, N4-acetylcytidine, or 5-formylcytidine. In some aspects, at least 25%, at least 50%, at least 75% or at least 100% of uridines have been replaced with pseudouridine. In some aspects, at least 25%, at least 50%, at least 75% or at least 100% of uridines have been replaced with 2-thiouridine. In other aspects, at least 25%, at least 50%, at least 75% or at least 100% of uridines have been replaced with 4-thiouridine. In some aspects, at least 25%, at least 50%, at least 75% or at least 100% of uridines have been replaced with N1-methylpseudouridine.

In some aspects, 100% of the uridine nucleosides in the isolated nucleic acid molecule (e.g., an mRNA) have been replaced with a nucleoside selected from the group consisting of pseudouridine, 5-methoxyuridine, 2-thiouridine, 4-thiouridine, N1-methylpseudouridine, 5-aza-uridine, 2-thio-5-aza-uridine, 4-thio-pseudouridine, 2-thio-pseudouridine, 5-hydroxyuridine, 4-methoxy-pseudouridine, 4-methoxy-2-thio-pseudouridine, 3-methyluridine, 5-carboxymethyl-uridine, 1-carboxymethyl-pseudouridine, 5-propynyl-uridine, 1-propynyl-pseudouridine, 2-methoxy-4-thio-uridine, 5-taurinomethyluridine, 1-taurinomethyl-pseudouridine, 5-taurinomethyl-2-thio-uridine, 1-taurinomethyl-4-thio-uridine, 5-methyl-uridine, 2-methoxyuridine, 1-methyl-pseudouridine, 4-thio-1-methyl-pseudouridine, 2-thio-1-methyl-pseudouridine, 1-methyl-1-deaza-pseudouridine, 2-thio-1-methyl-1-deaza-pseudouridine, and 2-thio-dihydrouridine.

In some aspects, 100% of the adenosine nucleosides in the isolated nucleic acid molecule (e.g., an mRNA) have been replaced with a nucleoside selected from the group consisting of 2-aminopurine, 2,6-diaminopurine, 7-deaza-adenine, 7-deaza-8-aza-adenine, 7-deaza-2-aminopurine, 7-deaza-8-aza-2-aminopurine, 7-deaza-2,6-diaminopurine, 7-deaza-8-aza-2,6-diaminopurine, 1-methyladenosine, N6-methyladenosine, N6-isopentenyladenosine, N6-(cis-hydroxyisopentenyl)adenosine, 2-methylthio-N6-(cis-hydroxyisopentenyl)adenosine, N6-glycinylcarbamoyladenosine, N6-threonylcarbamoyladenosine, 2-methylthio-N6-threonyl carbamoyladenosine, N6,N6-dimethyladenosine, and 7-methyladenine.

In some aspects, 100% of guanosine nucleosides in the isolated nucleic acid molecule (e.g., an mRNA) have been replaced with a nucleoside selected from the group consisting of inosine, 1-methyl-inosine, wyosine, wybutosine, 7-deaza-guanosine, 7-deaza-8-aza-guanosine, 6-thio-guanosine, 6-thio-7-deaza-guanosine, 6-thio-7-deaza-8-aza-guanosine, 7-methyl-guanosine, 6-thio-7-methyl-guanosine, 7-methylinosine, 6-methoxy-guanosine, 1-methylguanosine, N2-methylguanosine, N2,N2-dimethylguanosine, 8-oxo-guanosine, 7-methyl-8-oxo-guanosine, and 1-methyl-6-thio-guanosine.

In some aspects, 100% of cytidine nucleosides in the isolated nucleic acid molecule (e.g., an mRNA) have been replaced with a nucleoside selected from the group consisting of 5-methylcytidine, 5-aza-cytidine, pseudoisocytidine, 3-methyl-cytidine, N4-acetylcytidine, 5-formylcytidine, N4-methylcytidine, 5-hydroxymethylcytidine, 1-methyl-pseudoisocytidine, pyrrolo-cytidine, pyrrolo-pseudoisocytidine, 2-thio-cytidine, 2-thio-5-methyl-cytidine, 4-thio-pseudoisocytidine, 4-thio-1-methyl-pseudoisocytidine, 4-thio-1-methyl-1-deaza-pseudoisocytidine, 1-methyl-1-deaza-pseudoisocytidine, zebularine, 5-aza-zebularine, 5-methyl-zebularine, 5-aza-2-thio-zebularine, 2-thio-zebularine, 2-methoxy-cytidine, 2-methoxy-5-methyl-cytidine, 4-methoxy-pseudoisocytidine, and 4-methoxy-1-methyl-pseudoisocytidine.

The present disclosure also provides vector or set of vectors comprising the optimized nucleic acid molecule (e.g., an mRNA) or set of optimized nucleic acid molecules prepared according to the multiparametric codon optimization methods disclosed herein.

The present disclosure also provides a method for producing a protein encoded by an optimized nucleic acid molecule (e.g., an mRNA) prepared according to the multiparametric codon optimization methods disclosed herein comprising contacting a target tissue or cell with an optimized nucleic acid molecule disclosed herein (e.g., a synthetic mRNA).

Also provided is method for producing a protein encoded by an optimized nucleic acid molecule (e.g., an mRNA) prepared according to the multiparametric codon optimization methods disclosed herein, wherein the expression is conducted using an *in vitro* translation system.

Also provided is pharmaceutical composition comprising an optimized nucleic acid molecule (e.g., an mRNA), or a vector comprising said optimized nucleic acid, and a pharmaceutically acceptable vehicle or excipient, wherein said optimized nucleic acid has been prepared according to the multiparametric codon optimization methods disclosed herein..

### BRIEF DESCRIPTION OF THE DRAWINGS/FIGURES

FIG. 1A shows the amino acid sequence and secondary structure of Apolipoprotein A-1 (ApoA1).
FIG. 1B shows the distribution of codons in ApoA1 according to order of codon frequency. Each amino acid and number of synonymous codons is indicated (i.e., A(4) indicates that 4 synonymous codon encode alanine).

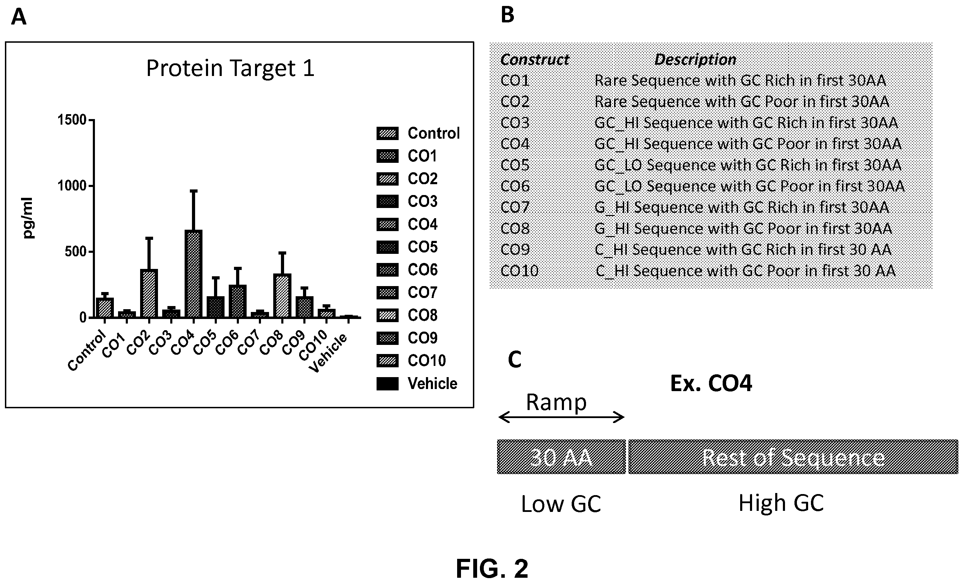

FIG. 2 shows the expression levels corresponding to 10 different synthetic mRNA constructs (CO1 to CO10) for a protein target (Target Protein 1) generated using different codon sets (e.g., GC_HI is a codon set rich in GC, GC_LO is a codon set with low GC, G_HI is a codon set with only high G, C_HI is a codon set with only high C), wherein the composition of the codons for the first 30 amino acids (a "ramp") has been biased by selecting codons with high GC or low GC content. The topology of an exemplary construct (CO4) is shown, indicating the presence of a 30 aa (i.e., 30 codon) ramp located the 5' end of the construct, whereas the rest of the construct is encoded by an optimized codon set with a high G/C content bias.
FIG. 3A shows the expression levels corresponding to the 10 different constructs presented in FIG. 2, but applying a specific chemistry (Chemi) to the generation of the synthetic mRNA for Target Protein 1.
FIG. 3B shows the expression levels corresponding to 10 constructs generated using the same strategy used in FIG. 2, but applied to a different target protein (Target Protein 2). The mRNAs in FIG. 3B were generated using the same chemistry used in FIG. 3A (Chemi).
FIG. 3C shows the expression levels corresponding to 10 constructs generated using the same strategy used in FIG. 2, but applied to a different target protein (Target Protein 3). The mRNAs in FIG. 3B were generated using the same chemistry used in FIG. 3A (Chemi).
FIG. 4 illustrates the correlation between G/C content and codon frequency. In each synonymous group, the codon with the highest frequency is highlighted. 19 out of 20 of the highest frequency codons also are highest G/C-content codons in each group. 15 out of 20 lowest frequency codons are also one of the lowest G/C-content codons in each group.

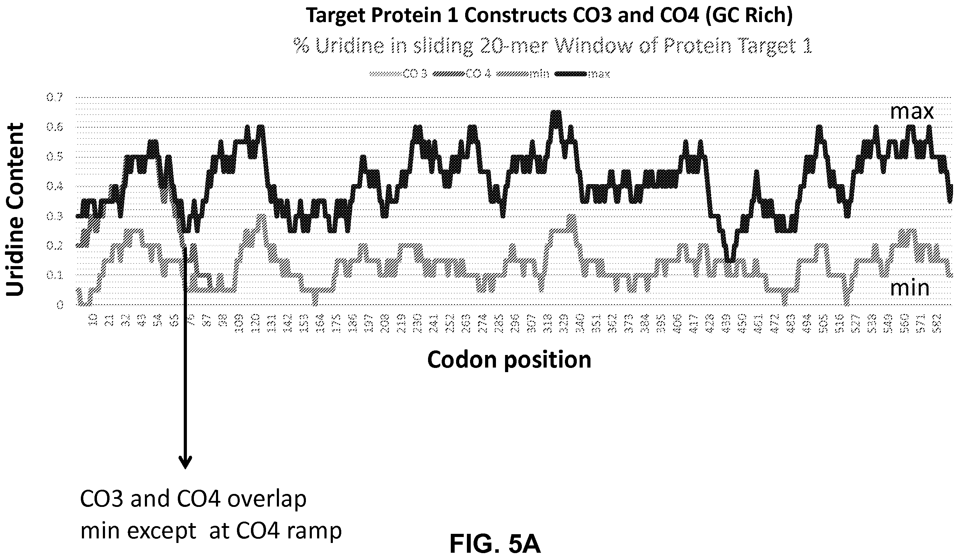

FIG. 5A shows the uridine distribution in a target protein selected for optimization (Target Protein 1) illustrating the differences between the CO3 and CO4 constructs, both G/C rich, and how the selection of G/C rich codon sets correlates with low uridine content. The representation indicates the theoretical maximum (max) and theoretical minimum (min) uridine content for the target protein. The CO4 construct contains a 5'-end uridine ramp, where uridine frequency is closest to the maximum possible uridine content for that region, and uridine content for the rest of the construct corresponds to the lowest possible uridine content for that region of the target protein. The uridine content profile for CO4 overlaps with lowest possible uridine content profile for the target protein.
FIG. 5B shows the uridine distribution in a target protein selected for optimization (Target Protein 1) illustrating the differences between the CO5 and CO6 constructs, both G/C poor, and how the selection of G/C poor codon sets correlates with high uridine content. The representation indicates the theoretical maximum (max) and theoretical minimum (min) uridine content for the target protein. Uridine content for both CO5 and CO6 constructs is close to the highest possible uridine content for the target protein.
FIG. 6A shows the amino acid prevalence in luciferase.
FIG. 6B shows codon bias in 50 orthogonal unbiased codon maps generated via machine learning (normal) and 50 uridine-biased codon maps subsampling of 200 codon maps and their luciferase uridine 2-mer max and AUC measures.
FIG. 7 shows a codon frequency map highlighting the selection of low frequency codons to generate a low uridine content ramp (panel A). Each set of information shows "amino acid, codon frequency, codon." Codons are highlighted to indicate whether the lowest uridine content codon has the lowest frequency, or second lowest frequency. The exception is UCG (gly), which despite having the lowest frequency still contains uridine. Panel B present a 30aa ramp sequence from luciferase color coding the amino acids according to frequency/uridine content.
FIG. 8A shows the uridine distribution in luciferase ramps generated using HI-GC and LO-GC codon maps.
FIG. 8B shows the uridine distribution in luciferase ramp generated using the uridine sensitive approach presented in FIG. 7. The ramp matches the minimum uridine distribution curve.

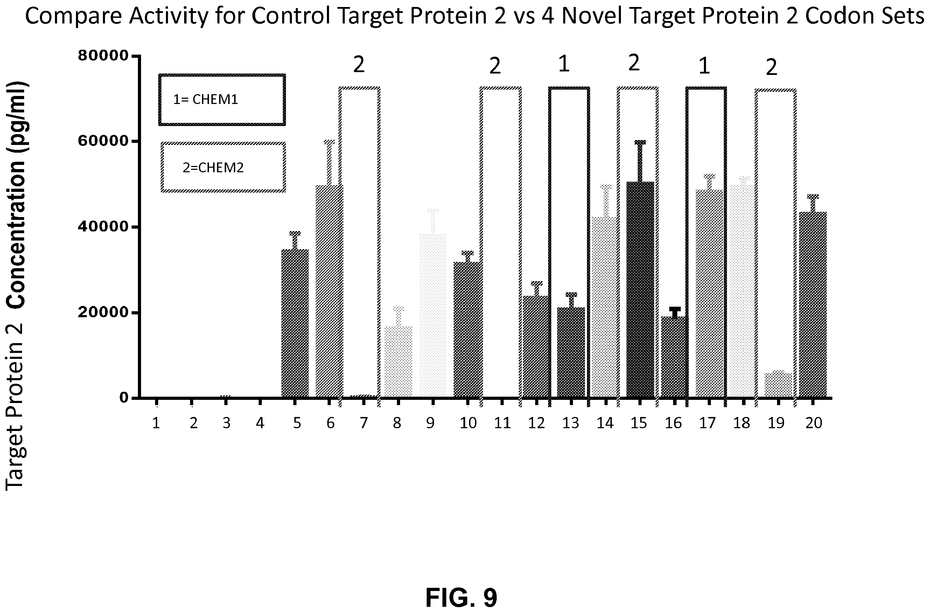

FIG. 9 shows *in vitro* expression levels for synthetic mRNAs encoding target protein 2 in HeLa cells. Several chemistries were used to generate the mRNAs (Chemi, Chem2, Chem3, and Chem4). Four optimized target specific codon sets were used (CO1, CO2, CO3, and CO4). The samples at positions 1 to 20 correspond respectively to: (1) Chem1 control; (2) Chem2 control; (3) Chem3 control; (4) Chem4 control; (5) Chem1 CO1; (6) Chem2 CO1; (7) Chem3 CO1; (8) Chem4 CO1; (9) Chem1 CO2; (10) Chem2 CO2; (11) Chem3 CO2; (12) Chem4 CO2; (13) Chem1 CO3; (14) Chem2 CO3; (15) Chem3 CO3; (16) Chem4 CO3; (17) Chem1 CO4; (18) Chem2 CO4; (19) Chem3 CO4; and (20) Chem4 CO4.

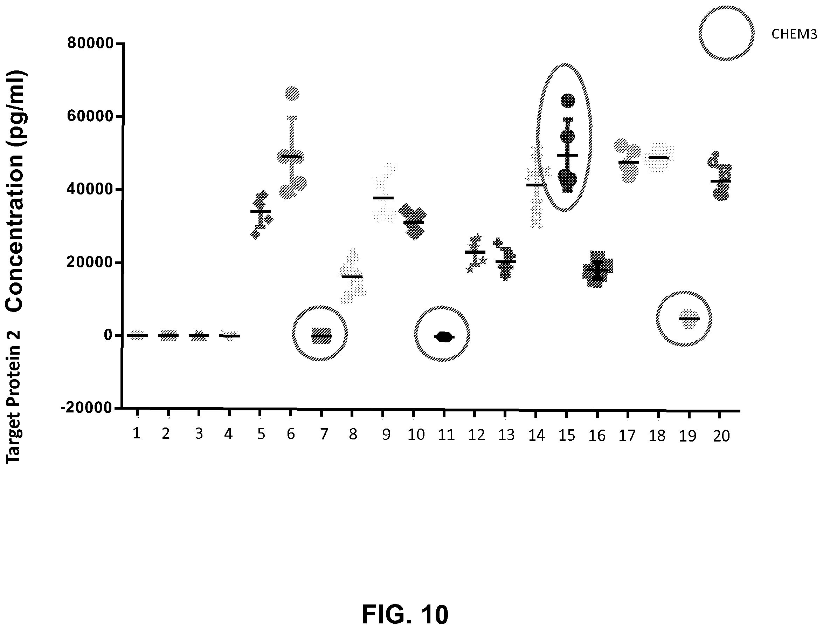

FIG. 10 shows *in vivo* activity levels for synthetic mRNAs encoding target protein 2 in mice. Several chemistries were used to generate the mRNAs (Chemi, Chem2, Chem3, and Chem4). Four optimized target specific codon sets were used (CO1, CO2, CO3, and CO4). The samples at positions 1 to 20 correspond respectively to: (1) Chem1 control; (2) Chem2 control; (3) Chem3 control; (4) Chem4 control; (5) Chem1 CO1; (6) Chem2 CO1; (7) Chem3 CO1; (8) Chem4 CO1; (9) Chem1 CO2; (10) Chem2 CO2; (11) Chem3 CO2; (12) Chem4 CO2; (13) Chem1 CO3; (14) Chem2 CO3; (15) Chem3 CO3; (16) Chem4 CO3; (17) Chem1 CO4; (18) Chem2 CO4; (19) Chem3 CO4; and (20) Chem4 CO4.

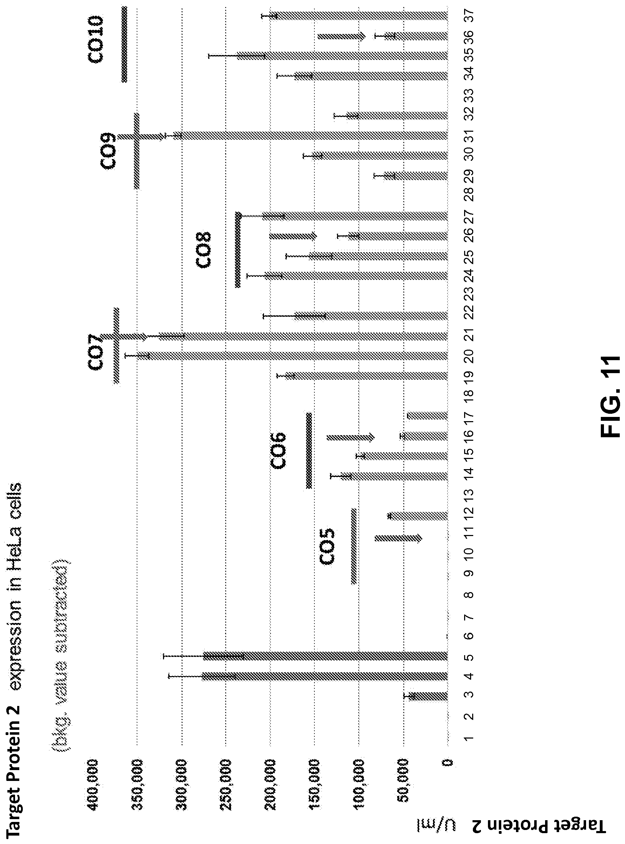

FIG. 11 shows *in vitro* expression levels for synthetic mRNAs encoding target protein 2 in HeLa cells. Several chemistries were used to generate the mRNAs (ChemO, Chem1, Chem2, Chem3, and Chem4). Six optimized target specific codon sets were used (CO5, CO6, CO7, CO8, CO9, and CO10). The samples at positions 1 to 31 respectively correspond to: (1) untreated HeLa cells; (2) L2000 control; (3) Chem0 control; (4) Chem1 control; (5) Chem2 control; (6) Chem3 control; (7) Chem4 control; (8) Chem1 CO5; (9) Chem2 CO5; (10) Chem3 CO5; (11) Chem4 CO5; (12) Chem1 CO6; (13) Chem2 CO6; (14) Chem3 CO6; (15) Chem4 CO6; (16) Chem1 CO7; (17) Chem2 CO7; (18) Chem3 CO7; (19) Chem4 CO7; (20) Chem1 CO8; (21) Chem2 CO8; (22) Chem3 CO8; (23) Chem4 CO8; (24) Chem1 CO9; (25) Chem2 CO9; (26) Chem3 CO9; (27) Chem4 CO9; (28) Chem1 CO10; (29) Chem2 CO10; (30) Chem3 CO10; and (31) Chem4 CO10.

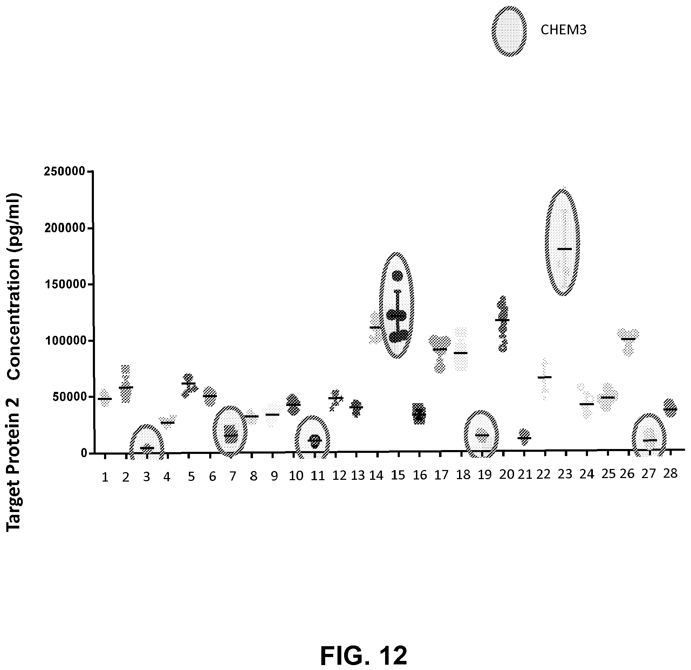

FIG. 12 shows *in vivo* activity levels for synthetic mRNAs encoding target protein 2 in mice. Several chemistries were used to generate the mRNAs. Six optimized target specific codon sets were used (CO5, CO6, CO7, CO8, CO9, and CO10). The samples at positions 1 to 28 correspond respectively to: (1) Chem1 control; (2) Chem2 control; (3) Chem3 control; (4) Chem4 control; (5) Chem1 CO5; (6) Chem2 CO5; (7) Chem3 CO5; (8) Chem4 CO5; (9) Chem1 CO6; (10) Chem2 CO6; (11) Chem3 CO6; (12) Chem4 CO6; (13) Chem1 CO7; (14) Chem2 CO7; (15) Chem3 CO7; (16) Chem4 CO7; (17) Chem1 CO8; (18) Chem2 CO8; (19) Chem3 CO8; (20) Chem4 CO8; (21) Chem1 CO9; (22) Chem2 CO9; (23) Chem3 CO9; (24) Chem4 CO9; (25) Chem1 CO10; (26) Chem2 CO10; (27) Chem3 CO10; and (28) Chem4 CO10.

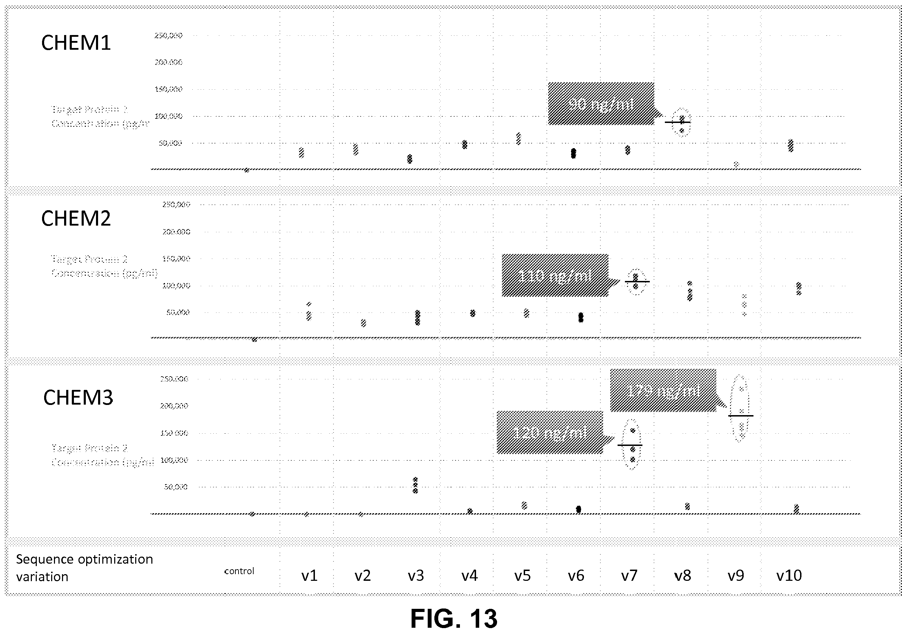

FIG. 13 shows *in vivo* activity levels for synthetic mRNAs encoding target protein 2. Three different chemistries, Chem1, Chem2 and Chem3 were used to generate the mRNAs.
FIG. 14 shows *in vivo* activity levels for synthetic mRNAs encoding target protein 4, target protein 5, and target protein 6. Four different chemistries, Chem0, Chem1, Chem2, and Chem3 were used to generate the mRNAs.
FIG. 15 shows a schematic representation of an exemplary embodiment of a multiparametric nucleic acid optimization method disclosed in the application.
FIG. 16 presents a flowchart diagram of an exemplary embodiment of a multiparametric nucleic acid optimization method disclosed in the application.
FIG. 17 shows a block diagram of a codon optimization system 1700 according to an embodiment of the present invention.
FIG. 18 illustrates an example computing device 1800 implementing the multiparametric method for nucleic acid optimization as disclosed in the present application. Due to the complexity of the calculations involved, a multiparametric codon optimizer needs to be implemented on a computer specially programmed to conduct the complex optimization process.

### DETAILED DESCRIPTION

The present disclosure is directed to multiparametric methods to optimize the production of natural and synthetic nucleic acid sequences, e.g., mRNA sequences, and to their use express proteins, for example, *in vivo* in a host organism (e.g., in a particular tissue or cell). These multiparametric methods can be used to optimize parameters related to expression efficacy, for example, of an mRNA (e.g., a synthetic mRNA) administered *in vivo* to a subject in need thereof. Such parameters include, but are not limited to, improving nucleic acid stability (e.g., mRNA stability), increasing translation efficacy in the target tissue, reducing the number of truncated proteins expressed, improving the folding or prevent misfolding of the expressed proteins, reducing toxicity of the expressed products, reducing cell death caused by the expressed products, increasing or decreasing protein aggregation, etc.

In addition, the disclosed methods can be used to select the optimal expression system to produce a recombinant protein (e.g., a certain protein cell line) by evaluating some or all the parameters related to expression efficacy mentioned above in a panel of candidate expression systems.

The present disclosure also provides polynucleotides (e.g., mRNAs, synthetic mRNAs, etc.), vectors, and pharmaceutical compositions generated by using the multiparametric nucleic acid optimization methods disclosed herein. Also provided are methods of making (e.g., methods to synthesize mRNA sequences optimized according to the multiparametric optimization disclosed herein) as well as methods of using the optimized nucleic acids disclosed herein, for example, as therapeutic mRNAs.

The present disclosure provides methods that can be applied *in vitro*, for example, by generating a library of optimized nucleic acids (e.g., mRNAs, synthetic mRNAs, etc.) and then testing them experimentally to determine the degree of improvement of properties related to protein expression efficacy.

Also provided are methods that can be applied *in silico*, for example, by modeling the generation of a library of optimized nucleic acids (e.g., mRNAs, synthetic mRNAs, etc.) and scoring each one of them to predict the degree of improvement of properties related to translational efficacy in each one of them, or by iteratively modeling changes in a candidate (non-optimized) nucleic acid sequence (e.g., a natural mRNA or a synthetic mRNA) and scoring the effect of the sequential or concurrent *in silico* modifications to the candidate nucleic acid sequence (e.g., a natural mRNA or a synthetic mRNA) until a predicted set of characteristics or a certain predetermined scoring threshold has been reached.

The disclosure also provides methods in which a nucleotide sequence (e.g., a natural mRNA or a synthetic mRNA) is optimized or a nucleotide sequence (e.g., a natural mRNA or a synthetic mRNA) is selected from a population of optimized sequences generated *in silico*, wherein such synthetic nucleotide sequences (e.g., natural mRNA or synthetic mRNA) are specifically optimized for a particular form of administration (e.g., administration of a synthetic mRNA to a particular tissue or using a particular formulation or delivery system) and/or for expression *in vivo* in a particular tissue or cell, with the aid of a computer. Also provided are implementations of the disclosed methods in computer systems and the implementation of the disclosed methods as software to be stored in computer readable media.

In order that the present disclosure can be more readily understood, certain terms are first defined. Additional definitions are set forth throughout the detailed description.

### I. Definitions

Before describing the present invention in detail, it is to be understood that this invention is not limited to specific compositions or process steps, as such can vary. As used in this specification and the appended claims, the singular forms "a", "an" and "the" include plural referents unless the context clearly dictates otherwise. The terms "a" (or "an"), as well as the terms "one or more," and "at least one" can be used interchangeably herein.

Furthermore, "and/or" where used herein is to be taken as specific disclosure of each of the two specified features or components with or without the other. Thus, the term "and/or" as used in a phrase such as "A and/or B" herein is intended to include "A and B," "A or B," "A" (alone), and "B" (alone). Likewise, the term "and/or" as used in a phrase such as "A, B, and/or C" is intended to encompass each of the following aspects: A, B, and C; A, B, or C; A or C; A or B; B or C; A and C; A and B; B and C; A (alone); B (alone); and C (alone).

Unless defined otherwise, all technical and scientific terms used herein have the same meaning as commonly understood by one of ordinary skill in the art to which this disclosure is related. For example, the Concise Dictionary of Biomedicine and Molecular Biology, Juo, Pei-Show, 2nd ed., 2002, CRC Press; The Dictionary of Cell and Molecular Biology, 3rd ed., 1999, Academic Press; and the Oxford Dictionary Of Biochemistry And Molecular Biology, Revised, 2000, Oxford University Press, provide one of skill with a general dictionary of many of the terms used in this disclosure.

It is understood that wherever aspects are described herein with the language "comprising," otherwise analogous aspects described in terms of "consisting of" and/or "consisting essentially of' are also provided.

The headings provided herein are not limitations of the various aspects, which can be had by reference to the specification as a whole. Accordingly, the terms defined immediately below are more fully defined by reference to the specification in its entirety.

Units, prefixes, and symbols are denoted in their Système International de Unites (SI) accepted form. Numeric ranges are inclusive of the numbers defining the range. Where a range of values is recited, it is to be understood that each intervening integer value, and each fraction thereof, between the recited upper and lower limits of that range is also specifically disclosed, along with each subrange between such values. The upper and lower limits of any range can independently be included in or excluded from the range, and each range where either, neither or both limits are included is also encompassed within the invention. Where a value being discussed has inherent limits, for example where a component can be present at a concentration of from 0 to 100%, or where the pH of an aqueous solution can range from 1 to 14, those inherent limits are specifically disclosed. Where a value is explicitly recited, it is to be understood that values which are about the same quantity or amount as the recited value are also within the scope of the invention. Where a combination is disclosed, each subcombination of the elements of that combination is also specifically disclosed and is within the scope of the invention. Conversely, where different elements or groups of elements are individually disclosed, combinations thereof are also disclosed. Where any element of an invention is disclosed as having a plurality of alternatives, examples of that invention in which each alternative is excluded singly or in any combination with the other alternatives are also hereby disclosed; more than one element of an invention can have such exclusions, and all combinations of elements having such exclusions are hereby disclosed.

Nucleotides are referred to by their commonly accepted single-letter codes. Unless otherwise indicated, nucleic acids are written left to right in 5' to 3' orientation. Nucleotides are referred to herein by their commonly known one-letter symbols recommended by the IUPAC-IUB Biochemical Nomenclature Commission. Accordingly, A represents adenine, C represents cytosine, G represents guanine, T represents thymine, U represents uracil, R represents A or G, Y represents C or T, S represents G or C, W represents A or T, K represents G or T, M represents A or C, B represents C or G or T, D represents A or G or T, H represents A or C or T, V represents A or C or G, and N represents any base.

Amino acids are referred to herein by either their commonly known three letter symbols or by the one-letter symbols recommended by the IUPAC-IUB Biochemical Nomenclature Commission. Unless otherwise indicated, amino acid sequences are written left to right in amino to carboxy orientation.

The terms "nucleic acid" or "nucleic acid molecule," "gene," "polynucleotide," or "oligonucleotide," are used interchangeably herein to refer to polymers of nucleotides of any length, and ribonucleotides, deoxyribonucleotides, analogs thereof, or mixtures thereof. This term refers only to the primary structure of the molecule. Thus, the term includes triple-, double- and single-stranded deoxyribonucleic acid ("DNA"), as well as triple-, double- and single-stranded ribonucleic acid ("RNA" ). It also includes modified, for example by alkylation, and/or by capping, and unmodified forms of the polynucleotide. More particularly, the terms "polynucleotide," "oligonucleotide," "nucleic acid" and "nucleic acid molecule" include polydeoxyribonucleotides (containing 2-deoxy-D-ribose), polyribonucleotides (containing D-ribose), including tRNA, rRNA, hRNA, siRNA and mRNA, whether spliced or unspliced, any other type of polynucleotide which is an N- or C-glycoside of a purine or pyrimidine base, and other polymers containing normucleotidic backbones, for example, polyamide (e.g., peptide nucleic acids "PNAs") and polymorpholino polymers, and other synthetic sequence-specific nucleic acid polymers providing that the polymers contain nucleobases in a configuration which allows for base pairing and base stacking, such as is found in DNA and RNA. In particular aspects, the nucleic acid is an mRNA. In other aspect, the mRNA is a synthetic mRNA. In some aspects, the synthetic mRNA comprises at least one unnatural nucleobase. In some aspects, all nucleobases of a certain class have been replaced with unnatural nucleobases (e.g., all uridines in a nucleic acid of the present invention can be replaced with a unnatural nucleobase, e.g., 5-methoxyuridine).

There is no intended distinction in length between the terms "polynucleotide," "oligonucleotide," "nucleic acid," and "nucleic acid molecule," and these terms are used interchangeably herein. These terms refer only to the primary structure of the molecule. Thus, these terms include, for example, 3'-deoxy-2', 5'-DNA, oligodeoxyribonucleotide N3' P5' phosphoramidates, 2'-O-alkyl-substituted RNA, double- and single-stranded DNA, as well as double- and single-stranded RNA, and hybrids thereof including for example hybrids between DNA and RNA or between PNAs and DNA or RNA, and also include known types of modifications, for example, labels, alkylation, "caps," substitution of one or more of the nucleotides with an analog, internucleotide modifications such as, for example, those with uncharged linkages (e.g., methyl phosphonates, phosphotriesters, phosphoramidates, carbamates, etc.), with negatively charged linkages (e.g., phosphorothioates, phosphorodithioates, etc.), and with positively charged linkages (e.g., aminoalkylphosphoramidates, amino-alkyl-phosphotriesters), those containing pendant moieties, such as, for example, proteins (including enzymes (e.g. nucleases), toxins, antibodies, signal peptides, poly-L-lysine, etc.), those with intercalators (e.g., acridine, psoralen, etc.), those containing chelates (of, e.g., metals, radioactive metals, boron, oxidative metals, etc.), those containing alkylators, those with modified linkages (e.g., alpha anomeric nucleic acids, etc.), as well as unmodified forms of the polynucleotide or oligonucleotide.

Where the polynucleotides are to be used to express encoded proteins, nucleotides that can perform that function or which can be modified (e.g., reverse transcribed) to perform that function are used. Where the polynucleotides are to be used in a scheme that requires that a complementary strand be formed to a given polynucleotide, nucleotides are used which permit such formation.

It will be appreciated that, as used herein, the terms "nucleoside" and "nucleotide" will include those moieties which contain not only the known purine and pyrimidine bases, but also other heterocyclic bases which have been modified. Such modifications include methylated purines or pyrimidines, acylated purines or pyrimidines, or other heterocycles. Modified nucleosides or nucleotides can also include modifications on the sugar moiety, e.g., where one or more of the hydroxyl groups are replaced with halogen, aliphatic groups, or is functionalized as ethers, amines, or the like.

Standard A-T and G-C base pairs form under conditions which allow the formation of hydrogen bonds between the N3-H and C4-oxy of thymidine and the N1 and C6-NH2, respectively, of adenosine and between the C2-oxy, N3 and C4-NH2, of cytidine and the C2-NH2, N'-H and C6-oxy, respectively, of guanosine. Thus, for example, guanosine (2-amino-6-oxy-9-β-D-ribofuranosyl-purine) may be modified to form isoguanosine (2-oxy-6-amino-9-β-D-ribofuranosyl-purine). Such modification results in a nucleoside base which will no longer effectively form a standard base pair with cytosine. However, modification of cytosine (1-β-D-ribofuranosyl-2-oxy-4-amino-pyrimidine) to form isocytosine (1-β-D-ribofuranosyl-2-amino-4-oxy-pyrimidine-) results in a modified nucleotide which will not effectively base pair with guanosine but will form a base pair with isoguanosine (U.S. Pat. No. 5,681,702 to Collins et al., hereby incorporated by reference in its entirety). Isocytosine is available from Sigma Chemical Co. (St. Louis, Mo.); isocytidine may be prepared by the method described by Switzer et al. (1993) Biochemistry 32:10489-10496 and references cited therein; 2'-deoxy-5-methyl-isocytidine may be prepared by the method of Tor et al., 1993, J. Am. Chem. Soc. 115:4461-4467 and references cited therein; and isoguanine nucleotides may be prepared using the method described by Switzer et al., 1993, supra, and Mantsch et al., 1993, Biochem. 14:5593-5601, or by the method described in U.S. Pat. No. 5,780,610 to Collins et al., each of which is hereby incorporated by reference in its entirety. Other nonnatural base pairs may be synthesized by the method described in Piccirilli et al., 1990, Nature 343:33-37, hereby incorporated by reference in its entirety, for the synthesis of 2,6-diaminopyrimidine and its complement (1-methylpyrazolo-[4,3]pyrimidine-5,7-(4H,6H)-dione. Other such modified nucleotide units which form unique base pairs are known, such as those described in Leach et al. (1992) J. Am. Chem. Soc. 114:3675-3683 and Switzer et al., supra.

The phrases "DNA sequence" or "nucleic acid sequence" refer to a contiguous nucleic acid sequence, and corresponds to nucleotide polymer wherein the polynucleotide monomer are covalenty bound. The term "sequence" as applied to a nucleic acid molecule, is well known in the art. In the context of the present disclosure, the term "sequence" encompasses both the physical nucleic acid (i.e., a nucleic acid molecule) and its symbolic representation (e.g., a string of characters, etc. ATCG, wherein each character in the string represents a nucleotide). The sequence can be either single stranded or double stranded, DNA or RNA, but double stranded DNA sequences are preferable. The sequence can be an oligonucleotide of 6 to 20 nucleotides in length to a full length genomic sequence of thousands or hundreds of thousands of base pairs. As used herein, the term "subsequence" refers to a subset of contiguous nucleotides in a sequence (either the physical sequence or its symbolic representation). E.g., for the sequence "AAACGATTT", CGA would be a subsequence.

The term "vector" means a construct, which is capable of delivering, and in some aspects, expressing, one or more gene(s) or sequence(s) of interest in a host cell. Examples of vectors include, but are not limited to, viral vectors, naked DNA or RNA expression vectors, plasmid, cosmid or phage vectors, DNA or RNA expression vectors associated with cationic condensing agents, DNA or RNA expression vectors encapsulated in liposomes, and certain eukaryotic cells, such as producer cells.

The term "expression system" refers to any *in vivo* or *in vitro* biological system that is used to produce one or more proteins encoded by a polynucleotide (e.g., a therapeutic mRNA). In particular aspects of the present disclosure, the term expression system encompasses tissues or cells of a subject to whom a nucleic acid optimized according to the methods disclosed herein (e.g., a synthetic mRNA) has been administered.

A polypeptide, polynucleotide, vector, or composition which is "isolated" is a polypeptide, polynucleotide, vector, cell, or composition which is in a form not found in nature. Isolated polypeptides, polynucleotides, vectors, or compositions include those which have been purified to a degree that they are no longer in a form in which they are found in nature. In some aspects, a polynucleotide, vector, or composition which is isolated is substantially pure.

The terms "polypeptide," "peptide," and "protein" are used interchangeably herein to refer to polymers of amino acids of any length. The polymer can be linear or branched, it can comprise modified amino acids, and it can be interrupted by non-amino acids. The terms also encompass an amino acid polymer that has been modified naturally or by intervention; for example, disulfide bond formation, glycosylation, lipidation, acetylation, phosphorylation, or any other manipulation or modification, such as conjugation with a labeling component. Also included within the definition are, for example, polypeptides containing one or more analogs of an amino acid (including, for example, unnatural amino acids such as homocysteine, ornithine, p-acetylphenylalanine, D-amino acids, and creatine), as well as other modifications known in the art.

The terms "codon substitution" or "codon replacement" refer to replacing a codon present in a parent sequence, e.g., a candidate nucleic acid sequence (e.g., an mRNA), with another codon. A codon can be substituted in a candidate nucleic acid sequence, for example, via chemical peptide synthesis or through recombinant methods known in the art. Accordingly, references to a "substitution" or "replacement" at a certain location in a nucleic acid sequence (e.g., an mRNA) or within a certain region or subsequence of a nucleic acid sequence (e.g., an mRNA) refers to the substitution of a codon at such location or region with an alternative codon.

The term "percent sequence identity" between two polypeptide or polynucleotide sequences refers to the number of identical matched positions shared by the sequences over a comparison window, taking into account additions or deletions (i.e., gaps) that must be introduced for optimal alignment of the two sequences. A matched position is any position where an identical nucleotide or amino acid is presented in both the target and reference sequence. Gaps presented in the target sequence are not counted since gaps are not nucleotides or amino acids. Likewise, gaps presented in the reference sequence are not counted since target sequence nucleotides or amino acids are counted, not nucleotides or amino acids from the reference sequence.

The percentage of sequence identity is calculated by determining the number of positions at which the identical amino-acid residue or nucleic acid base occurs in both sequences to yield the number of matched positions, dividing the number of matched positions by the total number of positions in the window of comparison and multiplying the result by 100 to yield the percentage of sequence identity. The comparison of sequences and determination of percent sequence identity between two sequences can be accomplished using readily available software both for online use and for download. Suitable software programs are available from various sources, and for alignment of both protein and nucleotide sequences. One suitable program to determine percent sequence identity is bl2seq, part of the BLAST suite of program available from the U.S. government's National Center for Biotechnology Information BLAST web site (blast.ncbi.nlm.nih.gov). Bl2seq performs a comparison between two sequences using either the BLASTN or BLASTP algorithm. BLASTN is used to compare nucleic acid sequences, while BLASTP is used to compare amino acid sequences. Other suitable programs are, e.g., Needle, Stretcher, Water, or Matcher, part of the EMBOSS suite of bioinformatics programs and also available from the European Bioinformatics Institute (EBI) at www.ebi.ac.uk/Tools/psa.

Different regions within a single polynucleotide or polypeptide target sequence that aligns with a polynucleotide or polypeptide reference sequence can each have their own percent sequence identity. It is noted that the percent sequence identity value is rounded to the nearest tenth. For example, 80.11, 80.12, 80.13, and 80.14 are rounded down to 80.1, while 80.15, 80.16, 80.17, 80.18, and 80.19 are rounded up to 80.2. It also is noted that the length value will always be an integer.

In certain aspects, the percentage identity "X" of a first amino acid sequence to a second amino acid sequence is calculated as 100 x (Y/Z), where Y is the number of amino acid residues scored as identical matches in the alignment of the first and second sequences (as aligned by visual inspection or a particular sequence alignment program) and Z is the total number of residues in the second sequence. If the length of a first sequence is longer than the second sequence, the percent identity of the first sequence to the second sequence will be higher than the percent identity of the second sequence to the first sequence.

One skilled in the art will appreciate that the generation of a sequence alignment for the calculation of a percent sequence identity is not limited to binary sequence-sequence comparisons exclusively driven by primary sequence data. It will also be appreciated that sequence alignments can be generated by integrating sequence data with data from heterogeneous sources such as structural data (e.g., crystallographic protein structures), functional data (e.g., location of mutations), or phylogenetic data. A suitable program that integrates heterogeneous data to generate a multiple sequence alignment is T-Coffee, available at www.tcoffee.org, and alternatively available, e.g., from the EBI. It will also be appreciated that the final alignment used to calculate percent sequence identity can be curated either automatically or manually.

The term "subject" refers to any animal (*e*.*g*., a mammal), including, but not limited to humans, non-human primates, rodents, and the like, which is to be the recipient of a particular treatment. Typically, the terms "subject" and "patient" are used interchangeably herein in reference to a human subject.

The term "pharmaceutical composition" refers to a preparation which is in such form as to permit the biological activity of the active ingredient to be effective, and which contains no additional components which are unacceptably toxic to a subject to which the composition would be administered. Such composition can be sterile.

As used herein, the term "candidate nucleic acid sequence" refers to a nucleic sequence (e.g., an mRNA sequence) that can be optimized, for example, to improve its translation efficacy, according to the methods disclosed herein. In some aspects, the candidate nucleic acid sequence (e.g., an mRNA sequence) is optimized for improved translation efficacy after *in vivo* administration. When the multiparametric codon optimization methods disclosed herein are applied iteratively, the optimized nucleic acid sequence obtained after one cycle of optimization would become the candidate nucleic acid sequence for the subsequent cycle of optimization.

In some aspects of the methods disclosed herein, the nucleobase composition of a candidate nucleic acid sequence can be modified through enrichment or rarefaction in uridine, cytidine, guanosine, or adenosine, to yield modified sequences, i.e., a uridine-modified sequence, a cytidine-modified sequence, a guanosine-modified sequence, or an adenoside-modified sequence, respectively.

The term "uridine-modified sequence" refers to an optimized nucleic acid sequence (e.g., a synthetic mRNA sequence) with a different overall or local uridine content (higher or lower uridine content) or with different uridine patterns (e.g., gradient distribution or clustering) with respect to the uridine content and/or uridine patterns of a candidate nucleic acid sequence. A "high uridine codon" is defined as a codon comprising two or three uridines, a "low uridine codon" is defined as a codon comprising one uridine, and a "no uridine codon" is a codon without any uredines. In some aspects, a uridine-modified sequence comprises substitutions of high uridine codons with low uridine codons, substitutions of high uridine codons with no uridine codons, substitutions of low uridine codons with high uridine codons, substitutions of low uridine codons with no uridine codons, substitution of no uridine codons with low uridine codons, substitutions of no uridine codons with high uridine codons, and combinations thereof. In some aspects, a high uridine codon can be replaced with another high uridine codon. In some aspects, a low uridine codon can be replaced with another low uridine codon. In some aspects, a no uridine codon can be replaced with another no uridine codon.

As used herein, the terms "uridine enrichment" and grammatical variants (e.g., "uridine enriched") refer to the increase in uridine content (expressed in absolute value or as a percentage value) in an optimized nucleic acid sequence (e.g., a synthetic mRNA sequence) with respect to the uridine content of the corresponding candidate nucleic acid sequence. Uridine enrichment can be implemented by substituting codons in the candidate nucleic acid sequence with synonymous codons containing less uridine nucleobases. Uridine enrichment can be global (i.e., relative to the entire length of a candidate nucleic acid sequence) or local (i.e., relative to a subsequence or region of a candidate nucleic acid sequence).

As used herein, the terms "uridine rarefication" and grammatical variants (e.g., "uridine rarefied") refer to a decrease in uridine content (expressed in absolute value or as a percentage value) in an optimized nucleic acid sequence (e.g., a synthetic mRNA sequence) with respect to the uridine content of the corresponding candidate nucleic acid sequence. Uridine rarefication can be implemented by substituting codons in the candidate nucleic acid sequence with synonymous codons containing less uridine nucleobases. Uridine rarefication can be global (i.e., relative to the entire length of a candidate nucleic acid sequence) or local (i.e., relative to a subsequence or region of a candidate nucleic acid sequence).

The term "cytidine-modified sequence" refers to an optimized nucleic acid sequence (e.g., a synthetic mRNA sequence) with a different overall or local cytidine content (higher or lower cytidine content) or with different cytidine patterns (e.g., gradient distribution or clustering) with respect to the cytidine content and/or cytidine patterns of a candidate nucleic acid sequence. A "high cytidine codon" is defined as a codon comprising two or three cytidines, a "low cytidine codon" is defined as a codon comprising one cytidine, and a "no cytidine codon" is a codon without any cytidine. In some aspects, a cytidine-modified sequence comprises substitutions of high cytidine codons with low cytidine codons, substitutions of high cytidine codons with no cytidine codons, substitutions of low cytidine codons with high cytidine codons, substitutions of low cytidine codons with no cytidine codons, substitution of no cytidine codons with low cytidine codons, substitutions of no cytidine codons with high cytidine codons, and combinations thereof. In some aspects, a high cytidine codon can be replaced with another high cytidine codon. In some aspects, a low cytidine codon can be replaced with another low cytidine codon. In some aspects, a no cytidine codon can be replaced with another no cytidine codon.

As used herein, the terms "cytidine enrichment" and grammatical variants (e.g., "cytidine enriched") refer to the increase in cytidine content (expressed in absolute value or as a percentage value) in an optimized nucleic acid sequence (e.g., a synthetic mRNA sequence) with respect to the cytidine content of the corresponding candidate nucleic acid sequence. Cytidine enrichment can be implemented by substituting codons in the candidate nucleic acid sequence with synonymous codons containing less cytidine nucleobases. Cytidine enrichment can be global (i.e., relative to the entire length of a candidate nucleic acid sequence) or local (i.e., relative to a subsequence or region of a candidate nucleic acid sequence).

As used herein, the terms "cytidine rarefication" and grammatical variants (e.g., "cytidine rarefied") refer to a decrease in cytidine content (expressed in absolute value or as a percentage value) in an optimized nucleic acid sequence (e.g., a synthetic mRNA sequence) with respect to the cytidine content of the corresponding candidate nucleic acid sequence. Cytidine rarefication can be implemented by substituting codons in the candidate nucleic acid sequence with synonymous codons containing less cytidine nucleobases. Cytidine rarefication can be global (i.e., relative to the entire length of a candidate nucleic acid sequence) or local (i.e., relative to a subsequence or region of a candidate nucleic acid sequence).

The term "adenosine-modified sequence" refers to an optimized nucleic acid sequence (e.g., a synthetic mRNA sequence) with a different overall or local adenosine content (higher or lower adenosine content) or with different adenosine patterns (e.g., gradient distribution or clustering) with respect to the adenosine content and/or cytidine adenosine of a candidate nucleic acid sequence. A "high adenosine codon" is defined as a codon comprising two or three adenosines, a "low adenosine codon" is defined as a codon comprising one adenosine, and a "no adenosine codon" is a codon without any adenosine. In some aspects, an adenosine-modified sequence comprises substitutions of high adenosine codons with low adenosine codons, substitutions of high adenosine codons with no adenosine codons, substitutions of low adenosine codons with high adenosine codons, substitutions of low adenosine codons with no adenosine codons, substitution of no adenosine codons with low adenosine codons, substitutions of no adenosine codons with high adenosine codons, and combinations thereof. In some aspects, a high adenosine codon can be replaced with another high adenosine codon. In some aspects, a low adenosine codon can be replaced with another low adenosine codon. In some aspects, a no adenosine codon can be replaced with another no adenosine codon.

As used herein, the terms "adenosine enrichment" and grammatical variants (e.g., "adenosine enriched") refer to the increase in adenosine content (expressed in absolute value or as a percentage value) in an optimized nucleic acid sequence (e.g., a synthetic mRNA sequence) with respect to the adenosine content of the corresponding candidate nucleic acid sequence. Adenosine enrichment can be implemented by substituting codons in the candidate nucleic acid sequence with synonymous codons containing less adenosine nucleobases. Adenosine enrichment can be global (i.e., relative to the entire length of a candidate nucleic acid sequence) or local (i.e., relative to a subsequence or region of a candidate nucleic acid sequence).

As used herein, the terms "adenosine rarefication" and grammatical variants (e.g., "adenosine rarefied") refer to a decrease in adenosine content (expressed in absolute value or as a percentage value) in an optimized nucleic acid sequence (e.g., a synthetic mRNA sequence) with respect to the adenosine content of the corresponding candidate nucleic acid sequence. Adenosine rarefication can be implemented by substituting codons in the candidate nucleic acid sequence with synonymous codons containing less adenosine nucleobases. Adenosine rarefication can be global (i.e., relative to the entire length of a candidate nucleic acid sequence) or local (i.e., relative to a subsequence or region of a candidate nucleic acid sequence).

The term "guanosine-modified sequence" refers to an optimized nucleic acid sequence (e.g., a synthetic mRNA sequence) with a different overall or local guanosine content (higher or lower guanosine content) or with different guanosine patterns (e.g., gradient distribution or clustering) with respect to the guanosine content and/or guanosine patterns of a candidate nucleic acid sequence. A "high guanosine codon" is defined as a codon comprising two or three cytidines, a "low guanosine codon" is defined as a codon comprising one guanosine, and a "no guanosine codon" is a codon without any guanosine. In some aspects, a guanosine-modified sequence comprises substitutions of high guanosine codons with low guanosine codons, substitutions of high guanosine codons with no guanosine codons, substitutions of low guanosine codons with high guanosine codons, substitutions of low guanosine codons with no guanosine codons, substitution of no guanosine codons with low guanosine codons, substitutions of no guanosine codons with high guanosine codons, and combinations thereof. In some aspects, a high guanosine codon can be replaced with another high guanosine codon. In some aspects, a low guanosine codon can be replaced with another low guanosine codon. In some aspects, a no guanosine codon can be replaced with another no guanosine codon.

As used herein, the terms "guanosine enrichment" and grammatical variants (e.g., "guanosine enriched") refer to the increase in guanosine content (expressed in absolute value or as a percentage value) in an optimized nucleic acid sequence (e.g., a synthetic mRNA sequence) with respect to the guanosine content of the corresponding candidate nucleic acid sequence. Guanosine enrichment can be implemented by substituting codons in the candidate nucleic acid sequence with codons containing less guanosine nucleobases. Guanosine enrichment can be global (i.e., relative to the entire length of a candidate nucleic acid sequence) or local (i.e., relative to a subsequence or region of a candidate nucleic acid sequence).

As used herein, the terms "guanosine rarefication" and grammatical variants (e.g., "guanosine rarefied") refer to a decrease in guanosine content (expressed in absolute value or as a percentage value) in an optimized nucleic acid sequence (e.g., a synthetic mRNA sequence) with respect to the guanosine content of the corresponding candidate nucleic acid sequence. Guanosine rarefication can be implemented by substituting codons in the candidate nucleic acid sequence with codons containing less guanosine nucleobases. Guanosine rarefication can be global (i.e., relative to the entire length of a candidate nucleic acid sequence) or local (i.e., relative to a subsequence or region of a candidate nucleic acid sequence).

### II. Multiparametric Methods for Nucleic Acid Optimization

The present disclosure provides multiparametric methods for nucleic acid optimization (e.g., to optimize the *in vivo* expression of synthetic mRNA). In some aspects the present disclosure provides a method for optimizing a candidate nucleic acid sequence (e.g., an mRNA), the method comprising:
(i) modifying at least one subsequence in the candidate nucleic acid sequence to generate a ramp subsequence;
(ii) substituting at least one codon in the candidate nucleic acid with an alternative codon to increase or decrease uridine content to generate a uridine-modified sequence;
(iii) substituting at least one codon in the candidate nucleic acid sequence or the uridine-modified sequence with a fast recharging codon (i.e., a codon with a fast recharging rate, which can be a species specific, tissue type specific, or cell type specific recharging rate);
(iv) substituting at least one codon in the candidate nucleic acid with an alternative codon having a higher codon frequency in the synonymous codon set;
(v) substituting at least one natural nucleobase in the candidate nucleic acid with an alternative synthetic nucleobase (e.g., LNA, PNA, etc.);
(vi) substituting at least one internucleoside linkage in the candidate nucleic acid with a non-natural internucleoside linkage (e.g., a phosphorothioate linkage);
(vii) substituting at least one motif in the candidate nucleic acid with an alternative motif; and,
(viii) combinations thereof,
wherein the resulting optimized nucleic acid sequence has at least one improved property (e.g., increased protein expression efficacy) with respect to the candidate nucleic acid sequence.

In some aspects, the multiparametric methods disclosed can be used, for example, to optimize the expression of a protein (e.g., *in vivo* expression of a protein encoded by a therapeutic mRNA), to optimize transcription, to optimize nucleic acid stability (e.g., *in vivo* or *in vitro* stability of a mRNA), to reduce host cell death during protein expression (e.g., *in vivo* expression of a protein encoded by a therapeutic mRNA ), to increase expressed product yield and/or to reduce the abundance of truncated expression products, to increase the half-life of an mRNA, to reduce the half-life of an mRNA, to improve the folding or to prevent misfolding of the protein expression product, to increase the solubility of the protein expression product, to reduce the amount of expressed protein in aggregate form, etc.

The methods disclosed herein make possible the design of a number of optimized nucleic acid sequences (e.g., mRNA sequences for administration as therapeutic agents) based in the application of a set of optimization tools, wherein each one of the optimization tools operates according to limited set of rules designed to optimize, e.g., the translation efficacy of a mRNA in a specific target tissue. Such a set of rules can be gene sequence specific, chemistry specific (i.e., the optimization rules may depend on the nucleobase modification(s) used to generate a synthetic mRNA product), tissue specific (i.e., the desired properties of the mRNA can depend on the specific target tissue), or combinations thereof.

In addition to the parameters disclosed herein, nucleic acid sequences can be optimized, for example, for expression efficiency by integrating information related to the variation of codon biases between two or more organisms or genes or synthetically constructed bias tables; variation in the degree of codon bias within an organism, gene, or set of genes; systematic variation of codons including context; variation of codons according to their decoding tRNAs; variation in degree of similarity to a reference sequence, for example a naturally occurring sequence; structural properties of mRNAs transcribed from the DNA sequence; prior knowledge about the function of the DNA sequences upon which the codon substitution is to be based; systematic variation of codon sets for each amino acid; or combinations thereof.

In some aspects, the multiparametric methods disclosed herein comprise repeating the methods (or variations of the methods) iteratively until an optimized nucleic acid sequence (e.g., a mRNA) exhibits a value for the desired expression property (e.g., stable expression of a therapeutic mRNA administered to a subject in need thereof for a certain amount of time or reaching a certain expression level) that exceeds or is less than a predetermined value, or the optimized nucleic acid sequence (e.g., a therapeutic mRNA) and/or its expression product (e.g., a therapeutic protein) have one or desirable properties.

In some aspects, the multiparametric methods disclosed herein apply the same set of parameters in each successive iteration, whereas in other aspects, the parameters used in the multiparametric methods can potentially vary in each iteration. The implementation of the multiparametric methods disclosed herein can be conducted *in vitro*, e.g., a non-optimized nucleic acid sequence (e.g., a mRNA) can be mutated *in vitro* according to the optimization parameters disclosed herein to generate a set of optimized nucleic acid sequences (e.g., a library of mRNAs) which would then be expressed and tested for a certain expression property. In specific aspects, instead of generating a nucleic acid library, a single nucleic acid sequence (e.g., an mRNA) is generated.

In other cases, the implementation of the multiparametric methods disclosed herein can be conducted *in silico,* e.g., a non-optimized nucleic acid sequence (e.g., mRNA) can be mutated *in silico* based on rules implemented in a computer system to generate a set of optimized nucleic acid sequences (e.g., a library of mRNAs) which then would be synthesized, expressed, and tested for a certain expression property. In specific aspects, instead of generating a library, a single sequence is generated.

Accordingly, in some aspects, the predetermined value is a physically determined property (e.g., milligrams of protein/gram of tissue or plasma half-life), i.e., when the multiparametric method is applied *in vivo* or *in vitro*, whereas in other aspects the predetermined value is a computational cut-off, i.e., when the multiparametric method is applied *in silico.*

When the multiparametric methods disclosed herein are applied iteratively, they can be applied for a predetermined number of times (e.g., two, three, four, five, six, seven, eight, nine, or ten times), or they can be applied iteratively until a certain cut-off value or iteration limit is reached.

In one specific aspect, the multiparametric nucleic acid optimization method disclosed herein comprises one optimization method selected from the group consisting of (i) modifying at least one subsequence in the candidate nucleic acid sequence (e.g., an mRNA) to generate a ramp subsequence; (ii) substituting at least one codon in the candidate nucleic acid sequence (e.g., an mRNA) with an alternative codon to increase or decrease uridine content to generate a uridine-modified sequence; (iii) substituting at least one codon in the candidate nucleic acid sequence or the uridine-modified sequence with a fast recharging codon; (iv) substituting at least one codon in the candidate nucleic acid sequence with an alternative codon having a higher codon frequency in the synonymous codon set; (v) substituting at least one natural nucleobase in the candidate nucleic acid sequence with an alternative synthetic nucleobase; and (vi) substituting at least one internucleoside linkage in the candidate nucleic acid sequence with a non-natural internucleoside linkage.

In other specific aspect, the multiparametric nucleic acid optimization method disclosed herein comprises two optimization methods selected from the group consisting of (i) modifying at least one subsequence in the candidate nucleic acid sequence (e.g., an mRNA) to generate a ramp subsequence; (ii) substituting at least one codon in the candidate nucleic acid sequence with an alternative codon to increase or decrease uridine content to generate a uridine-modified sequence; (iii) substituting at least one codon in the candidate nucleic acid sequence or the uridine-modified sequence with a fast recharging codon; (iv) substituting at least one codon in the candidate nucleic acid sequence with an alternative codon having a higher codon frequency in in the synonymous codon set; (v) substituting at least one natural nucleobase in the candidate nucleic acid sequence with an alternative synthetic nucleobase; and (vi) substituting at least one internucleoside linkage in the candidate nucleic acid sequence with a non-natural internucleoside linkage.

In another aspect, the multiparametric nucleic acid optimization method disclosed herein comprises three optimization methods selected from the group consisting of (i) modifying at least one subsequence in the candidate nucleic acid sequence (e.g., an mRNA) to generate a ramp subsequence; (ii) substituting at least one codon in the candidate nucleic acid sequence with an alternative codon to increase or decrease uridine content to generate a uridine-modified sequence; (iii) substituting at least one codon in the candidate nucleic acid sequence or the uridine-modified sequence with a fast recharging codon; (iv) substituting at least one codon in the candidate nucleic acid sequence with an alternative codon having a higher codon frequency in the synonymous codon set; (v) substituting at least one natural nucleobase in the candidate nucleic acid sequence with an alternative synthetic nucleobase; and (vi) substituting at least one internucleoside linkage in the candidate nucleic acid sequence with a non-natural internucleoside linkage.

In another aspect, the multiparametric nucleic acid optimization method disclosed herein comprises four optimization methods selected from the group consisting of (i) modifying at least one subsequence in the candidate nucleic acid sequence (e.g., an mRNA) to generate a ramp subsequence; (ii) substituting at least one codon in the candidate nucleic acid sequence with an alternative codon to increase or decrease uridine content to generate a uridine-modified sequence; (iii) substituting at least one codon in the candidate nucleic acid sequence or the uridine-modified sequence with a fast recharging codon; (iv) substituting at least one codon in the candidate nucleic acid sequence with an alternative codon having a higher codon frequency in the synonymous codon set; (v) substituting at least one natural nucleobase in the candidate nucleic acid sequence with an alternative synthetic nucleobase; and (vi) substituting at least one internucleoside linkage in the candidate nucleic acid sequence with a non-natural internucleoside linkage.

In another aspect, the multiparametric nucleic acid optimization method disclosed herein comprises five optimization methods selected from the group consisting of (i) modifying at least one subsequence in the candidate nucleic acid sequence (e.g., an mRNA) to generate a ramp subsequence; (ii) substituting at least one codon in the candidate nucleic acid sequence with an alternative codon to increase or decrease uridine content to generate a uridine-modified sequence; (iii) substituting at least one codon in the candidate nucleic acid sequence or the uridine-modified sequence with a fast recharging codon; (iv) substituting at least one codon in the candidate nucleic acid sequence with an alternative codon having a higher codon frequency in the synonymous codon set; (v) substituting at least one natural nucleobase in the candidate nucleic acid sequence with an alternative synthetic nucleobase; and (vi) substituting at least one internucleoside linkage in the candidate nucleic acid sequence with a non-natural internucleoside linkage.

In another aspect, the multiparametric nucleic acid optimization method disclosed herein comprises six optimization methods selected from the group consisting of (i) modifying at least one subsequence in the candidate nucleic acid sequence (e.g., an mRNA) to generate a ramp subsequence; (ii) substituting at least one codon in the candidate nucleic acid sequence with an alternative codon to increase or decrease uridine content to generate a uridine-modified sequence; (iii) substituting at least one codon in the candidate nucleic acid sequence or the uridine-modified sequence with a fast recharging codon; (iv) substituting at least one codon in the candidate nucleic acid sequence with an alternative codon having a higher codon frequency in the synonymous codon set; (v) substituting at least one natural nucleobase in the candidate nucleic acid sequence with an alternative synthetic nucleobase; and (vi) substituting at least one internucleoside linkage in the candidate nucleic acid sequence with a non-natural internucleoside linkage.

In some aspects, the multiparametric nucleic acid optimization method disclosed herein comprises 7, 8, 9, 10, 11, 12, 13, 14, 15, 16, 17, 18, 19 or 20 optimization methods. In some aspects, the multiparametric nucleic acid optimization method disclosed herein comprises more than 20 optimization methods.

In some aspects of the present disclosure, at least 5%, at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 85%, at least 90%, at least 95%, at least 96%, at least 97%, at least 98%, at least 99% or 100% of the codons in the candidate nucleic acid sequence are replaced. Accordingly, the final product of the disclosed optimization process is a nucleic acid (e.g., a synthetic mRNA) in which at least 5%, at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 85%, at least 90%, at least 95%, at least 96%, at least 97%, at least 98%, at least 99% or 100% of the codons in the candidate nucleic acid sequence have been replaced by synonymous codons.

In certain aspects, the optimization methods implemented in multiparametric nucleic acid optimization methods disclosed herein are executed sequentially, concurrently, recursively, or iteratively.

The multiparametric nucleic acid optimization methods disclosed herein can be applied to a method to express a therapeutic protein interest *in vivo* in a specific tissue or cell in a subject in need thereof or in an *in vitro* translation system, the method comprising:
(a) obtaining an optimized gene sequence according to the multiparametric nucleic acid optimization methods disclosed herein;
(b) synthesizing a nucleic acid molecule (e.g., a synthetic mRNA) comprising the optimized gene sequence;
(c) administering the nucleic acid molecule (e.g., an mRNA) to a subject or combining it with the *in vitro* translation system,
wherein the nucleic acid molecule (e.g., a synthetic mRNA) has at least one optimized property with respect to a candidate nucleic acid sequence is selected from:
(i) increased *in vivo* or *in vitro* translational efficacy;
(ii) increased *in vivo* or *in vitro* half-life (e.g., plasma half-life);
(iii) decreased *in vivo* or *in vitro* half-life (e.g., plasma half-life);
(iv) increased *in vivo* or *in vitro* protein expression levels;
(v) decreased protein degradation;
(vi) decreased mRNA degradation;
(vii) increased nucleic acid (e.g., mRNA) structural stability;
(viii) decreased toxicity of the translation product;
(ix) increased viability of cells expressing the translation;
(x) decreased protein aggregation; and,
(xi) combinations thereof.

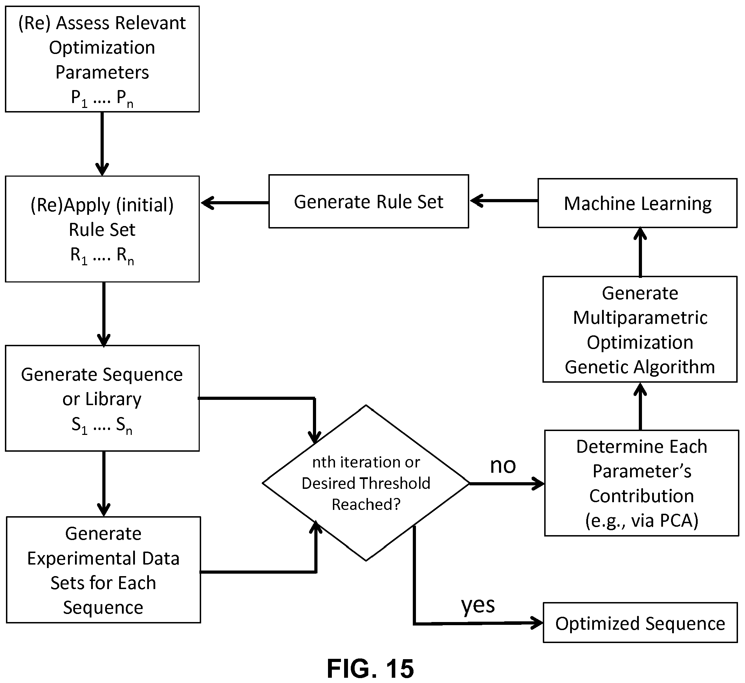

FIG. 15 presents a schema showing a general implementation of the multiparametric methods disclosed herein. Accordingly, in a first step a candidate sequence (e.g., a candidate nucleic acid sequence such as an mRNA) can be assessed to determine which set of parameters (optimization methods) in the multiparametric method would be applicable to the specific optimization process. In some aspects, the choice of optimization methods to apply can depend, for example, from particular characteristics of the candidate nucleic acid sequence (or properties of the protein encoded by the candidate nucleic acid sequence), from the chemistry used for the synthesis of the final product (e.g., if the final product will be a nucleic acid with uridines substituted by 4-thiouridines, the set of optimization methods may be different than if 50% or 100% of uridines were replaced by pseudouridines).

The application of an initial rules set corresponding to the elected optimization methods introduces a modification in the candidate sequence, transforming it into an optimized sequence or a library of optimized sequences. These sequences can then be chemically synthesized and experimentally evaluated for a desired experimental property, e.g., expression level in a target tissue. The contribution of each one of the optimization methods to the improvement of the desired experimental property would be determined, for example, using principal component analysis. Data corresponding to the first optimization cycle would then be used as input for a multiparametric genetic algorithm, which would in turn yield a refined rule-set. Such a refined rule set could be reapplied to the candidate sequence, or to one or more of the sequences modified/optimized after the first iteration.

The process could be used iteratively, for example, until the desired experimental property reached a certain threshold (e.g., a certain level of protein expression in a target tissue), until a set number of iterations was reached (e.g., the optimization process may be stopped after n cycles), or until the optimization process converged and additional cycles of optimization resulted in improvement below a certain threshold (e.g., the optimization process may be stopped if an optimization cycle improved the desired experimental property to the point of diminishing returns).

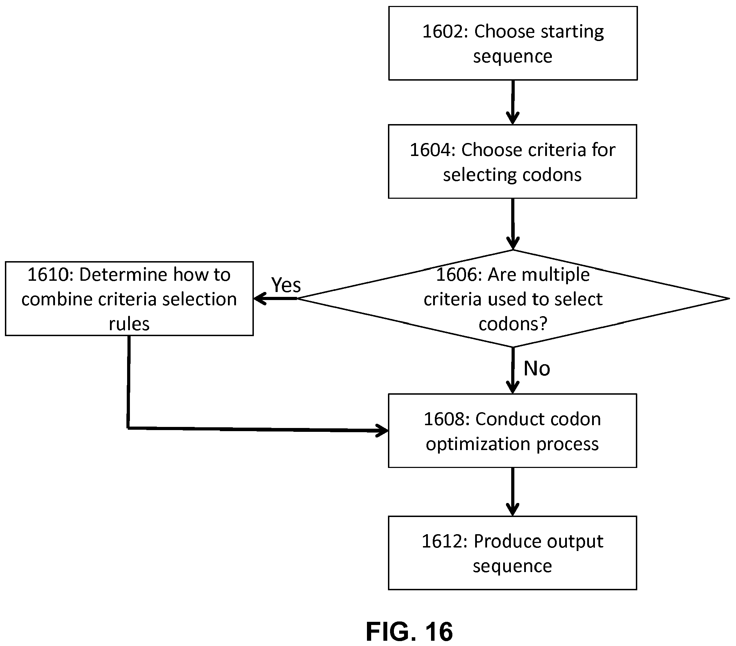

FIG. 16 illustrates a flowchart of a multiparametric method 1600 for codon optimization, according to an embodiment of the present invention. In block 1602, a starting sequence (i.e., a candidate sequence, for example a candidate nucleic acid sequence such as an mRNA) is chosen for optimization. The starting sequence may be any sequence of interest. For example, the starting sequence may be:
(i) a wild type amino acid sequence;
(ii) a wild type nucleotide sequence (e.g., an mRNA);
(iii) a non-wild type amino acid sequence (e.g., a mutated protein, a fusion protein, etc.); or,
(iv) a non-wild type nucleotide sequence (e.g., a mutated nucleic acid, a nucleic acid encoding a fusion protein or a chimeric protein, a chimeric nucleic acid, or a synthetic nucleic acid sequence such as a synthetic mRNA).

The starting sequence can be identified from various sources. For example, the starting sequence could be newly identified and sequenced in a laboratory, provided by a partner, obtained from the literature, provided by a customer, etc. The nucleotide sequence (e.g., an mRNA) can be one identified from a previous iteration of a codon optimization process different from the multiparametric methods disclosed herein. Alternatively, the nucleotide sequence (e.g., an mRNA) can be one identified from a previous iteration of the multiparametric methods disclosed herein. Such a sequence, i.e., a previously optimized sequence, may be identified as promising but in need of further optimization.

Once the starting sequence is selected, method 1600 moves to block 1604. In block 1604, one or more criteria for selecting codons is chosen. The selection process comprises two components:
(i) codon selection, e.g., how to select a certain codon from an ordered list; and,
(ii) codon ordering, e.g., how to order the list of codon per amino acid from which they are selected.

Criteria regarding how the codon is to be selected for optimization from that ordered list include (a) selection by positions, wherein the selected codon can be the first, the last (which is equivalent to inverting the sorting order of the list and then selecting the first), or the nth (i.e., any codon between the first and the last); (b) selection by pattern, which determines the selected codon for successive occurrences of an amino acid, and can be repeated throughout the optimization process as necessary; (c) random selection, (d) biased random selection, (e) strict rotation, or (f) combinations thereof.

If selection by pattern is applied, such pattern can be, for example, uniform (e.g., 2-2-2-2-2-2, which would be equivalent to selection by positions; blocks (e.g., 1-1-1-2-2-2); alternating (e.g., 1-2-1-2-1-2); or attempting to reflect a metric, e.g., codon frequency or recharging rate (for example, 1-1-1-1-2-2-2-3, wherein frequency is 1 > 2 > 3. If random biased selection is applied, the selection could be biased, for example, according to a metric, e.g., codon frequency or recharging rate (which can be an species specific, tissue type specific, or cell type specific recharging rate). Strict rotation, e.g., 1-2-3-1-2-3, would be in fact a variant of selection by pattern.

Independently of the method used, all the codons in the input sequence would be processed until all relevant codons in the sequence have been optimized.

The original codon can be kept in the input sequence, if the input sequence is a nucleic acid (e.g., an mRNA), which allows selective codon optimization on top of, e.g., a wild type sequence.

Criteria regarding how to order the list of codons per amino acid from which they are selected, i.e., the various ways that an amino acid sequence can be transformed into an ordered list of codons, include (a) ordering by nucleotide content (e.g., by A, C, G, U content or a combination thereof), (b) ordering by frequency, (c) ordering by recharging rate (which can be an species specific, tissue type specific, or cell type specific recharging rate), or combinations thereof.

When codons are ordered by nucleotide content, they can be sorted in ascending or descending order. In some aspects, codons can be ordered, for example, based on G content, GC content, or U content. This approach will typically result in many ties, because the total content of each codon is 0, 1, 2, or 3, and 1 and 2 tend to be the most common.

In another example, the codons may be ordered based on a frequency of each codon, e.g., frequencies in *Homo sapiens* if the input sequence is a human nucleic acid sequence. Codon frequency maps can be obtained, for example, from kazusa.or.jp. In a particular aspect, the codon map is a human codon frequency map, e.g., the human codon frequency map available at www.kazusa.or.jp/codon/cgi-bin/showcodon.cgi?species=9606, which is shown in TABLE 1.

**TABLE 1: Homo sapiens codon map. The map was calculated from 93,487 CD's, corresponding to 40662582 codons. Fields in the table correspond to [triplet] [frequency: per thousand] ([number of codon]). Coding GC 52.27% 1st letter GC 55.72% 2nd letter GC 42.54% 3rd letter GC 58.55%**

| UUU | UCU 15.2(618711) | UAU 12.2(495699) | UGU 10.6(430311) |
|---|---|---|---|
| 17.6(714298) | | | |
| UUC 20.3(824692) | UCC 17.7(718892) | UAC 15.3(622407) | UGC 12.6(513028) |
| UUA 7.7 (311881) | UCA 12.2(496448) | UAA 1.0( 40285) | UGA 1.6( 63237) |
| UUG 12.9(525688) | UCG 4.4(179419) | UAG 0.8( 32109) | UGG 13.2(535595) |
| CUU 13.2(536515) | CCU 17.5(713233) | CAU 10.9(441711) | CGU 4.5(184609) |
| CUC 19.6(796638) | CCC 19.8(804620) | CAC 15.1(613713) | CGC 10.4(423516) |
| CUA 7.2(290751) | CCA 16.9(688038) | CAA 12.3(501911) | CGA 6.2 (250760) |
| CUG 39.6(1611801) | CCG 6.9(281570) | CAG 34.2(1391973) | CGG 11.4(464485) |
| AUU 16.0(650473) | ACU 13.1(533609) | AAU 17.0(689701) | AGU 12.1(493429) |
| AUC 20.8(846466) | ACC 18.9(768147) | AAC 19.1(776603) | AGC 19.5(791383) |
| AUA 7.5(304565) | ACA 15.1(614523) | AAA 24.4(993621) | AGA 12.2(494682) |
| AUG 22.0(896005) | ACG 6.1(246105) | AAG 31.9(1295568) | AGG 12.0(486463) |
| GUU 11.0(448607) | GCU 18.4(750096) | GAU 21.8(885429) | GGU 10.8(437126) |
| GUC 14.5(588138) | GCC 27.7(1127679) | GAG 25.1(1020595) | GGC 22.2(903565) |
| GUA 7.1(287712) | GCA 15.8(643471) | GAA 29.0(1177632) | GGA 16.5(669873) |
| GUG 28.1(1143534) | GCG 7.4(299495) | GAG 39.6(1609975) | GGG 16.5(669768) |

In yet another example, the codons may be ordered based on codon recharging rates (which can be a species specific, tissue type specific, or cell type specific recharging rate), which can be measured directly or inferred via proxy measures from other data, for example, from codon frequencies.

Each of these exemplary ordering schemes can be used, for example, to order a full wild type set of synonymous codons (e.g., 6 choices for Arginine) or a deliberately chosen subset (e.g., using only 'CGA' and 'CGG' for Arginine). The same effect as a subset may be achieved using a pattern that only uses the first two codons, for example (e.g., 1-1-1-1-2-2-2-2).

In block 104, both the selection and ordering rules can be applied uniformly to each amino acid or differentially per amino acid, either different versions of the same rule or entirely different rules. That is, there can be different versions of the same rules for each amino acid, or there can be an entirely different set of rules for each amino acid. For example, the first codon in a synonymous group (e.g., the highest/lowest frequency codon, or the highest/lowest uridine content codon, or the codon with the fastest/slowest recharging rate in the group) could be used for all amino acids, and then the fourth codon would be used in the synonymous codon group for arginine. Or for example, the first codon could be used for all amino acids except for cysteine, glutamic acid, leucine, proline, arginine and serine, and for those use 1212 alternating patterns. The selection of specific amino acid groups can be based, for example, on position in the protein sequence (e.g., close to the N-terminus or C-terminus, or within n amino acids from the N- or C- terminus), proximity to a secondary structure element (e.g., location in a random coil region within n amino acids from an alpha helix), location within a certain secondary structure element (e.g., a random coil, alpha helix, beta strand, turn, etc.), possession of a certain physicochemical property (e.g., amino acid hydrophobicity, volume, aromaticity, polarity, charge, etc.), protein structure location (e.g., buried in the structure of the protein, surface location, interface between polypeptides in a homomeric or heteromeric protein), location relative to a certain functional site (e.g., proximity to an enzymatic active site, proximity to a cofactor binding site, proximity to a receptor recognition site, etc.).

In block 1606, it is determined whether multiple criteria are used to select a codon for optimization, or whether a single criteria is used. If using one single criterion to select a codon for optimization, the criterion will be applied equally across the whole sequence (global), and method 1600 proceeds to step 1608.

If it is determined in block 1606 that multiple criteria are used for codon selection, method 1600 proceeds to block 1610. In block 1610, a determination is made regarding how to combine the multiple criteria. For example, the criteria selection rules may be combined
(i) individually in disjoint subsequences, e.g., 1-30 and then 31+;
(ii) in ranked order for the same (sub)sequence (e.g., by GC content first and then frequency to break ties);
(iii) in a consensus scoring manner; or,
(iv) using any combination of the above.

Once a determination is made in block 1610, method 1600 proceeds to block 1608.

In block 1608, the codon optimization process is conducted for the sequence. Specific methods of optimizing selected codons are discussed in further detail below. The codon optimization process is iteratively conducted over the amino acids or codons of the input sequence. As part of this optimization process, the appropriate codon selection criteria (e.g., as identified in blocks 1606 or 1610) are applied at each position. It is possible to address many variants in a single iteration. For example, between 10 and 250 variants may be processed in a single iteration. In another example, more than 250 variants may be processed in a single iteration. The number of variants processed may be constrained, however, by capacity. It is noted that the number of possible synonymous nucleotide sequences for a typical protein sequence is larger than the number of atoms in the universe. Accordingly, such an optimization process must be performed by a computing device having sufficient capability to process such large amounts of data. Such processing cannot be performed manually.

In block 1612, an output sequence is produced. Each fully specified set of rules (e.g., selection criteria, sort criteria, and combination thereof) produces a single output sequence (except for random methods, which can produce many output sequences).

mRNA can then be synthesized with the sequence output in block 1612. Once the mRNA is made, it may be quality controlled ("QC'd") to confirm its integrity, and then tested. Testing may be conducted to confirm one or more of the following, for example: *in vitro* expression, *in vivo* expression, immunogenicity, stability, and efficacy (pharmacological effect). Data produced by the testing may be analyzed to detect patterns. It may also be possible to "score" the codon optimized sequences that are output in block 1610. Additionally, secondary structure (either predicted or experimentally determined) may be incorporated.

### III. Codon Optimization Methods

The present disclosure provides multiparametric nucleic acid optimization methods where a number of discrete optimization methods are integrated in a single model to predict the optimal sequence of a nucleic acid (e.g., an mRNA) according to a desired characteristic or set of desired characteristics, for example, expression efficacy of an mRNA in a target tissue or cell.

In some aspects, the present disclosure provides a number of codon optimization methods, which can be combined into a single model in order to optimize a candidate nucleic sequence (e.g., a mRNA), for example, to improve protein expression efficacy in a target tissue or cells.

Certain optimization methods which are an integral part of the multiparametric nucleic acid optimization methods disclosed herein are described in detail below. This list of methods is not comprehensive or limiting, thus, additional optimizations methods can be integrated in the multiparametric methods disclosed herein.

It will be appreciated that the design principles and rules described for each one of the optimized methods discussed below can be combined in many different ways, for example introducing a rare codon ramp in a certain region of the candidate nucleic acid sequence, followed by high G/C content optimization or uridine content optimization for other regions of the candidate nucleic acid sequence, as well as targeted nucleotide mutations to minimize secondary structure throughout the sequence or to eliminate deleterious motifs.

The choice of potential combinations of optimization methods can be, for example, dependent on the specific chemistry used to produce a synthetic mRNA. Such a choice can also depend on characteristics of the target protein encoded by the candidate nucleic acid sequence. In some aspects, such a choice can depend on the specific tissue or cell targeted by the optimized nucleic acid (e.g., a therapeutic synthetic mRNA).

The mechanisms of combining the optimization methods or design rules derived from the application and analysis of the optimization methods can be either simple or complex. For example, the combination can be:
(i) *Sequential*: Each optimization method or set of design rules applies to a different subsequence of the overall sequence, for example a ramp rule from 1 to 30 and then high frequency codons for the remainder of the sequence;
(ii) *Hierarchical*: Several optimization methods or sets of design rules are combined in a hierarchical, deterministic fashion. For example, use the most GC-rich codons, breaking ties (which are common) by choosing the most frequent of those codons.
(iii) *Multifactorial* / *Multiparametric*: Machine learning or other modeling techniques are used to design a single sequence that best satisfies multiple overlapping and possibly contradictory requirements. This approach would require the use of a computer applying a number of mathematical techniques, for example, genetic algorithms.

Ultimately, each one of these approaches can result in a specific set of rules which in many cases can be summarized in a single codon table, i.e., a sorted list of codons for each amino acid in the target protein, with a specific rule or set of rules indicating how to select a specific codon for each amino acid position.

In some aspects, the multiparametric nucleic acid optimization methods disclosed herein can be used to optimize the encoding sequences of proteins about 5, 10, 15, 20, 25, 30, 35, 40, 45, 50, 55, 60, 65, 70, 75, 80, 85, 90, 95, or 100 amino acids in length. In some aspects, they can be used to optimize the encoding sequences of proteins about 100, 110, 120, 130, 140, 150, 160, 170, 180, 190, 200, 210, 220, 230, 240, 250, 260, 270, 280, 290, 300, 310, 320, 330, 340, 350, 360, 370, 380, 390, 400, 410, 420, 430, 440, 450, 460, 470, 480, 490, 500, 510, 520, 530, 540, 550, 560, 570, 580, 590, 600, 610, 620, 630, 640, 650, 660, 670, 680, 690, 700, 710, 720, 730, 740, 750, 760, 770, 780, 790, 800, 810, 820, 830, 840, 850, 860, 870, 880, 890, 900, 910, 920, 930, 940, 950, 960, 970, 980, 990, or 1000 amino acids in length. In some aspects, they can be used to optimize the encoding sequences of proteins about 1100, 1200, 1300, 1400, 1500, 1600, 1700, 1800, 1900, 2000, 2100, 2200, 2300, 2400, 2500, 2600, 2700, 2800, 2900, 3000, 3100, 3200, 3300, 3400, 3500, 3600, 3700, 3800, 3900, 4000, 4100, 4200, 4300, 4400, 4500, 4600, 4700, 4800, 4900, 5000, 5100, 5200, 5300, 5400, 5500, 5600, 5700, 5800, 5900, 6000, 6100, 6200, 6300, 6400, 6500, 6600, 6700, 6800, 6900, 7000, 7100, 7200, 7300, 7400, 7500, 7600, 7700, 7800, 7900, 8000, 8100, 8200, 8300, 8400, 8500, 8600, 8700, 8800, 8900, 9000, 9100, 9200, 9300, 9400, 9500, 9600, 9700, 9800, 9900, 10000 amino acids in length.

### a. Ramps

Slowing down translation early in the sequence can streamline the progress of the ribosome later and overall increase translation rates. See, e.g., Gingold & Pilpel et al. (2011) Mol. Syst. Biol. 7:481. Having a slow "ramp", e.g., a stretch of rare codons within approximately the first 50 codons of an mRNA, serves as a late stage of translation initiation, forming an optimal and robust means to reduce ribosomal traffic jams, thus minimizing the cost of protein expression. See Tuller et al. (2010) Cell 141(2):344-54; Li & Qu (2013) PLoS One. 8(3):e59706, Shah et al. (2013) Cell 153:1589-1601.

The design of a ramp can be based on many different parameters to design a ramp, e.g., length, start position (see, e.g., Navon & Pilpel (2011) Genome Biology 12:R12, and Kudla et al. (2009) Science 324:255-258), metric (e.g., how to impart a property to the ramp such as slowness, which can depend on codon frequency, tRNA recharging rate, or some other measure), or profile (e.g., slowly ramping up or ramping down the speed, aiming for example, for a moderate slowing down rate throughout the ramp).

Accordingly, the present disclosure provides multiparametric nucleic acid optimization methods comprising the use of expression ramps. As used herein, the terms "ramp," "expression ramp," "ramp subsequence" and grammatical variants thereof refer to a nucleic acid subsequence in an optimized nucleic acid sequence, the translation speed of which is increased or decreased with respect to the translation speed of the corresponding subsequence in a candidate nucleic acid sequence. Thus, a ramp or ramp subsequence comprises a variable translation rate sequence with a translation rate that differs from a translation rate of the corresponding sequence in the wild type gene.

A candidate nucleic acid sequence can be optimized by modifying subsequences or appending subsequences (for example, a heterologous sequence covalently attached to the 5' or 3' end of the candidate nucleic acid sequence) that alter the translation kinetics of the candidate nucleic sequence. These regions with altered kinetics (i.e., ramps) can locally increase or decrease the translation speed, therefore preventing stoppages or bottlenecks in translation. For example, ramps that slow down translation can prevent stoppages in translation caused when the candidate nucleic acid contains an excess of codons corresponding to tRNAs with low concentrations in the expression system (e.g., low frequency codons or low tRNA recharge codons). Accordingly, translation can be improved by altering the candidate nucleic acid sequence to introduce codons with more abundant tRNAs or codons with faster recharging tRNAs (the recharging rates of which can be, for example, species specific, tissue type specific, or cell type specific).

In a particular aspects, ramps that slow down translation, e.g., ramps generated by modifying local or global G/C content (absolute or relative), G/C clustering, local or global uridine content, uridine clustering, or combinations thereof in a certain region of the candidate nucleic acid sequence, can prevent stoppages in translation which are caused when the candidate nucleic acid contains an excess of codons corresponding to tRNAs with low recharging rates (which can be an species specific, tissue type specific, or cell type specific recharging rates). In that case, the introduction of a ramp can slow translation sufficiently to allow the translation system to recharge tRNAs to a level that makes it possible for translation to proceed efficiently and without bottlenecks/stoppages. This strategy can be combined, for example, with the substitution of codons in the ramp region(s), in specific regions of the candidate nucleic acid sequence (e.g., regions with a certain secondary structure), or throughout the candidate nucleic acid sequence with codons corresponding to fast recharging tRNA (e.g., codons corresponding to tRNAs with a recharging rate that is higher than the recharging rate of the original codon in the candidate nucleic acid sequence). For a discussion of codon recharge-based optimization, see Section 3.f, *infra.*

In some aspects, ramps that slow down translation or speed up translation, e.g., ramps generated by modifying local or global G/C content (absolute or relative), G/C clustering, local or global uridine content, uridine clustering, codon composition based on tRNA recharging rates (which can be a species specific, tissue type specific, or cell type specific recharging rate), or combinations thereof, in a certain region of the candidate nucleic acid sequence, can improve protein folding. In that case, the introduction of a ramp can slow translation or speed up translation sufficiently for translation to proceed at an appropriate speed that is optimal for the correct folding of specific regions of the expressed protein.

In some aspects of the present disclosure, an optimized nucleic acid sequence generated according to the multiparametric optimization methods disclosed herein can comprise at least one ramp subsequence. In some aspects, the optimized nucleic acid sequence can comprise at least one, two, three, four, five, six, seven, eight, nine, or ten ramp subsequences. In some aspects, the optimized nucleic acid sequence comprises more than ten ramps subsequences.

Possible ramp designs include constructs with initial fast translation followed by slower translation for the reminder of the sequence, fast translation throughout most of the sequence and then slowing down at the end, or one or more fast or slow spots interspersed throughout the sequence.

In some aspects, a ramp subsequence can comprise at least 1, at least 2, at least 3, at least 4, at least 5, at least 6, at least 7, at least 8, at least 9, at least 10, at least 11, at least 12, at least 13, at least 14, at least 15, at least 16, at least 17, at least 18, at least 19, at least 20, at least 21, at least 22, at least 23, at least 24 or at least 25 consecutive codons. In some aspects, a ramp can comprise at least about 25, at least about 30, at least about 35, at least about 40, at least about 45, at least about 50, at least about 55, at least about 60, at least about 65, at least about 70, at least about 75, at least about 80, at least about 85, at least about 90, at least about 95, or at least about 100 consecutive codons. In some aspects, a ramp can comprise more than 100 consecutive codons. In some aspects, a ramp subsequence comprises between 1 and 5 codons, between 5 and 10 codons, between 10 and 15 codons, between 15 and 20 codons, between 20 and 25 codons, between 25 and 30 codons, between 30 and 35 codons, between 35 and 40 codons, between 40 and 45 codons, or between 45 and 50 codons.

In specific aspects, the ramp subsequence is 10 codons long (i.e., 10 amino acids long, or 30 nucleotides long). In other aspects, the ramp subsequence is 20 codons long. In yet another aspect, the ramp subsequence is 30 codons long.

A person skilled in the art would appreciate that the sizes of ramps, distances between ramps, locations of ramps, etc. provided herein using codons as units can also be expressed in terms of nucleobases, i.e., a ramp at least 3 codons in length is equivalent to a ramp at least 9 nucleobases in length.

In some aspects of the present disclosure, a ramp subsequence can be located at least 1, at least 2, at least 3, at least 4, at least 5, at least 6, at least 7, at least 8, at least 9, at least 10, at least 11, at least 12, at least 13, at least 14, at least 15, at least 16, at least 17, at least 18, at least 19, at least 20, at least 21, at least 22, at least 23, at least 24 or at least 25 codons from the 5' end of the optimized nucleic acid sequence. In other aspects, the ramp subsequence is located at least about 25, at least about 30, at least about 35, at least about 40, at least about 45, at least about 50, at least about 55, at least about 60, at least about 65, at least about 70, at least about 75, at least about 80, at least about 85, at least about 90, at least about 95, or at least about 100 codons from the 5' end of the optimized nucleic acid sequence. In other aspects, the ramp subsequence is at least about 100, at least about 150, at least about 200, at least about 250, at least about 300, at least about 350, at least about 400, at least about 450, at least about 500, at least about 550, at least about 600, at least about 650, at least about 700, at least about 750, at least about 800, at least about 850, at least about 900, at least about 950, or at least about 1000 codons from the 5' end of the optimized nucleic acid sequence. In some aspects, a ramp subsequence can be located more than 1000 codons from the 5' end of the optimized nucleic acid sequence.

In some aspects of the present disclosure, a ramp subsequence can be located at least 1, at least 2, at least 3, at least 4, at least 5, at least 6, at least 7, at least 8, at least 9, at least 10, at least 11, at least 12, at least 13, at least 14, at least 15, at least 16, at least 17, at least 18, at least 19, at least 20, at least 21, at least 22, at least 23, at least 24 or at least 25 codons from the 3' end of the optimized nucleic acid sequence. In other aspects, the ramp subsequence is located at least about 25, at least about 30, at least about 35, at least about 40, at least about 45, at least about 50, at least about 55, at least about 60, at least about 65, at least about 70, at least about 75, at least about 80, at least about 85, at least about 90, at least about 95, or at least about 100 codons from the 3' end of the optimized nucleic acid sequence. In other aspects, the ramp subsequence is at least about 100, at least about 150, at least about 200, at least about 250, at least about 300, at least about 350, at least about 400, at least about 450, at least about 500, at least about 550, at least about 600, at least about 650, at least about 700, at least about 750, at least about 800, at least about 850, at least about 900, at least about 950, or at least about 1000 codons from the 3' end of the optimized nucleic acid sequence. In some aspects, a ramp subsequence can be located more than 1000 codons from the 3' end of the optimized nucleic acid sequence.

In some aspects, the position of a ramp can be expressed in relative terms as a fraction of the length of the candidate nucleic acid sequence (e.g., an mRNA). In some aspects, a ramp disclosed herein can be centered (i.e., the central codon or central pair of codons in the ramp will be at that position) at a relative position about 0.01, 0.02, 0.03, 0.04, 0.05, 0.06, 0.07, 0.08, 0.09, 0.10, 0.11, 0.12, 0.13, 0.14, 0.15, 0.16, 0.17, 0.18, 0.19. 0.20. 0.21, 0.22, 0.23, 0.24, 0.25, 0.26, 0.27, 0.28, 0.29, 0.30, 0.31, 0.32, 0.33, 0.34, 0.35, 0.36, 0.37, 0.38, 0.39, 0.40, 0.41, 0.42, 0.43, 0.44, 0.45, 0.46, 0.47, 0.48, 0.49, 0.50, 0.51, 0.52, 0.53, 0.54, 0.55, 0.56, 0.57, 0.58, 0.59, 0.60, 0.61, 0.62, 0.63, 0.64, 0.65, 0.66, 0.67, 0.68, 0.69, 0.70, 0.71, 0.72, 0.73, 0.74, 0.75, 0.76, 0.77, 0.78, 0.79, 0.80, 0.81, 0.82, 0.83, 0.84, 0.85, 0.86, 0.87, 0.88, 0.89, 0.90, 0.91, 0.92, 0.93, 0.94, 0.95, 0.96, 0.97, 0.98, or 0.99, wherein 1 would be the total length of the candidate nucleic acid sequence. In some aspects, the ramp is centered at a relative position between about 0.01 and about 0.40. In other aspects, the ramp is centered at a relative position between about 0.10 and about 0.30. In other aspects, the ramp is centered at a relative position between about 0.15 and about 0.25. In some aspect the ramp is centered at a relative position at about 0.2.

In some aspects, the ramp subsequence is a speed-up subsequence. In other aspects, the ramp subsequence is a speed-down ramp subsequence. In some specific aspects, the optimized nucleic acid subsequence comprises at least two ramp subsequences.

As used herein, the term "speed-up ramp" is defined as a ramp subsequence with a translation speed that is higher that the translation speed of the corresponding subsequence in the candidate nucleic acid sequence. Similarly, a "speed-down ramp" is defined as a ramp subsequence with a translation speed that is lower than the translation speed of the corresponding subsequence in the candidate nucleic acid sequence.

In some aspects, both ramp subsequences are speed-up ramp subsequences. In other aspects, both ramps are speed-down ramp subsequences. In other aspects, a ramp subsequence is a speed-up ramp subsequence and a ramp subsequence is a speed-down ramp subsequence. In some aspects, when more than one ramp is present, two consecutive ramp subsequences are at least about 5, at least about 10, at least about 15, at least about 20, at least about 25, at least about 30, at least about 35, at least about 40, at least about 45, at least about 50, at least about 55, at least about 60, at least about 65, at least about 70, at least about 75, at least about 80, at least about 85, at least about 90, at least about 95, or at least about 100 codons apart in the optimized nucleic acid sequence.

In some aspects, two ramp subsequences are at least about 120, at least about 140, at least about 160, at least about 180, at least about 200, at least about 240, at least about 260, at least about 280, at least about 300, at least about 320, at least about 340, at least about 360, at least about 380, at least about 400, at least about 420, at least about 440, at least about 460, at least about 480, or at least about 500 codons apart in the optimized nucleic acid sequence. In certain aspects, two ramp subsequences are more than 500 codons apart in the optimized nucleic acid sequence.

In some aspects, the distance between ramps can be expressed as a function of the length of the candidate nucleic sequence (e.g., an mRNA). Thus, in some aspects, the distance between two ramps can be about 2%, about 3%, about 4%, about 5%, about 6%, about 7%, about 8%, about 9%, about 10%, about 11%, about 12%, about 13%, about 14%, about 15%, about 16%, about 17%, about 18%, about 19%, about 20%, about 21%, about 22%, about 23%, about 24%, about 25%, about 26%, about 27%, about 28%, about 29%, about 30%, about 31%, about 32%, about 33%, about 34%, about 35%, about 36%, about 37%, about 38%, about 39%, about 40%, about 41%, about 42%, about 43%, about 44%, about 45%, about 446%, about 47%, about 48%, about 49%, or about 50%. In some aspects, the distance between ramps is higher than 50% of the length of candidate nucleic acid sequence (e.g., an mRNA).

In some particular aspects, the optimized nucleic acid sequence comprises two speed-down ramps, one located close to the 5' end of the optimized nucleic acid sequence and a second ramp located close to the 3' end of the optimized nucleic acid sequence. In some aspects, the 5' terminal ramp and the 3' terminal ramp are located within 90 nucleobases (i.e., 30 codons) from the 5' end or the 3' end respectively. Thus, the effect of those ramps is to slow down the translation of a subsequence within the first 30 amino acids or last 30 amino acids of the translated protein product.

In other aspects, a speed-down ramp can be introduced in a region encoding a certain secondary structure element, for example, to facilitate the correct folding of a long alpha helix. Accordingly, in some aspects, a speed-down ramp can be introduced in a subsequence of a candidate nucleic acid sequence encoding an alpha helix if the length of such alpha helix is above a certain threshold. In some aspects, such a threshold is a length of about 20, about 25, about 30, about 35, about 40, about 45, about 50, about 55, about 60, about 65, about 70, about 75, about 80, about 85, about 90, about 95, or about 100 amino acids. In specific aspects, such a threshold is a length of 50 amino acids.

In some aspects, the translation speed of the speed-up ramp subsequence is at least 10% higher than the translation speed of the corresponding subsequence in the candidate nucleic acid sequence. In other aspects, the translation speed of a speed-up ramp subsequence is at least about 10%, at least about 15%, at least about 20%, at least about 25%, at least about 30%, at least about 35%, at least about 40%, at least about 45%, at least about 50%, at least about 55%, at least about 60%, at least about 65%, at least about 70%, at least about 75%, at least about 80%, at least about 85%, at least about 90%, at least about 95%, or at least about 100% higher than the translation speed of the corresponding subsequence in the candidate nucleic acid sequence. In some aspects, the translation speed of a speed-up ramp subsequence is at least 100% higher than the translation speed of the corresponding subsequence in the candidate nucleic acid sequence.

In some aspects, the translation speed of a speed-up ramp subsequence is at least about 2-fold, at least about 3-fold, at least about 4-fold, at least about 5-fold, at least about 6-fold, at least about 7-fold, at least about 8-fold, at least about 9-fold, or at least about 10-fold higher than the translation speed of the corresponding subsequence in the candidate nucleic acid sequence. In some aspects, the translation speed of a speed-up ramp subsequence is at least 10-fold higher than the translation speed of the corresponding subsequence in the candidate nucleic acid sequence.

In some aspects, the translation speed of the speed-down ramp subsequence is at least 10% lower than the translation speed of the corresponding subsequence in the candidate nucleic acid sequence. In other aspects, the translation speed of a speed-down ramp subsequence is at least about 10%, at least about 15%, at least about 20%, at least about 25%, at least about 30%, at least about 35%, at least about 40%, at least about 45%, at least about 50%, at least about 55%, at least about 60%, at least about 65%, at least about 70%, at least about 75%, at least about 80%, at least about 85%, at least about 90%, at least about 95%, or at least about 100% lower than the translation speed of the corresponding subsequence in the candidate nucleic acid sequence. In some aspects, the translation speed of a speed-down ramp subsequence is at least 100% lower than the translation speed of the corresponding subsequence in the candidate nucleic acid sequence.

In some aspects, the translation speed of a speed-down ramp subsequence is at least about 2-fold, at least about 3-fold, at least about 4-fold, at least about 5-fold, at least about 6-fold, at least about 7-fold, at least about 8-fold, at least about 9-fold, or at least about 10-fold lower than the translation speed of the corresponding subsequence in the candidate nucleic acid sequence. In some aspects, the translation speed of a speed-down ramp subsequence is at least 10-fold lower than the translation speed of the corresponding subsequence in the candidate nucleic acid sequence.

In some aspects, the ramp subsequence is a homologous ramp subsequence, i.e., a subsequence of the candidate nucleic acid sequence has been modified to generate a speed-up or a speed-down ramp, e.g., by modifying local or global G/C content (absolute or relative), modifying G/C clustering, modifying local or global uridine content, modifying uridine clustering, modifying codon composition based on tRNA recharging rates (which can be a species specific, tissue type specific, or cell type specific recharging rates), or combinations thereof.

In other aspects, the ramp subsequence is a heterologous ramp subsequence, i.e., a subsequence not present in the candidate nucleic acid subsequence which has been appended to the 5' or 3' terminus of the candidate nucleic acid sequence. In some aspects, the heterologous ramp subsequence is at least about 5, at least about 10, at least about 15, at least about 20, at least about 25, at least 30, at least about 35, at least about 40, at least about 45, or at least about 50 codons in length. In some aspects, the heterologous ramp sequence can be more than 50 codons in length. In some aspects, a heterologous ramp sequence can be appended to the candidate nucleic acid sequence using molecular biology techniques known in the art, e.g., enzymatic ligation. In other aspects, a heterologous ramp sequence can be chemically synthesized before the 5' end or after the 3' end of the candidate nucleic acid sequence.

In some aspects, the ramp subsequence is generated by modifying the GC content (absolute or relative) of a subsequence in the candidate nucleic acid sequence. Accordingly, in some aspects, the ramp subsequence has a GC content (absolute or relative) at least about 5%, at least about 10%, at least about 15%, at least about 20%, at least about 25%, at least about 30%, at least about 35%, at least about 40%, at least about 45%, at least about 50%, at least about 55%, at least about 60%, at least about 65%, at least about 70%, at least about 75%, at least about 80%, at least about 85%, at least about 90%, at least about 95%, or at least about 100% higher than the GC content (absolute or relative) of the corresponding subsequence in the candidate nucleic acid sequence.

In other aspects, the ramp subsequence has a GC content (absolute or relative) at least about 5%, at least about 10%, at least about 15%, at least about 20%, at least about 25%, at least about 30%, at least about 35%, at least about 40%, at least about 45%, at least about 50%, at least about 55%, at least about 60%, at least about 65%, at least about 70%, at least about 75%, at least about 80%, at least about 85%, at least about 90%, at least about 95%, or at least about 100% lower than the GC content (absolute or relative) of the corresponding subsequence in the candidate nucleic acid sequence.

In some aspects, the ramp subsequence is generated by modifying the overall uridine content (absolute or relative) and/or uridine patterns (clustering) of a subsequence in the candidate nucleic acid sequence. Accordingly, in some aspects, the ramp subsequence has a uridine (U) content (absolute or relative) at least about 5%, at least about 10%, at least about 15%, at least about 20%, at least about 25%, at least about 30%, at least about 35%, at least about 40%, at least about 45%, at least about 50%, at least about 55%, at least about 60%, at least about 65%, at least about 70%, at least about 75%, at least about 80%, at least about 85%, at least about 90%, at least about 95%, or at least about 100% higher than the uridine (U) content (absolute or relative) of the corresponding subsequence in the candidate nucleic acid sequence.

In some aspects, the ramp subsequence is generated by modifying the overall uridine content and/or uridine patterns (clustering) of a subsequence in the candidate nucleic acid sequence. Accordingly, in some aspects, the ramp subsequence has a uridine (U) content (absolute or relative) at least about 5%, at least about 10%, at least about 15%, at least about 20%, at least about 25%, at least about 30%, at least about 35%, at least about 40%, at least about 45%, at least about 50%, at least about 55%, at least about 60%, at least about 65%, at least about 70%, at least about 75%, at least about 80%, at least about 85%, at least about 90%, at least about 95%, or at least about 100% lower than the uridine (U) content (absolute or relative) of the corresponding subsequence in the candidate nucleic acid sequence.

In some specific aspects, the protein sequence encoded by the ramp subsequence has an alpha-helical, beta-sheet, or random coil secondary structure. In other aspects, the protein sequence encoded by the ramp subsequence corresponds to an interface region or transition region between two secondary structure elements, i.e., the ramp subsequence encodes at least two types of protein structure secondary conformations. In such cases, the presence of a speed-down ramp would facilitate the correct folding of the protein product by slowing down the translation rate when a certain protein secondary structure transitions to a different secondary structure. Thus, in some aspects, the protein sequence encoded by the ramp subsequence comprises amino acid sequences, for example, with (i) alpha-helix and beta strand secondary structure; (ii) alpha-helix and random coil secondary structure; (iii) beta strand and random coil secondary structure; (iv) alpha-helix, beta strand, and random coil secondary structure, etc.

A person skilled in the art would understand that there are numerous types of protein secondary structures, such as 3-turn helices (DSSP: G), 4-turn helices (DSSP: H), 5-turn helices (DSSP: I), hydrogen bonded turns (DSSP: T), extended strands in parallel and/or antiparallel beta-sheet conformation (DSSP: E), beta bridged (DSSP:B), bends (DSSP: S), or random coil (DSSP: C). Accordingly, in some aspects, the protein sequence encoded by the ramp subsequence comprises amino acid sequences corresponding to any binary combination of secondary structures known in the art, e.g., a ramp subsequence could comprise codons encoding for amino acids in a 3-turn helix (DSSP: G) conformation and amino acids in a bends (DSSP: S) conformation.

In some specific aspects, the translation of specific secondary structure elements is optimized, e.g., the translation speed is adjusted to facilitate the correct folding of the protein product, by engineering speed-up ramps or speed-down ramps according to the occurrence of a particular secondary structure element. For example, in some aspects the translation can be slowed down in random coil regions via the introduction of speed-down ramps, whereas the translation of helical and/or beta strand regions can be kept at the native translation speed or can be sped up via the introduction of speed-up ramps.

In other aspects, the translation can be slowed down at the interfacial regions between secondary structure elements, e.g., random coil to alpha helix, alpha helix to random coil, random to beta strand, or beta strand to random coil, via the introduction of speed-down ramps, whereas the translation speed within secondary structure elements (e.g., non-interface region of an alpha helix) can be kept at the native translation speed or can be sped up via the introduction of speed-up ramps.

In some aspects, an interface region comprises 1, 2, 3, 4, 5, 6, 7, 8, 9, or 10 codons. In some aspects, an interface regions comprises several codons encoding part of a first secondary structure element and several codons encoding part of a second secondary structure element. For example, the interface regions between a random coil region and an alpha helical region could be, for example, 8 codons in length, and comprise 4 codons encoding random coil amino acids and 4 codons encoding alpha helical amino acids. In other aspects, an interface region comprises several codons preceding or being part of the secondary structure element. Thus, for example, the interface regions between a random coil region and an alpha helical region could be, for example, 4 codons in length and comprise 4 codons encoding random coil amino acids preceding the alpha helix, or it could be 4 codons in length and comprise the first 4 codons encoding alpha helical amino acids.

It should be noted that in some aspects, a ramp may be ineffective or even deleterious to the expression of some protein. It those specific cases, ramp design would not be included as one of the optimization methods in the multiparametric methods disclosed herein.

See also U.S. Publ. Nos. US20130203113, US20080046192, US20120329091, US20130149699; and Int'l. Publ. No. WO2014142453, all of which are incorporated herein by reference in their entireties.

### b. Limited Codon Set Optimization

The present disclosure provides multiparametric nucleic acid optimization methods which comprise the use of optimized codon sets. In some particular aspects, such optimized codon sets are limited codon sets, e.g., codon sets wherein less than the native number of codons is used to encode the 20 natural amino acids, a subset of the 20 natural amino acids, or an expanded set of amino acids including, for example, non-natural amino acids.

A codon set may be optimized by reducing the codon number, by replacing natural codons with codons having unnatural bases, expanding the codon number to incorporate non-natural amino acids, or even introducing codons that have lengths different than 3. For example, 4 base codons are disclosed in Taira et al. (2005) J. Biosci. Bioeng. 99:473-6; and 5 base codons are disclosed in Hohsaka et al. (2001) Nucl. Acids Res. 29:3646-3651), both of which are herein incorporated by reference in their entireties.

The genetic code is highly similar among all organisms and can be expressed in a simple table with 64 entries which would encode the 20 standard amino acids involved in protein translation plus start and stop codons. The genetic code is degenerate, i.e., in general, more than one codon specifies each amino acid. For example, the amino acid leucine is specified by the UUA, UUG, CUU, CUC, CUA, or CUG codons, while the amino acid serine is specified by UCA, UCG, UCC, UCU, AGU, or AGC codons (difference in the first, second, or third position). Native genetic codes comprise 62 codons encoding naturally occurring amino acids. Thus, in some aspects of the methods disclosed herein optimized codon sets (genetic codes) comprising less than 62 codons to encode 20 amino acids can comprise 61, 60, 59, 58, 57, 56, 55, 54, 53, 52, 51, 50, 49, 48, 47, 46, 45, 44, 43, 42, 41, 40, 39, 38, 37, 36, 35, 34, 33, 32, 31, 30, 30, 29, 28, 27, 26, 25, 24, 23, 22, 21, or 20 codons. In some aspects, the limited codon set comprises less than 20 codons. For example, if a protein contains less than 20 types of amino acids, such protein could be encoded by a codon set with less than 20 codons. Accordingly, in some aspects, an optimized codon set comprises as many codons as different types of amino acids are present in the protein encoded by the candidate nucleic acid sequence. In some aspects, the optimized codon set comprises 19, 18, 17, 16, 15, 14, 13, 12, 11, 10, 9, 8, 7, 6, 5, 4, 3, 2 or even 1 codon.

In some aspects, at least one amino acid selected from the group consisting of Ala, Arg, Asn, Asp, Cys, Gln, Glu, Gly, His, Ile, Leu, Lys, Phe, Pro, Ser, Thr, Tyr, and Val, i.e., amino acids which are naturally encoded by more than one codon, is encoded with less codons than the naturally occurring number of synonymous codons. For example, in some aspects, Ala can be encoded in the optimized nucleic acid sequence by 3, 2 or 1 codons; Cys can be encoded in the optimized nucleic acid sequence by 1 codon; Asp can be encoded in the optimized nucleic acid sequence by 1 codon; Glu can be encoded in the optimized nucleic acid sequence by 1 codon; Phe can be encoded in the optimized nucleic acid sequence by 1 codon; Gly can be encoded in the optimized nucleic acid sequence by 3 codons, 2 codons or 1 codon; His can be encoded in the optimized nucleic acid sequence by 1 codon; Ile can be encoded in the optimized nucleic acid sequence by 2 codons or 1 codon; Lys can be encoded in the optimized nucleic acid sequence by 1 codon; Leu can be encoded in the optimized nucleic acid sequence by 5 codons, 4 codons, 3 codons, 2 codons or 1 codon; Asn can be encoded in the optimized nucleic acid sequence by 1 codon; Pro can be encoded in the optimized nucleic acid sequence by 3 codons, 2 codons, or 1 codon; Gln can be encoded in the optimized nucleic acid sequence by 1 codon; Arg can be encoded in the optimized nucleic acid sequence by 5 codons, 4 codons, 3 codons, 2 codons, or 1 codon; Ser can be encoded in the optimized nucleic acid sequence by 5 codons, 4 codons, 3 codons, 2 codons, or 1 codon; Thr can be encoded in the optimized nucleic acid sequence by 3 codons, 2 codons, or 1 codon; Val can be encoded in the optimized nucleic acid sequence by 3 codons, 2 codons, or 1 codon; and, Tyr can be encoded in the optimized nucleic acid sequence by 1 codon.

In some aspects, at least one amino acid selected from the group consisting of Ala, Arg, Asn, Asp, Cys, Gln, Glu, Gly, His, Ile, Leu, Lys, Phe, Pro, Ser, Thr, Tyr, and Val, i.e., amino acids which are naturally encoded by more than one codon, is encoded by a single codon in the limited codon set.

In some specific aspects, the optimized nucleic acid sequence is a DNA and the limited codon set consists of 20 codons, wherein each codon encodes one of 20 amino acids. In some aspects, the optimized nucleic acid sequence is a DNA and the limited codon set comprises at least one codon selected from the group consisting of GCT, GCC, GCA, and GCG; at least a codon selected from the group consisting of CGT, CGC, CGA, CGG, AGA, and AGG; at least a codon selected from AAT or ACC; at least a codon selected from GAT or GAC; at least a codon selected from TGT or TGC; at least a codon selected from CAA or CAG; at least a codon selected from GAA or GAG; at least a codon selected from the group consisting of GGT, GGC, GGA, and GGG; at least a codon selected from CAT or CAC; at least a codon selected from the group consisting of ATT, ATC, and ATA; at least a codon selected from the group consisting of TTA, TTG, CTT, CTC, CTA, and CTG; at least a codon selected from AAA or AAG; an ATG codon; at least a codon selected from TTT or TTC; at least a codon selected from the group consisting of CCT, CCC, CCA, and CCG; at least a codon selected from the group consisting of TCT, TCC, TCA, TCG, AGT, and AGC; at least a codon selected from the group consisting of ACT, ACC, ACA, and ACG; a TGG codon; at least a codon selected from TAT or TAC; and, at least a codon selected from the group consisting of GTT, GTC, GTA, and GTG.

In other aspects, the optimized nucleic acid sequence is an RNA (e.g., an mRNA) and the limited codon set consists of 20 codons, wherein each codon encodes one of 20 amino acids. In some aspects, the optimized nucleic acid sequence is an RNA and the limited codon set comprises at least one codon selected from the group consisting of GCU, GCC, GCA, and GCG; at least a codon selected from the group consisting of CGU, CGC, CGA, CGG, AGA, and AGG; at least a codon selected from AAU or ACC; at least a codon selected from GAU or GAC; at least a codon selected from UGU or UGC; at least a codon selected from CAA or CAG; at least a codon selected from GAA or GAG; at least a codon selected from the group consisting of GGU, GGC, GGA, and GGG; at least a codon selected from CAU or CAC; at least a codon selected from the group consisting of AUU, AUC, and AUA; at least a codon selected from the group consisting of UUA, UUG, CUU, CUC, CUA, and CUG; at least a codon selected from AAA or AAG; an AUG codon; at least a codon selected from UUU or UUC; at least a codon selected from the group consisting of CCU, CCC, CCA, and CCG; at least a codon selected from the group consisting of UCU, UCC, UCA, UCG, AGU, and AGC; at least a codon selected from the group consisting of ACU, ACC, ACA, and ACG; a UGG codon; at least a codon selected from UAU or UAC; and, at least a codon selected from the group consisting of GUU, GUC, GUA, and GUG.

In some aspects, the DNA limited codon set is:
(a) TTC, TTG, CTG, ATC, ATG, GTG, AGC, CCC, ACC, GCC, TAC, CAC, CAG, AAC, AAG, GAG, TGC, TGG, AGG, GGC;
(b) TTT, CTA, ATA, ATG, GTA, TCG, CCG, ACG, GCG, TAT, CAT, CAA, AAT, AAA, GAT, GAA, TGT, TGG, CGT, GGT;
(c) TTC, CTV, ATM, ATG, GTV, AGC, CCV, ACV, GCV, TAC, CAC, CAR, AAC, AAR, GAC, GAR, TGC, TGG, CGV, GGV; or,
(d) TTC, CTV, ATM, ATG, GTV, AGC, CCV, ACV, GCV, TAC, CAC, CAR, AAC, AAR, GAC, GAR, TGC, TGG, AGR, GGV.

In some aspects, the RNA limited codon set is:
(a) UUC, UUG, CUG, AUC, AUG, GUG, AGC, CCC, ACC, GCC, UAC, CAC, CAG, AAC, AAG, GAG, UGC, UGG, AGG, GGC;
(b) UUU, CUA, AUA, AUG, GUA, UCG, CCG, ACG, GCG, UAU, CAU, CAA, AAU, AAA, GAU, GAA, UGU, UGG, CGU, GGU;
(c) UUC, CUV, AUM, AUG, GUV, AGC, CCV, ACV, GCV, UAC, CAC, CAR, AAC, AAR, GAC, GAR, UGC, UGG, CGV, GGV; or,
(d) UUC, CUV, AUM, AUG, GUV, AGC, CCV, ACV, GCV, UAC, CAC, CAR, AAC, AAR, GAC, GAR, UGC, UGG, AGR, GGV.

In some specific aspects, the limited codon set has been optimized for *in vivo* expression of an optimized nucleic acid sequence (e.g., a synthetic mRNA) following administration to a certain tissue or cell.

In some aspects, the optimized codon set comprises at least one codon consisting of more than 3 nucleobases, for example, 4 nucleobases or 5 nucleobases. In some aspects, the optimized codon set comprises at least one codon encoding an unnatural amino acid (i.e., a non-canonical amino acid). See, e.g., Liu et al. (1997) Proc. Natl. Acad Sci. USA 94:10092-10097; Link et al. (2003) Curr. Opin. Biotechnol. 14:603-609; Sakamoto et al. (2002) Nucl. Acids Res. 30:4692-4699; Zhang et al. (2013) Curr. Opin. Struct. Biol. 23:581-587; Ma (2003) Chem. Today, 65; Dougherty (2000) Curr Opin Chem Biol. 6:645; Kitamura et al. (2005) Chem. Int. Ed. 44: 1549; Ooi et al. (2007) Aldrichimica Acta 40:77; Rutjes et al. (2005) J. Org. Biol. Chem. 3:3435; Rutjes et al. (2000) J. Chem. Soc., Perkin Trans. 1:4197; Vignola (2003) Am. Chem. Soc. 125:450; Dalko (2004) Chem. Int. Ed. 43:5138; Lelais (2004) Biopolymers 76:206; and Seebach et al. (2004) Chem. & Biodiv. 1:1 11 1, all of which are herein incorporated by reference in their entireties.

In some aspects, the optimized codon set comprises at least one codon comprising an unnatural nucleobase. In some aspects, the unnatural nucleobase is an adenosine analog. In other aspects, the unnatural nucleobase in a cytidine analog. In other aspects, the unnatural nucleobase is a thymidine analog. In other aspects, the unnatural nucleobase is a guanidine analog. In yet other aspects, the unnatural nucleobase is a uridine analog.

In some specific aspects, the optimized codon set comprises at least one codon comprising a nucleobase selected from the group consisting of 5-trifluoromethyl-cytosine, 1-methyl-pseudo-uracil, 5-hydroxymethyl-cytosine, 5-bromo-cytosine, 5-methoxy-uracil, or 5-methyl-cytosine. See, for example, International Publication Nos. WO2014093924A1 and WO2013052523 A1, which are herein incorporated by reference in their entireties. A detailed description of possible chemical modifications of nucleobases is included in Section IV of this application, *infra.*

In some aspects, the optimized codon set (e.g., a 20 codon set encoding 20 amino acids) complies at least with one of the following properties:
(i) the optimized codon set has a higher average G/C content than the original or native codon set; or,
(ii) the optimized codon set has a lower average U content than the original or native codon set; or,
(iii) the optimized codon set is composed of codons with the highest frequency; or,
(iv) the optimized codon set is composed of codons with the lowest frequency; or,
(v) the optimized codon set is composed of codons with the highest tRNA recharging rate (which can be a species specific, tissue type specific, or cell type specific recharging rate); or,
(vi) the optimized codon set is composed of codons with lowest tRNA recharging rate (which can be a species specific, tissue type specific, or cell type specific recharging rate); or,
(vii) a combination thereof.

In some specific aspects, at least one codon in the optimized codon set has the second highest, the third highest, the fourth highest, the fifth highest or the sixth highest frequency in the synonymous codon set. In some specific aspects, at least one codon in the optimized codon has the second lowest, the third lowest, the fourth lowest, the fifth lowest, or the sixth lowest frequency in the synonymous codon set.

As used herein, the term "native codon set" refers to the codon set used natively by the source organism to encode the candidate nucleic acid sequence. As used herein, the term "original codon set" refers to the codon set used to encode the candidate nucleic acid sequence before the beginning of multiparametric codon optimization, or to a codon set used to encode an optimized variant of the candidate nucleic acid sequence at the beginning of a new optimization iteration when multiparametric codon optimization is applied iteratively or recursively.

In some aspects, 5%, 10%, 15%, 20%, 25%, 30%, 35%, 40%, 45%, 50%, 55%, 60%, 65%, 70%, 75%, 80%, 85%, 90%, 95% or 100% of codons in the codon set are those with the highest frequency. In other aspects, 5%, 10%, 15%, 20%, 25%, 30%, 35%, 40%, 45%, 50%, 55%, 60%, 65%, 70%, 75%, 80%, 85%, 90%, 95% or 100% of codons in the codon set are those with the lowest frequency.

In some aspects, 5%, 10%, 15%, 20%, 25%, 30%, 35%, 40%, 45%, 50%, 55%, 60%, 65%, 70%, 75%, 80%, 85%, 90%, 95% or 100% of codons in the codon set are those with the highest tRNA recharging rate (which can be a species specific, tissue type specific, or cell type specific recharging rate). In some aspects, 5%, 10%, 15%, 20%, 25%, 30%, 35%, 40%, 45%, 50%, 55%, 60%, 65%, 70%, 75%, 80%, 85%, 90%, 95% or 100% of codons in the codon set are those with the lowest tRNA recharging rate (which can be a species specific, tissue type specific, or cell type specific recharging rate).

In some aspects, 5%, 10%, 15%, 20%, 25%, 30%, 35%, 40%, 45%, 50%, 55%, 60%, 65%, 70%, 75%, 80%, 85%, 90%, 95% or 100% of codons in the codon set are those with the highest uridine content. In some aspects, 5%, 10%, 15%, 20%, 25%, 30%, 35%, 40%, 45%, 50%, 55%, 60%, 65%, 70%, 75%, 80%, 85%, 90%, 95% or 100% of codons in the codon set are those with the lowest uridine content.

In some aspects, the average G/C content (absolute or relative) of the codon set is 5%, 10%, 15%, 20%, 25%, 30%, 35%, 40%, 45%, 50%, 55%, 60%, 65%, 70%, 75%, 80%, 85%, 90%, 95% or 100% higher than the average G/C content (absolute or relative) of the original codon set. In some aspects, the average G/C content (absolute or relative) of the codon set is 5%, 10%, 15%, 20%, 25%, 30%, 35%, 40%, 45%, 50%, 55%, 60%, 65%, 70%, 75%, 80%, 85%, 90%, 95% or 100% lower than the average G/C content (absolute or relative) of the original codon set.

In some aspects, the uridine content (absolute or relative) of the codon set is 5%, 10%, 15%, 20%, 25%, 30%, 35%, 40%, 45%, 50%, 55%, 60%, 65%, 70%, 75%, 80%, 85%, 90%, 95% or 100% higher than the average uridine content (absolute or relative) of the original codon set. In some aspects, the uridine content (absolute or relative) of the codon set is 5%, 10%, 15%, 20%, 25%, 30%, 35%, 40%, 45%, 50%, 55%, 60%, 65%, 70%, 75%, 80%, 85%, 90%, 95% or 100% lower than the average uridine content (absolute or relative) of the original codon set.

See also, U.S. Publ. No. US20110082055, Int'l. Publ. No. WO2000018778, all of which are incorporated herein by reference in their entireties.

### c. Uridine Content Optimization

The presence of local high concentrations of uridine in a certain nucleic acid sequence can have detrimental effects on translation, e.g., slow or prematurely terminated translation, especially when modified uridine analogs are used in the production of synthetic mRNAs. Furthermore, high uridine content can also reduce the in vivo half-life of synthetic mRNAs due to TLR activation. Accordingly, the present disclosure provides multiparametric nucleic acid optimization methods comprising at least one uridine content optimization step. Such a step comprises, e.g., substituting at least one codon in the candidate nucleic acid with an alternative codon to generate a uridine-modified sequence, wherein the uridine-modified sequence has at least one of the following properties:
(i) increase or decrease in global uridine content; or,
(ii) increase or decrease in local uridine content (i.e., changes in uridine content are limited to specific subsequences); or,
(iii) changes in uridine distribution without altering the global uridine content; or,
(iv) changes in uridine clustering (e.g., number of clusters, location of clusters, or distance between clusters); or,
(v) combinations thereof.

In some aspects, the optimization process comprises reducing the global uridine content, i.e., reducing the percentage of uridine nucleobases in the optimized nucleic acid sequence with respect to the percentage of uridine nucleobases in the candidate nucleic acid sequence. For example, 30% of nucleobases may be uridines in the candidate sequence and 10% of nucleobases may be uridines in the optimized nucleic acid sequence.

In other aspects, the optimization process comprises reducing the local uridine content in specific regions of the candidate nucleic acid sequence, i.e., reducing the percentage of uridine nucleobases in a subsequence of the optimized nucleic acid sequence with respect to the percentage of uridine nucleobases in the corresponding subsequence of the candidate nucleic acid sequence. For example, the candidate nucleic acid sequence may have a 5'-end region (e.g., 30 codons) with a local uridine content of 30%, and the uridine content in that same region could be reduced to 10% in the optimized nucleic acid sequence.

In specific aspects, codons are replaced in the candidate nucleic acid sequence to reduce or modify, for example, the number, size, location, or distribution of uridine clusters that could have deleterious effects on protein translation. Although as a general rule it is desirable to reduce the uridine content of the candidate nucleic acid sequence, in certain aspects the uridine content, and in particular the local uridine content, of some subsequences of the candidate nucleic acid sequence can be increased when slow-recharging codons are replaced with fast-recharging codons (or vice versa), or when substituting codons to generate a ramp.

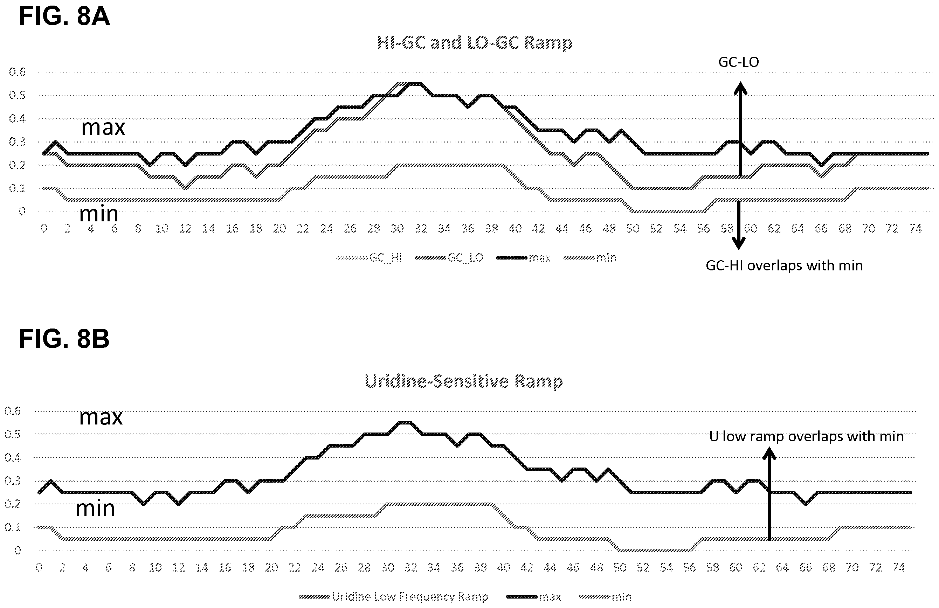

The reduction of uridine content to avoid adverse effects on translation can be done in combination with other optimization methods disclosed here to achieve other design goals. For example, uridine content optimization can be combined with ramp design, since using the rarest codons for most amino acids will, with a few exceptions, reduce the U content. See, e.g., FIG. 8.

In some aspects, the uridine-modified sequence is designed to induce a lower Toll-Like Receptor (TLR) response when compared to the candidate nucleic acid sequence. Several TLRs recognize and respond to nucleic acids. Double-stranded (ds)RNA, a frequent viral constituent, has been shown to activate TLR3. See Alexopoulou et al. (2001) Nature, 413:732-738 and Wang et al. (2004) Nat. Med., 10:1366-1373. Single-stranded (ss)RNA activates TLR7. See Diebold et al. (2004) Science 303 :1529-1531. RNA oligonucleotides, for example RNA with phosphorothioate internucleotide linkages, are ligands of human TLR8. See Heil et al. (2004) Science 303:1526-1529. DNA containing unmethylated CpG motifs, characteristic of bacterial and viral DNA, activate TLR9. See Hemmi et al. (2000) Nature, 408: 740-745.

As used herein, the term "TLR response" is defined as the recognition of single-stranded RNA by a TLR7 receptor, and in some aspects encompasses the degradation of the RNA and/or physiological responses caused by the recognition of the single-stranded RNA by the receptor. Methods to determine and quantitate the binding of an RNA to a TLR7 are known in the art. Similarly, methods to determine whether an RNA has triggered a TLR7-mediated physiological response (e.g., cytokine secretion) are well known in the art. In some aspects, a TLR response can be mediated by TLR3, TLR8, or TLR9 instead of TLR7.

Suppression of TLR7-mediated response can be accomplished via nucleoside modification. RNA undergoes over hundred different nucleoside modifications in nature (see the RNA Modification Database, available at mods.rna.albany.edu). Human rRNA, for example, has ten times more pseudouridine (Ψ) and 25 times more 2'-O-methylated nucleosides than bacterial rRNA. Bacterial mRNA contains no nucleoside modifications, whereas mammalian mRNAs have modified nucleosides such as 5-methylcytidine (m5C), N6-methyladenosine (m6A), inosine and many 2'-O-methylated nucleosides in addition to N7-methylguanosine (m7G).

Uridine and ribose, the two defining features of RNA, are both necessary and sufficient for TLR7 stimulation, and short single-stranded RNA (ssRNA) act as TLR7 agonists in a sequence-independent manner as long as they contain several uridines in close proximity. See Diebold et al. (2006) Eur. J. Immunol. 36:3256-3267, which is herein incorporated by reference in its entirety. Accordingly, one or more of the optimization methods used in the multiparametric codon optimization method disclosed herein comprises reducing the uridine content (locally and/or locally) and/or reducing or modifying uridine clustering to reduce or to suppress a TLR7-mediated response.

In some aspects, the TLR response (e.g., a response mediated by TLR7) caused by the uridine-modified sequence is at least about 10%, at least about 15%, at least about 20%, at least about 25%, at least about 30%, at least about 35%, at least about 40%, at least about 45%, at least about 50%, at least about 55%, at least about 60%, at least about 65%, at least about 70%, at least about 75%, at least about 80%, at least about 85%, at least about 90%, at least about 95%, or at least 100% lower than the TLR response caused by the candidate nucleic acid sequence.

In some aspects, the TLR response caused by the candidate nucleic acid is at least about 1-fold, at least about 1.1-fold, at least about 1.2-fold, at least about 1.3-fold, at least about 1.4-fold, at least about 1.5-fold, at least about 1.6-fold, at least about 1.7-fold, at least about 1.8-fold, at least about 1.9-fold, at least about 2-fold, at least about 3-fold, at least about 4-fold, at least about 5-fold, at least about 6-fold, at least about 7-fold, at least about 8-fold, at least about 9-fold, or at least about 10-fold higher than the TLR response caused by the uridine-modified sequence.

In some aspects, the uridine content (average global uridine content) (absolute or relative) of the uridine-modified sequence is higher than the uridine content (absolute or relative) of the candidate nucleic acid sequence. Accordingly, in some aspects, the uridine-modified sequence contains at least about 5%, at least about 10%, at least about 15%, at least about 20%, at least about 25%, at least about 30%, at least about 35%, at least about 40%, at least about 45%, at least about 50%, at least about 55%, at least about 60%, at least about 65%, at least about 70%, at least about 75%, at least about 80%, at least about 85%, at least about 90%, at least about 95%, or at least about 100% more uridine that the candidate nucleic acid sequence.

In other aspects, the uridine content (average global uridine content) (absolute or relative) of the uridine-modified sequence is lower than the uridine content (absolute or relative) of the candidate nucleic acid sequence. Accordingly, in some aspects, the uridine-modified sequence contains at least about 5%, at least about 10%, at least about 15%, at least about 20%, at least about 25%, at least about 30%, at least about 35%, at least about 40%, at least about 45%, at least about 50%, at least about 55%, at least about 60%, at least about 65%, at least about 70%, at least about 75%, at least about 80%, at least about 85%, at least about 90%, at least about 95%, or at least about 100% less uridine that the candidate nucleic acid sequence.

In some aspects, the uridine content (average global uridine content) (absolute or relative) of the uridine-modified sequence is less than 50%, 49%, 48%, 47%, 46%, 45%, 44%, 43%, 42%, 41%, 40%, 39%, 38%, 37%, 36%, 35%, 34%, 33%, 32%, 31%, 30%, 29%, 28%, 27%, 26%, 25%, 24%, 23%, 22%, 21%, 20%, 19%, 18%, 17%, 16%, 15%, 14%, 13%, 12%, 11%, 10%, 9%, 8%, 7%, 6%, 5%, 4%, 3%, 2% or 1% of the total nucleobases in the uridine-modified sequence. In some aspects, the uridine content of the uridine-modified sequence is between about 10% and about 20%. In some particular aspects, the uridine content of the uridine-modified sequence is between about 12% and about 16%.

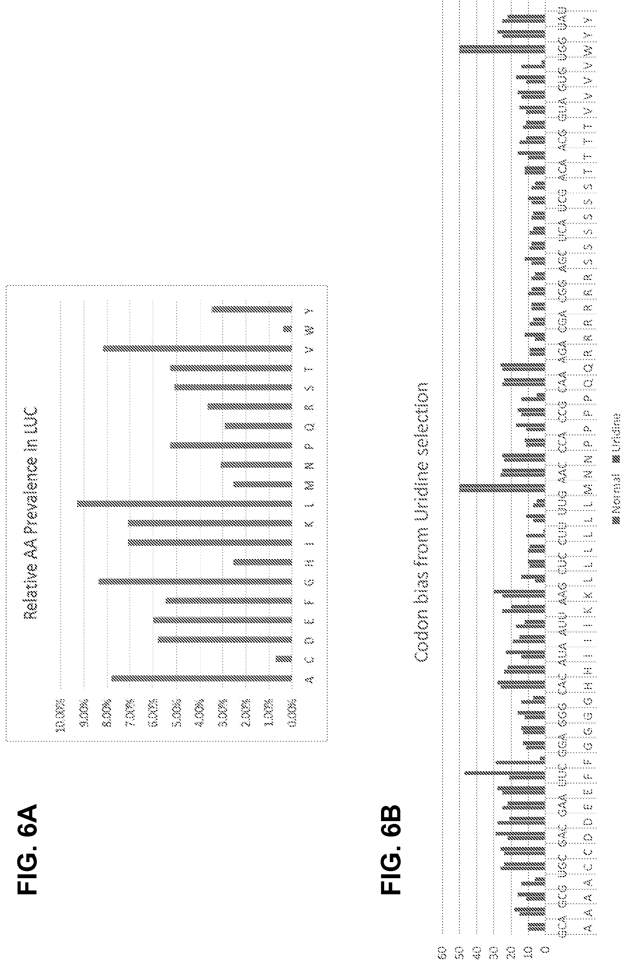

In some aspects, the uridine content of the candidate nucleic acid sequence can be measured using a sliding window. In some aspects, the length of the sliding window is 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, or 40 nucleobases. In some aspects, the sliding window is over 40 nucleobases in length. In some aspects, the sliding window is 20 nucleobases in length. Based on the uridine content measured with a sliding window, it is possible to generate a histogram representing the uridine content throughout the length of the candidate nucleic acid sequence and optimized nucleic acid sequences (example of such representations are show in FIGS. 6A and 6B). In some aspects, the candidate nucleic acid sequence can be modified to reduce or eliminate peaks in the representation that are above or below a certain percentage value. In some aspects, the candidate nucleic acid sequence can be modified to eliminate peaks in the sliding-window representation which are above 65%, 60%, 55%, 50%, 45%, 40%, 35%, or 30% uridine. In another aspect, the candidate nucleic acid sequence can be modified so no peaks are over 30% uridine in the optimized nucleic acid sequence, as measured using a 20 nucleobase sliding window. In some aspects, the candidate nucleic acid sequence can be modified so no more or no less than a predetermined number of peaks in the optimized nucleic sequence, as measured using a 20 nucleobase sliding window, are above or below a certain threshold value. For example, in some aspects, the candidate nucleic acid sequence can be modified so no peaks or no more than 1, 2, 3, 4, 5, 6, 7, 8, 9 or 10 peaks in the optimized nucleic acid sequence are above 10%, 15%, 20%, 25% or 30% uridine. In another aspect, the optimized nucleic acid sequence contains between 0 peaks and 2 peaks with uridine contents 30% of higher.

In some aspects, the candidate nucleic acid sequence can be optimized to reduce the incidence of consecutive uridines. For example, two consecutive leucines could be encoded by the sequence CUUUUG, which would include a four uridine cluster. Such subsequence could be substituted with CUGCUC, which would effectively remove the uridine cluster. Accordingly, a candidate nucleic sequence can be optimized by reducing or eliminating uridine pairs (UU), uridine triplets (UUU) or uridine quadruplets (UUUU). In some aspects, all uridine pairs (UU) and/or uridine triplets (UUU) and/or uridine quadruplets (UUUU) can be removed from the candidate nucleic acid sequence. In other aspects, uridine pairs (UU) and/or uridine triplets (UUU) and/or uridine quadruplets (UUUU) can be reduced below a certain threshold, e.g., no more than 1, 2, 3, 4, 5, 6, 7, 8, 9, or 10 occurrences in the optimized nucleic acid sequence. In a particular aspect, the optimized nucleic acid sequence contains less than 5, 4, 3, 2, or 1 uridine pairs. In another particular aspect, the optimized nucleic acid sequence contains no uridine pairs.

In some aspects, the candidate nucleic acid sequence can comprise uridine clusters which due to their number, size, location, distribution or combinations thereof have negative effects on translation. As used herein, the term "uridine cluster" refers to a subsequence in a candidate nucleic acid sequence or optimized nucleic sequence with contains a uridine content (usually described as a percentage) which is above a certain threshold. Thus, in certain aspects, if a subsequence comprises more than about 10%, 15%, 20%, 25%, 30%, 35%, 40%, 45%, 50%, 55%, 60% or 65% uridine content, such subsequence would be considered a uridine cluster.

The negative effects of uridine clusters can be, for example, eliciting a TLR7 response. Thus, in some implementations of the multiparametric nucleic acid optimization methods disclosed herein it is desirable to reduce the number of clusters, size of clusters, location of clusters (e.g., close to the 5' and/or 3' end of a nucleic acid sequence), distance between clusters, or distribution of uridine clusters (e.g., a certain pattern of cluster along a nucleic acid sequence, distribution of clusters with respect to secondary structure elements in the expressed product, or distribution of clusters with respect to the secondary structure of an mRNA).

In some aspects, the candidate nucleic acid sequence comprises at least one uridine cluster, wherein said uridine cluster is a subsequence of the candidate nucleic acid sequence wherein the percentage of total uridine nucleobases in said subsequence is above a predetermined threshold. In some aspects, the length of the subsequence is at least about 10, at least about 15, at least about 20, at least about 25, at least about 30, at least about 35, at least about 40, at least about 45, at least about 50, at least about 55, at least about 60, at least about 65, at least about 70, at least about 75, at least about 80, at least about 85, at least about 90, at least about 95, or at least about 100 nucleobases. In some aspects, the subsequence is longer than 100 nucleobases. In some aspects, the threshold is 1%, 2%, 3%, 4%, 5%, 6%, 7%, 8%, 9%, 10%, 11%, 12%, 13%, 14%, 15%, 16%, 17%, 18%, 19%, 20%, 21%, 22%, 23%, 24% or 25% uridine content. In some aspects, the threshold is above 25%.

For example, an amino acid sequence such as ADGSR could be encoded by the nucleic acid sequence GCU GAU GGU AGU CGU. Although such sequence does not contain any uridine pairs, triplets, or quadruplets, one third of the nucleobases would be uridines. Such a uridine cluster could be removed by using alternative codons, for example, by using the coding sequence GCC GAC GGC AGC CGC, which would contain no uridines.

In other aspects, the candidate nucleic acid sequence comprises at least one uridine cluster, wherein said uridine cluster is a subsequence of the candidate nucleic acid sequence wherein the percentage of uridine nucleobases of said subsequence as measured using a sliding window that is above a predetermined threshold. In some aspects, the length of the sliding window is 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, or 40 nucleobases. In some aspects, the sliding window is over 40 nucleobases in length. In some aspects, the threshold is 1%, 2%, 3%, 4%, 5%, 6%, 7%, 8%, 9%, 10%, 11%, 12%, 13%, 14%, 15%, 16%, 17%, 18%, 19%, 20%, 21%, 22%, 23%, 24% or 25% uridine content. In some aspects, the threshold is above 25%.

In some aspects, the candidate nucleic acid sequence comprises at least two uridine clusters. In some aspects, the uridine-modified sequence contains fewer uridine-rich clusters than the candidate nucleic acid sequence. In some aspects, the uridine-modified sequence contains more uridine-rich clusters than the candidate nucleic acid sequence. In some aspects, the uridine-modified sequence contains uridine-rich clusters with are shorter in length than corresponding uridine-rich clusters in the candidate nucleic acid sequence. In other aspects, the uridine-modified sequence contains uridine-rich clusters which are longer in length than the corresponding uridine-rich cluster in the candidate nucleic acid sequence.

See also, Kariko et al. (2005) Immunity 23:165-175; Kormann et al. (2010) Nature Biotechnology 29:154-157; or Sahin et al. (2014) Nature Reviews Drug Discovery | AOP, published online 19 September 2014m doi:10.1038/nrd4278; all of which are herein incorporated by reference their entireties.

### d. Guanine/Cytosine (G/C) Content

The present disclosure provides multiparametric nucleic acid optimization methods comprising altering the Guanine/Cytosine (G/C) content (absolute or relative) of a candidate nucleic acid sequence. Such optimization can comprise altering (e.g., increasing or decreasing) the global G/C content (absolute or relative) of the candidate nucleic acid sequence; introducing local changes in G/C content in the candidate nucleic acid sequence (e.g., increase or decrease G/C in selected regions or subsequences in the candidate nucleic acid sequence); altering the frequency, size, and distribution of G/C clusters in the candidate nucleic acid sequence, or combinations thereof.

In some aspects, the optimized nucleic acid sequence comprises an overall increase in G/C content (absolute or relative) relative to the G/C content (absolute or relative) of the candidate nucleic acid sequence. In some aspects, the overall increase in G/C content (absolute or relative) is at least about 5%, at least about 10%, at least about 15%, at least about 20%, at least about 25%, at least about 30%, at least about 35%, at least about 40%, at least about 45%, at least about 50%, at least about 55%, at least about 60%, at least about 65%, at least about 70%, at least about 75%, at least about 80%, at least about 85%, at least about 90%, at least about 95%, or at least about 100% relative to the G/C content (absolute or relative) of the candidate nucleic acid sequence.

In some aspects, the optimized nucleic acid sequence comprises an overall decrease in G/C content (absolute or relative) relative to the G/C content of the candidate nucleic acid sequence. In some aspects, the overall decrease in G/C content (absolute or relative) is at least about 5%, at least about 10%, at least about 15%, at least about 20%, at least about 25%, at least about 30%, at least about 35%, at least about 40%, at least about 45%, at least about 50%, at least about 55%, at least about 60%, at least about 65%, at least about 70%, at least about 75%, at least about 80%, at least about 85%, at least about 90%, at least about 95%, or at least about 100% relative to the G/C content (absolute or relative) of the candidate nucleic acid sequence.

In some aspects, the optimized nucleic acid sequence comprises a local increase in Guanine/Cytosine (G/C) content (absolute or relative) in a subsequence (i.e., a G/C modified subsequence) relative to the G/C content (absolute or relative) of the corresponding subsequence in the candidate nucleic acid sequence. In some aspects, the local increase in G/C content (absolute or relative) is by at least about 5%, at least about 10%, at least about 15%, at least about 20%, at least about 25%, at least about 30%, at least about 35%, at least about 40%, at least about 45%, at least about 50%, at least about 55%, at least about 60%, at least about 65%, at least about 70%, at least about 75%, at least about 80%, at least about 85%, at least about 90%, at least about 95%, or at least about 100% relative to the G/C content (absolute or relative) of the corresponding subsequence in the candidate nucleic acid sequence.

In some aspects, the optimized nucleic acid sequence comprises a local decrease in Guanine/Cytosine (G/C) content (absolute or relative) in a subsequence (i.e., a G/C modified subsequence) relative to the G/C content (absolute or relative) of the corresponding subsequence in the candidate nucleic acid sequence. In some aspects, the local decrease in G/C content (absolute or relative) is by at least about 5%, at least about 10%, at least about 15%, at least about 20%, at least about 25%, at least about 30%, at least about 35%, at least about 40%, at least about 45%, at least about 50%, at least about 55%, at least about 60%, at least about 65%, at least about 70%, at least about 75%, at least about 80%, at least about 85%, at least about 90%, at least about 95%, or at least about 100% relative to the G/C content (absolute or relative) of the corresponding subsequence in the candidate nucleic acid sequence.

In some aspects, the G/C content (absolute or relative) is increased or decreased in a subsequence which is at least about 5, 10, 15, 20, 25, 30, 35, 40, 45, 50, 55, 60, 65, 70, 75, 80, 85, 90, 95, or 100 nucleobases in length.

In some aspects, the G/C content (absolute or relative) is increased or decreased in a subsequence which is at least about 100, 110, 120, 130, 140, 150, 160, 170, 180, 190, 200, 210, 220, 230, 240, 250, 260, 270, 280, 290, 300, 310, 320, 330, 340, 350, 360, 370, 380, 390, 400, 410, 420, 430, 440, 450, 460, 470, 480, 490, 500, 510, 520, 530, 540, 550, 560, 570, 580, 590, 600, 610, 620, 630, 640, 650, 660, 670, 680, 690, 700, 710, 720, 730, 740, 750, 760, 770, 780, 790, 800, 810, 820, 830, 840, 850, 860, 870, 880, 890, 900, 910, 920, 930, 940, 950, 960, 970, 980, 990, or 1000 nucleobases in length.

In some aspects, the G/C content (absolute or relative) is increased or decreased in a subsequence which is at least about 1100, 1200, 1300, 1400, 1500, 1600, 1700, 1800, 1900, 2000, 2100, 2200, 2300, 2400, 2500, 2600, 2700, 2800, 2900, 3000, 3100, 3200, 3300, 3400, 3500, 3600, 3700, 3800, 3900, 4000, 4100, 4200, 4300, 4400, 4500, 4600, 4700, 4800, 4900, 5000, 5100, 5200, 5300, 5400, 5500, 5600, 5700, 5800, 5900, 6000, 6100, 6200, 6300, 6400, 6500, 6600, 6700, 6800, 6900, 7000, 7100, 7200, 7300, 7400, 7500, 7600, 7700, 7800, 7900, 8000, 8100, 8200, 8300, 8400, 8500, 8600, 8700, 8800, 8900, 9000, 9100, 9200, 9300, 9400, 9500, 9600, 9700, 9800, 9900, or 10000 nucleobases in length.

The increases or decreases in G and C content (absolute or relative) described herein can be conducted by replacing synonymous codons with low G/C content with synonymous codons having higher G/C content, or vice versa. For example, L has 6 synonymous codons: two of them have 2 G/C (CUC, CUG), 3 have a single G/C (UUG, CUU, CUA), and one has no G/C (UUA). So if the candidate nucleic acid had a CUC codon in a certain position, G/C content at that position could be reduced by replacing CUC with any of the codons having a single G/C or the codon with no G/C.

See also, U.S. Publ. Nos. US20140228558, US20050032730 A1; Gustafsson et al. (2012) Protein Expression and Purification 83: 37-46; all of which are incorporated herein by reference in their entireties.

### e. Codon Frequency - Codon Usage Bias

Numerous codon optimization methods known in the art are based on the substitution of codons in a candidate nucleic acid sequence with codons having higher frequencies. Thus, in some aspects, the present disclosure provides multiparametric nucleic acid optimization methods comprising the use of modifications in the frequency of use of one or more codons relative to other synonymous codons in the optimized nucleic acid sequence with respect to the frequency of use in the non-optimized sequence.

As used herein, the term "codon frequency" refers to codon usage bias, i.e., the differences in the frequency of occurrence of synonymous codons in coding DNA/RNA. It is generally acknowledged that codon preferences reflect a balance between mutational biases and natural selection for translational optimization. Optimal codons in fast-growing microorganisms, like *Escherichia coli* or *Saccharomyces cerevisiae* (baker's yeast), reflect the composition of their respective genomic tRNA pool. Optimal codons help to achieve faster translation rates and high accuracy. As a result of these factors, translational selection is expected to be stronger in highly expressed genes, as is indeed the case for the above-mentioned organisms.

In the field of bioinformatics and computational biology, many statistical methods have been proposed and used to analyze codon usage bias. See, e.g., Comeron & Aguadé (1998) J. Mol. Evol. 47: 268-74. Methods such as the 'frequency of optimal codons' (Fop) (Ikemura (1981) J. Mol. Biol. 151 (3): 389-409), the Relative Codon Adaptation (RCA) (Fox & Eril (2010) DNA Res. 17 (3): 185-96) or the 'Codon Adaptation Index' (CAI) (Sharp & Li (1987) Nucleic Acids Res. 15 (3): 1281-95) are used to predict gene expression levels, while methods such as the 'effective number of codons' (Nc) and Shannon entropy from information theory are used to measure codon usage evenness. Multivariate statistical methods, such as correspondence analysis and principal component analysis, are widely used to analyze variations in codon usage among genes (Suzuki et al. (2008) DNA Res. 15 (6): 357-65; Sandhu et al., In Silico Biol. 2008;8(2):187-92).

The present disclosure provides multiparametric methods for optimizing a candidate nucleic acid sequence (e.g., a wild type nucleic acid sequence, a mutant nucleic acid sequence, a chimeric nucleic sequence, etc. which can be, for example, an mRNA), the method comprising substituting at least one codon in the candidate nucleic acid sequence with an alternative codon having a higher or lower codon frequency in the synonymous codon set; wherein the resulting optimized nucleic acid sequence has at least one optimized property with respect to the candidate nucleic acid sequence.

In some aspects, at least about 5%, at least about 10%, at least about 15%, at least about 20%, at least about 25%, at least about 30%, at least about 35%, at least about 40%, at least about 45%, at least about 50%, at least about 55%, at least about 60%, at least about 65%, at least about 70%, at least about 75%, at least about 80%, at least about 85%, at least about 90%, at least about 95%, at least about 99%, or 100% of the codons in the candidate nucleic acid sequence are substituted with alternative codons, each alternative codon having a codon frequency higher than the codon frequency of the substituted codon in the synonymous codon set.

In some aspects, at least one codon in the candidate nucleic acid sequence is substituted with an alternative codon having a codon frequency higher than the codon frequency of the substituted codon in the synonymous codon set, and at least one codon in the candidate nucleic acid sequence is substituted with an alternative codon having a codon frequency lower than the codon frequency of the substituted codon in the synonymous codon set.

In some aspects, at least about 5%, at least about 10%, at least about 15%, at least about 20%, at least about 25%, at least about 30%, at least about 35%, at least about 40%, at least about 45%, at least about 50%, at least about 55%, at least about 60%, at least about 65%, at least about 70%, or at least about 75% of the codons in the candidate nucleic acid sequence are substituted with alternative codons, each alternative codon having a codon frequency higher than the codon frequency of the substituted codon in the synonymous codon set.

In some aspects, at least one alternative codon having a higher codon frequency has the highest codon frequency in the synonymous codon set. In other aspects, all alternative codons having a higher codon frequency have the highest codon frequency in the synonymous codon set.

In some aspects, at least one alternative codon having a lower codon frequency has the lowest codon frequency in the synonymous codon set. In some aspects, all alternative codons having a higher codon frequency have the highest codon frequency in the synonymous codon set.

In some specific aspects, at least one alternative codon has the second highest, the third highest, the fourth highest, the fifth highest or the sixth highest frequency in the synonymous codon set. In some specific aspects, at least one alternative codon has the second lowest, the third lowest, the fourth lowest, the fifth lowest, or the sixth lowest frequency in the synonymous codon set.

Optimization based on codon frequency can be applied globally, as described above, or locally to the candidate nucleic acid sequence. In some aspects, when applied locally, regions of the candidate nucleic acid sequence can modified based on codon frequency, substituting all or a certain percentage of codons in a certain subsequence with codons that have higher or lower frequencies in their respective synonymous codon sets. Thus, in some aspects, at least about 5%, at least about 10%, at least about 15%, at least about 20%, at least about 25%, at least about 30%, at least about 35%, at least about 40%, at least about 45%, at least about 50%, at least about 55%, at least about 60%, at least about 65%, at least about 70%, at least about 75%, at least about 80%, at least about 85%, at least about 90%, at least about 95%, at least about 99%, or 100% of the codons in a subsequence of the candidate nucleic acid sequence are substituted with alternative codons, each alternative codon having a codon frequency higher than the codon frequency of the substituted codon in the synonymous codon set.

In some aspects, at least one codon in a subsequence of the candidate nucleic acid sequence is substituted with an alternative codon having a codon frequency higher than the codon frequency of the substituted codon in the synonymous codon set, and at least one codon in a subsequence of the candidate nucleic acid sequence is substituted with an alternative codon having a codon frequency lower than the codon frequency of the substituted codon in the synonymous codon set.

In some aspects, at least about 5%, at least about 10%, at least about 15%, at least about 20%, at least about 25%, at least about 30%, at least about 35%, at least about 40%, at least about 45%, at least about 50%, at least about 55%, at least about 60%, at least about 65%, at least about 70%, or at least about 75% of the codons in a subsequence of the candidate nucleic acid sequence are substituted with alternative codons, each alternative codon having a codon frequency higher than the codon frequency of the substituted codon in the synonymous codon set. In some aspects, at least one alternative codon substituted in a subsequence of the candidate nucleic acid sequence and having a higher codon frequency has the highest codon frequency in the synonymous codon set. In other aspects, all alternative codons substituted in a subsequence of the candidate nucleic acid sequence and having a lower codon frequency have the lowest codon frequency in the synonymous codon set.

In some aspects, at least one alternative codon substituted in a subsequence of the candidate nucleic acid sequence and having a lower codon frequency has the lowest codon frequency in the synonymous codon set. In some aspects, all alternative codons substituted in a subsequence of the candidate nucleic acid sequence and having a higher codon frequency have the highest codon frequency in the synonymous codon set.

In specific aspects, an optimized nucleic acid sequence can comprise a subsequence having an overall codon frequency higher or lower than the overall codon frequency in the corresponding subsequence of the candidate nucleic acid sequence at a specific location, for example, at the 5' end or 3' end of the optimized nucleic acid sequence, or within a predetermined distance from those region (e.g., at least 1, 2, 3, 4, 5, 6, 7, 8, 9, 10, 15, 20, 25, 30, 35, 40, 45, 50, 55, 60, 65, 70, 75, 80, 85, 90, 95 or 100 codons from the 5' end or 3' end of the optimized nucleic acid sequence).

In some aspects, an optimized nucleic acid sequence can comprise more than one subsequence having an overall codon frequency higher or lower than the overall codon frequency in the corresponding subsequence of the candidate nucleic acid sequence. A skilled artisan would understand that subsequences with overall higher or lower overall codon frequencies can be organized in innumerable patterns, depending on whether the overall codon frequency is higher or lower, the length of the subsequence, the distance between subsequences, the location of the subsequences, etc.

See also, U.S. Pat. Nos. US5082767, US8126653, US7561973, US8401798; U.S. Publ. No. US 20080046192, US 20080076161; Int'l. Publ. No. WO2000018778; Welch et al. (2009) PLoS ONE 4(9): e7002; Gustafsson et al. (2012) Protein Expression and Purification 83: 37-46; Chung et al. (2012) BMC Systems Biology 6:134; all of which are incorporated herein by reference in their entireties.

### f. Codon Recharging - tRNA recharging/recycling

The use of different codons for a certain amino acid, in particular in target protein regions rich in a certain type of amino acid, requires a time "penalty" to find and recruit the appropriate tRNA. This problem can be ameliorated by selecting codons with large tRNAS pools. Conversely, when a single codon is used for a certain amino acid, the population of that particular tRNA in a charged state in the vicinity of the ribosome is depleted. In this scenario, the "penalty" would depend of the rate at which the depleted tRNA can be recharged. Thus, translation could be sped up if depleted tRNAs recharge fast, or could be slowed down if depleted tRNAs recharge slowly. Analyzing the codon preferences of wild type sequences shows a striking re-use of codons in some but not all cases, i.e., when a certain codon is used, it is highly likely that the following codon for the same amino acid will be the same codon (autocorrelation). See, e.g., Caniaroli & Lobardo (2014) Trieste, Phys. Rev. E89; Cannarozzi et al. (2010) Cell 141:355-367, both of which are herein incorporated by reference in their entireties.

Accordingly, the present disclosure provides multiparametric nucleic acid optimization methods comprising substituting at least one codon in a candidate nucleic acid sequence with a codon having a faster or slower codon recharging rate (which can be a species specific, tissue type specific, or cell type specific recharging rate).

As used herein, the term "codon recharge" refers to the enzymatic binding of a specific amino acid to a specific tRNA mediated by its respective aminoacyl-tRNA (aatRNA) synthetase. tRNAs provide the code that associates each sense nucleotide triplet (codon) with a given amino acid. tRNAs ensure that coding sequences are reproducibly translated into the same polypeptides. Thus, each of the 61 sense codons requires that at least one specific tRNA decodes it always into the same amino acid. Because there are more sense codons than amino acids, groups of codons are synonymous, i.e., they code for the same amino acid. Frequent amino acids can be encoded by up to six alternative codons. Ideally, these synonymous codons should be recognized and translated each by their own tRNA, presenting the corresponding anticodon sequence. However, numerous tRNAs compete with each other at the acceptor site of ribosomes, until the correct tRNA is stably selected. Two observations suggest that this competition antagonizes translation efficiency. First, evolution favored the emergence of multivalent tRNAs that can recognize more than one synonymous codon. This allows reducing the number of tRNAs needed, and hence, tRNA complexity. Consequently, most organisms translate the 61 sense codons with less than 61 tRNAs. Second, the different tRNA species are differentially expressed: some tRNAs are more abundant than their synonymous cognates. As a consequence, synonymous codons are not equivalent and are not used, and codons decoded by frequent tRNAs are more frequent in coding sequences than their synonyms. See Ikemura (1985) Mol. Biol. Evol. 2: 13-34; Sharp et al. (1993) Biochem. Soc. Trans. 21: 835-841; Dong et al. (1996) J. Mol. Biol. 260: 649-663; Duret (2000) Trends Genet. 16: 287-289; Cannarozi et al. (2010) Cell 141:355-367, all of which are herein incorporated by reference in their entireties.

The terms "recharging rate" or "tRNA recharging rate" refer to the rate at which a tRNA is recharged by aminoacyl-tRNA (aatRNA) synthetases after being used by the ribosome during protein synthesis. tRNA recharging rates can be experimentally measured, or calculated using other parameters that correlate or partially correlate with tRNA recharging rates, for example, codon frequency.

Recharging rates can vary, for example, according to species, tissue type, or cell type. Accordingly, there are "species specific recharging rates," "tissue specific recharging rates," and "cell type specific recharging rates." Therefore, the choice of a certain optimization strategy based on codon recharging depends, for example, on the specific organism to which the optimized nucleic acid will be administered (e.g., a non-human cell line for *in vitro* testing, or a non-human animal for *in vivo* testing), or to the tissue type in a certain organism (which is a critical factor to consider depending on which tissue or organ will be targeted by an optimized nucleic acid sequence produced according to the multiparametric nucleic acid optimization methods disclosed herein, e.g., an mRNA, and more in particular a synthetic mRNA), or a particular cell type.

As discussed above, a single amino acid can be encoded by more than one synonymous codon, which generally will differ in their recharging rate (which can be a species specific, tissue type specific, or cell type specific recharging rate). For amino acids encoded by two synonymous codons, the term "fast-recharging codon" refers to the codon with the fastest recharging rate (which can be a species specific, tissue type specific, or cell type specific recharging rate), and the term "slow-recharging codon" refers to the codon with the slowest recharging rate (which can be a species specific, tissue type specific, or cell type specific recharging rate). For amino acids encoded by more than two synonymous codons (e.g., arginine or leucine are each encoded by a codon set comprising 6 different codons): (i) the term "fast-recharging codon" refers to a codon with a recharging rate above the average recharging rate in the synonymous codon set;
(ii) the term "slow-recharging codon" refers to a codon with a recharging rate below the average recharging rate in the synonymous codon set;
(iii) the term "fastest-recharging codon" refers to the codon with fastest recharging rate in the synonymous codon set; and,
(iv) the term "slowest-recharging codon" refers to the codon with a slowest recharging rate in the synonymous codon set.

In some aspects, at least about 5%, at least about 10%, at least about 15%, at least about 20%, at least about 25%, at least about 30%, at least about 35%, at least about 40%, at least about 45%, at least about 50%, at least about 55%, at least about 60%, at least about 65%, at least about 70%, at least about 75%, at least about 80%, at least about 85%, at least about 90%, at least about 95%, at least about 99%, or 100% of the codons in the candidate nucleic acid sequence are substituted with alternative codons having faster recharging rates (which can be a species specific, tissue type specific, or cell type specific recharging rate).

In some aspects, at least one codon in the candidate nucleic acid sequence is substituted with an alternative codon having a faster recharging rate, and at least one codon in the candidate nucleic acid sequence is substituted with an alternative codon having a slower recharging rate (which can be a species specific, tissue type specific, or cell type specific recharging rate).

In other aspects, at least about 5%, at least about 10%, at least about 15%, at least about 20%, at least about 25%, at least about 30%, at least about 35%, at least about 40%, at least about 45%, at least about 50%, at least about 55%, at least about 60%, at least about 65%, at least about 70%, or at least about 75% of the codons in the candidate nucleic acid sequence are substituted with alternative codons, each codon having a having a slower recharging rate (which can be a species specific, tissue type specific, or cell type specific recharging rate).

In some aspects, at least one alternative codon having a faster recharging rate has the fastest recharging rate (which can be a species specific, tissue type specific, or cell type specific recharging rate). In other aspects, all alternative codons having a faster recharging rate have the fastest recharging rate (which can be a species specific, tissue type specific, or cell type specific recharging rate). In other aspects, at least one alternative codon having a slower recharging rate has the slowest recharging rate (which can be a species specific, tissue type specific, or cell type specific recharging rate). In some aspects, all alternative codons having a slower recharging rate have the slowest recharging rate (which can be a species specific, tissue type specific, or cell type specific recharging rate).

In some aspects, the replacement of codons with codons having faster or slower recharging rates is conducted according to patterns, for example, block patterns where all the codons in a certain region or subsequence in the candidate nucleic acid sequence are replaced with faster recharging codons, and all the codons in an adjacent or non-adjacent region or subsequence in the candidate nucleic acid sequence are replaced with slower recharging codons. In certain aspects, only a certain number of codons are replaced in each region or subsequence in a block pattern substitution strategy. The substitution pattern for a block strategy could be summarized according to the formula Aₓ[F/S]-a-B_{y}[F/S], wherein 'A' and 'B' represent a subsequence length, which can be between 1 codon and 100 codons; 'x' and 'y' represents the number of codons replaced in the block (e.g., from 1% to 100%); '[F/S]' indicates whether the recharging rate of each codon is higher or lower than the rate of corresponding codons in the corresponding block in the candidate nucleic acid sequence; and 'a' refers to the distance between codon blocks, in codons. Such pattern could be repeated a number of times throughout an optimized nucleic acid sequence, with blocs arranged consecutively, at variable distances between blocks, or at regular distances between blocks.

In other aspects, codons can be replaced in the candidate nucleic acid sequence according to alternating patterns, e.g., F-S-F-S-F-S, or F-F-S-S-F-F-S-S, etc. In other aspects, codons can be replaced in the candidate nucleic acid sequence according to rotating patterns, e.g. 1-2-3-4-1-2-3-4-1-2-3-4, wherein 1, 2, 3, and 4 represent different recharge rates. According to this type of strategy, it would be possible to rotate among different pools of tRNA to access pre-charged tRNA rather than continuing to use the same pool until it becomes depleted and translation slows down or stalls. For example, the translation of a sequence with a recharging rate distribution 111111111111111111111111111111, wherein the numeral refers to the recharging rate within a synonymous codon group, may stall due to repeated use of "1" codons, but it may continue without interruption if codons were rotated, e.g., according to a pattern 111112222233333111112222233333. Codon type "1" could be used several times and before the type "1" tRNA pool was fully depleted the codon would change to "2", and then to "3". At that point, the codon choice could cycle back to "1", with the tRNA population of type "1" codons being replenished.

In some aspects, recharging rate data can be used to optimize a codon set, for example, by generating a limited codon set with 20 codons, one codon per amino acid, wherein the representative codon selected for each amino acid has the fastest or lowest recharging rate in each synonymous codon group. A protein target-specific codon set can also be created based on recharging rate data, for example, selecting a representative codon with a recharging rate which is optimal for the amino acid distribution along the protein, which may be neither the codon with the faster rate nor the codon with the slower recharging rate.

In some aspects, codons encoding a certain amino acid are replaced by codons with faster or slower codon recharging rates, for example, only codons encoding alanines, or codons encoding glycines, etc. In other aspects, codons encoding a certain amino acid group are replaced by codons with faster or slower codon recharging rates, for example, only codons encoding acid amino acids, prolines, aromatic amino acids, etc. In certain aspects, codons are replaced by codons with faster or slower codon recharging rates according to:
(i) size of the amino acid encoded by the codon;
(ii) hydrophobicity of the amino acid encoded by the codon;
(iii) charge of the amino acid encoded by the codon;
(iv) location of the codon in a certain mRNA secondary structure region;
(v) location of the amino acid encoded by the codon in a certain protein secondary structure region (e.g., an alpha helix of beta strand);
(vi) location of the codon in a low flexibility or high flexibility region of the mRNA; or,
(vii) any combination thereof.

See also, Frederick et al. (2010) Cell 141(2):227-229; Elf et al. (2003) Science 1718-1722; Zhou et al. (2014) Nature 498:7439; Brackley et al. (2011) PLoS Comput Biol 7(10): e1002203; Plotkin et al. (2004) Proc. Natl. Acad. Sci. USA 101:12588-12591; Dittmar et al. (2006) PLoS Genet. 2(12): e221; Gingold et al. (2012) Nucl. Acids Res. 40 (20):10053-10063; Waldman et al. (2010) Nucl. Acids Res. 38(9): 2964-2974; Qian et al. (2012) PLoS Genet 8(3):e1002603, all of which are incorporated herein by reference in their entireties.

### g. Secondary Structure Optimization

In some aspects, the present disclosure provides a multiparametric method for optimizing a candidate nucleic acid sequence (e.g., a wild type nucleic acid sequence, a mutant nucleic acid sequence, a chimeric nucleic sequence, etc. which can be, for example, an mRNA), the method comprising substituting at least one codon in the candidate nucleic acid sequence wherein such substitution modifies the secondary structure of the candidate nucleic acid sequence (e.g., mRNA secondary structure), prevents the adoption of a certain secondary structure, disrupts a certain secondary structure, or hinders the adoption of a certain secondary structure that otherwise would have a negative effect on a certain property, for example, translational efficacy.

Changing the structure of a candidate nucleic acid sequence during the application of the multiparametric nucleic acid optimization methods disclosed herein can change the secondary structure of the nucleic acid sequence. In turn, changing the secondary structure can change (increase or decrease) expression levels for a variety of reasons, primarily due to the ability of the ribosome to "unwind" mRNA secondary structure in order to read and translate the mRNA. Accordingly, in some aspects, the multiparametric nucleic acid optimization methods disclosed herein comprise monitoring the secondary structure of the nucleic acid during optimization, using protein secondary structure as a post-hoc filtering stage to determine whether a certain modification which potentially could be introduced in the candidate nucleic acid sequence should be actually implemented or not. The secondary structure of an mRNA can be measured by SHAPE or similar biochemical techniques, and/or predicted using RNA structure or similar theoretical techniques.

See, e.g., U.S. Publ. No. 2014/0228558, which is herein incorporated by reference in its entirety.

### h. Destabilizing Motif Substitution

There is a variety of motifs that can affect codon optimization, which fall into various non-exclusive categories, for example:
(i) Primary sequence based motifs: Motifs defined by a simple arrangement of nucleotides.
(ii) Structural motifs: Motifs encoded by an arrangement of nucleotides that tends to form a certain secondary structure.
(iii) Local motifs: Motifs encoded in one contiguous subsequence.
(iv) Distributed motifs: Motifs encoded in two or more disjoint subsequences.
(v) Advantageous motifs: Motifs which improve nucleotide structure or function.
(vi) Disadvantageous motifs: Motifs with detrimental effects on nucleotide structure or function.

There are many motifs that fit into the category of disadvantageous motifs. Some examples include, for example, restriction enzyme motifs, which tend to be relatively short, exact sequences such as the restriction site motifs for *Xba1* (TCTAGA), *EcoRI* (GAATTC), *EcoRII* (CCWGG, wherein W means A or T, per the IUPAC ambiguity codes), or *HindIII* (AAGCTT); enzyme sites, which tend to be longer and based on consensus not exact sequence, such in the T7 RNA polymerase (GnnnnWnCRnCTCnCnnWnD, wherein n means any nucleotide, R means A or G, W means A or T, D means A or G or T but not C); structural motifs, such as GGGG repeats (Kim et al. (1991) Nature 351(6324):331-2); or other motifs such as CUG-triplet repeats (Querido et al. (2014) J. Cell Sci. 124:1703-1714).

Accordingly, the present disclosure provides multiparametric nucleic acid optimization methods comprising substituting at least one destabilizing motif in a candidate nucleic acid sequence, and removing such disadvantageous motif or replacing it with an advantageous motif. In some aspects, the optimization process comprises identifying advantageous and/or disadvantageous motifs in the candidate nucleic sequence, wherein such motifs are, e.g., specific subsequences that can cause a loss of stability in the candidate nucleic acid sequence prior or during the optimization process. For example, substitution of specific bases during optimization may generate a subsequence (motif) recognized by a restriction enzyme. Accordingly, during the optimization process the appearance of disadvantageous motifs can be monitored by comparing the optimized sequence with a library of motifs known to be disadvantageous. Then, the identification of disadvantageous motifs could be used as a post-hoc filter, i.e., to determine whether a certain modification which potentially could be introduced in the candidate nucleic acid sequence should be actually implemented or not.

In some aspects, the identification of disadvantageous motifs can be used prior to the application of the multiparametric optimization methods disclosed herein, i.e., the identification of motifs in the candidate nucleic acid sequence and their replacement with alternative nucleic acid sequences can be used as a preprocessing step.

In other aspects, the identification of disadvantageous motifs and their removal is used as an additional codon optimization technique integrated in the multiparametric nucleic acid optimization methods disclosed herein. When used in this fashion, a disadvantageous motif identified during the optimization process would be removed, for example, by substituting the lowest possible number of nucleobases in order to preserve as closely as possible the original design principle(s) (e.g., low U, high frequency, etc.).

See, e.g., U.S. Publ. Nos. US20140228558, US20050032730, or US20140228558, which are herein incorporated by reference in their entireties.

### IV. Nucleic Acid Chemical Synthesis

The multiparametric nucleic acid optimization methods disclosed herein can be used to design an optimized nucleic acid sequence (e.g., an mRNA), which in turn would be chemically synthesized.

Numerous chemistry synthesis methods and potential nucleobase substitutions are known in the art. See, for example, International Publication Nos. WO2014093924, WO2013052523, WO2013039857, WO2012135805, and WO2013151671, all of which are herein incorporated by reference in their entireties.

Naturally occurring nucleosides, non-naturally occurring nucleosides, or combinations thereof, replacing totally or partially naturally occurring nucleosides present in the candidate nucleic acid sequence can be incorporated into an optimized mRNA encoding a polypeptide of interest. The resultant mRNAs can then be examined for their ability to produce protein, induce cytokines, and/or produce a therapeutic outcome.

Examples of naturally occurring nucleosides that can be incorporated into the optimized nucleic acids (e.g., mRNAs) disclosed herein include 2'-O-methylcytidine, 4-thiouridine, 2'-O-methyluridine, 5-methyl-2-thiouridine, 5,2'-O-dimethyluridine, 5-aminomethyl-2-thiouridine, 5,2'-O-dimethylcytidine, 2-methylthio-N6-isopentenyladenosine, 2'-O-methyladenosine, 2'-O-methylguanosine, N6-methyl-N6-threonylcarbamoyladenosine, N6-hydroxynorvalylcarbamoyladenosine, 2-methylthio-N6-hydroxynorvalyl carbamoyl adenosine, 2'-O-ribosyladenosine (phosphate), N6,2'-O-dimethyladenosine, N6,N6,2'-O-trimethyladenosine, 1,2'-O-dimethyladenosine, N6-acetyladenosine, 2-methyladenosine, 2-methylthio-N6-methyladenosine, N2,2'-O-dimethylguanosine, N2,N2,2'-O-trimethylguanosine, 7-cyano-7-deazaguanosine, 7-aminomethyl-7-deazaguanosine, 2'-O-ribosylguanosine (phosphate), N2,7-dimethylguanosine, N2,N2,7-trimethylguanosine, 1,2'-O-dimethylguanosine, peroxywybutosine, hydroxywybutosine, undermodified hydroxywybutosine, methylwyosine, N2,7,2'-O-trimethylguanosine, 1,2'-O-dimethylinosine, 2'-O-methylinosine, 4-demethylwyosine, isowyosine, queuosine, epoxyqueuosine, galactosyl-queuosine, mannosyl-queuosine, archaeosine, and combinations thereof.

Examples of non-naturally occurring nucleosides that can be incorporated into the optimized nucleic acids (e.g., mRNAs) disclosed herein include 5-(1-propynyl)ara-uridine, 2'-O-methyl-5-(1-propynyl)uridine, 2'-O-methyl-5-(1-propynyl)cytidine, 5-(1-propynyl)aracytidine, 5-ethynylara-cytidine, 5-ethynylcytidine, 5-vinylarauridine, (Z)-5-(2-bromovinyl)ara-uridine, (E)-5-(2-bromo-vinyl)ara-uridine, (Z)-5-(2-bromo-vinyl)uridine, (E)-5-(2-bromo-vinyl)uridine, 5-methoxyuridine, 5-methoxycytidine, 5-formyluridine, 5-cyanouridine, 5-dimethylaminouridine, 5-trideuteromethyl-6-deuterouridine, 5-cyanocytidine, 5-(2-chloro-phenyl)-2-thiocytidine, 5-(4-amino-phenyl)-2-thiocytidine, 5-(2-furanyl)uridine, 5-phenylethynyluridine, N4,2'-O-dimethylcytidine, 3'-ethynylcytidine, 4'-carbocyclic adenosine, 4'-carbocyclic cytidine, 4'-carbocyclic guanosine, 4'-carbocyclic uridine, 4'-ethynyladenosine, 4'-ethynyluridine, 4'-ethynylcytidine, 4'-ethynylguanosine, 4'-azidouridine, 4'-azidocytidine, 4'-azidoadenosine, 4'-azidoguanosine, 2'-deoxy-2',2'-difluorocytidine, 2'-deoxy-2',2'-difluorouridine, 2'-deoxy-2',2'-difluoroadenosine, 2'-deoxy-2',2'-difluoroguanosine, 2'-deoxy-2'-b-fluorocytidine, 2'-deoxy-2'-b-fluorouridine, 2'-deoxy-2'-b-fluoroadenosine, 2'-deoxy-2'-b-fluoroguanosine, 8-trifluoromethyladenosine, 2'-deoxy-2'-b-chlorouridine, 2'-deoxy-2'-b-bromouridine, 2'-deoxy-2'-b-iodouridine, 2'-deoxy-2'-b-chlorocytidine, 2'-deoxy-2'-b-bromocytidine, 2'-deoxy-2'-b-iodocytidine, 2'-deoxy-2'-b-chloroadenosine, 2'-deoxy-2'-b-bromoadenosine, 2'-deoxy-2'-b-iodoadenosine, 2'-deoxy-2'-b-chloroguanosine, 2'-deoxy-2'-b-bromoguanosine, 2'-deoxy-2'-b-iodoguanosine, 5'-homo-cytidine, 5'-homo-adenosine, 5'-homo-uridine, 5'-homo-guanosine, 2'-deoxy-2'-a-mercaptouridine, 2'-deoxy-2'-a-thiomethoxyuridine, 2'-deoxy-2'-a-azidouridine, 2'-deoxy-2'-a-aminouridine, 2'-deoxy-2'-a-mercaptocytidine, 2'-deoxy-2'-a-thiomethoxycytidine, 2'-deoxy-2'-a-azidocytidine, 2'-deoxy-2'-a-aminocytidine, 2'-deoxy-2'-a-mercaptoadenosine, 2'-deoxy-2'-a-thiomethoxyadenosine, 2'-deoxy-2'-a-azidoadenosine, 2'-deoxy-2'-a-aminoadenosine, 2'-deoxy-2'-a-mercaptoguanosine, 2'-deoxy-2'-a-thiomethoxyguanosine, 2'-deoxy-2'-a-azidoguanosine, 2'-deoxy-2'-a-aminoguanosine, 2'-deoxy-2'-b-mercaptouridine, 2'-deoxy-2'-b-thiomethoxyuridine, 2'-deoxy-2'-b-azidouridine, 2'-deoxy-2'-b-aminouridine, 2'-deoxy-2'-b-mercaptocytidine, 2'-deoxy-2'-b-thiomethoxycytidine, 2'-deoxy-2'-b-azidocytidine, 2'-deoxy-2'-b-aminocytidine, 2'-deoxy-2'-b-mercaptoadenosine, 2'-deoxy-2'-b-thiomethoxyadenosine, 2'-deoxy-2'-b-azidoadenosine, 2'-deoxy-2'-b-aminoadenosine, 2'-deoxy-2'-b-mercaptoguanosine, 2'-deoxy-2'-b-thiomethoxyguanosine, 2'-deoxy-2'-b-azidoguanosine, 2'-deoxy-2'-b-aminoguanosine, 2'-b-trifluoromethyladenosine, 2'-b-trifluoromethylcytidine, 2'-b-trifluoromethylguanosine, 2'-b-trifluoromethyluridine, 2'-a-trifluoromethyladenosine, 2'-a-trifluoromethylcytidine, 2'-a-trifluoromethylguanosine, 2'-a-trifluoromethyluridine, 2'-b-ethynyladenosine, 2'-b-ethynylcytidine, 2'-b-ethynylguanosine, 2'-b-ethynyluridine, 2'-a-ethynyladenosine, 2'-a-ethynylcytidine, 2'-a-ethynylguanosine, 2'-a-ethynyluridine, (E)-5-(2-bromo-vinyl)cytidine, 2-trifluoromethyladenosine, 2-mercaptoadenosine, 2-aminoadenosine, 2-azidoadenosine, 2-fluoroadenosine, 2-chloroadenosine, 2-bromoadenosine, 2-iodoadenosine, formycin A, formycin B, oxoformycin, pyrrolosine, 9-deazaadenosine, 9-deazaguanosine, 3-deazaadenosine, 3-deaza-3-fluoroadenosine, 3-deaza-3-chloroadenosine, 3-deaza-3-bromoadenosine, 3-deaza-3-iodoadenosine, 1-deazaadenosine, or combinations thereof.

In some aspects, the candidate nucleic acid sequence is chemically modified prior to optimization. Accordingly, in some cases, the candidate nucleic sequence comprises a certain chemical modification (e.g., substitution of all uridines with 4-thiouridine), and all subsequent optimization steps would be conducted using the nucleic acid sequence with the initial chemical modification.

In other aspects, chemical modification is one of the parameters that can be varied during the optimization process. Accordingly, a sequence initially comprising no substitution may be subjected to different chemical substitution strategies during optimization. For example, a library of variants may be generated during optimization in which each member had a different percentage of 4-thiouridine substitution.

In yet other aspects, the candidate nucleic acid sequence can be chemically modified after optimization, i.e., a nucleic acid sequence can be optimized without any chemical modifications and a preferred chemical modification can be then incorporated into the optimized nucleic acid sequence. As an alternative approach, an optimized nucleic acid sequence prepared according to the methods disclosed herein can be subjected to one or more rounds of chemical optimization.

In some aspects, the optimized nucleic acid is an mRNA. In some aspects, the optimized nucleic acid is an mRNA encoding the same amino acid sequence as the candidate nucleic sequence (e.g., a wild type mRNA sequence) sharing at least about 55%, sequence identity with the candidate nucleic acid sequence. In some aspects, the level of sequence identity between the optimized nucleic acid sequence and the candidate nucleic acid sequence is at least about 60%, at least about 65%, at least about 70%, at least about 75%, at least about 80%, at least about 85%, at least about 90%, at least about 95%, at least about 96%, at least about 97%, at least 98%, or at least about 99%.

In some aspects, the optimized nucleic acid (e.g., an mRNA) comprises at least one nucleotide analogue. wherein at least one nucleotide analogue is selected from the group consisting of a 2'-O-methoxyethyl-RNA (2'-MOE-RNA) monomer, a 2'-fluoro-DNA monomer, a 2'-O-alkyl-RNA monomer, a 2'-amino-DNA monomer, a locked nucleic acid (LNA) monomer, a cEt monomer, a cMOE monomer, a 5'-Me-LNA monomer, a 2'-(3-hydroxy)propyl-RNA monomer, an arabino nucleic acid (ANA) monomer, a 2'-fluoro-ANA monomer, an anhydrohexitol nucleic acid (HNA) monomer, an intercalating nucleic acid (INA) monomer, and a combination of two or more of said nucleotide analogues. In some aspects, the optimized nucleic acid molecule comprises at least one backbone modification, for example, a phosphorothioate internucleotide linkage.

In some aspects, an isolated molecule disclosed herein (e.g., a candidate nucleic acid molecule or an optimized nucleic acid molecule) comprises at least one nucleoside selected from the group consisting of 2-pseudouridine, 5-methoxyuridine, 2-thiouridine, 4-thiouridine, N1-methylpseudouridine, 5-aza-uridine, 2-thio-5-aza-uridine, 4-thio-pseudouridine, 2-thio-pseudouridine, 5-hydroxyuridine, 4-methoxy-pseudouridine, 4-methoxy-2-thio-pseudouridine, 3-methyluridine, 5-carboxymethyl-uridine, 1-carboxymethyl-pseudouridine, 5-propynyl-uridine, 1-propynyl-pseudouridine, 2-methoxy-4-thio-uridine, 5-taurinomethyluridine, 1-taurinomethyl-pseudouridine, 5-taurinomethyl-2-thio-uridine, 1-taurinomethyl-4-thio-uridine, 5-methyl-uridine, 2-methoxyuridine, 1-methyl-pseudouridine, 4-thio-1-methyl-pseudouridine, 2-thio-1-methyl-pseudouridine, 1-methyl-1-deaza-pseudouridine, 2-thio-1-methyl-1-deaza-pseudouridine, 2-thio-dihydrouridine.

In some aspects, an isolated molecule disclosed herein (e.g., a candidate nucleic acid molecule or an optimized nucleic acid molecule) comprises at least one nucleoside selected from the group consisting of 2-aminopurine, 2,6-diaminopurine, 7-deaza-adenine, 7-deaza-8-aza-adenine, 7-deaza-2-aminopurine, 7-deaza-8-aza-2-aminopurine, 7-deaza-2,6-diaminopurine, 7-deaza-8-aza-2,6-diaminopurine, 1-methyladenosine, N6-methyladenosine, N6-isopentenyladenosine, N6-(cis-hydroxyisopentenyl)adenosine, 2-methylthio-N6-(cis-hydroxyisopentenyl)adenosine, N6-glycinylcarbamoyladenosine, N6-threonylcarbamoyladenosine, 2-methylthio-N6-threonyl carbamoyladenosine, N6,N6-dimethyladenosine, or 7-methyladenine.

In some aspects, an isolated molecule disclosed herein (e.g., a candidate nucleic acid molecule or an optimized nucleic acid molecule) comprises at least one nucleoside selected from the group consisting of inosine, 1-methyl-inosine, wyosine, wybutosine, 7-deaza-guanosine, 7-deaza-8-aza-guanosine, 6-thio-guanosine, 6-thio-7-deaza-guanosine, 6-thio-7-deaza-8-aza-guanosine, 7-methyl-guanosine, 6-thio-7-methyl-guanosine, 7-methylinosine, 6-methoxy-guanosine, 1-methylguanosine, N2-methylguanosine, N2,N2-dimethylguanosine, 8-oxo-guanosine, 7-methyl-8-oxo-guanosine, and 1-methyl-6-thio-guanosine.

In some aspects, an isolated molecule disclosed herein (e.g., a candidate nucleic acid molecule or an optimized nucleic acid molecule) comprises at least one nucleoside selected from the group consisting of 5-methylcytidine, 5-aza-cytidine, pseudoisocytidine, 3-methyl-cytidine, N4-acetylcytidine, 5-formylcytidine, N4-methylcytidine, 5-hydroxymethylcytidine, 1-methyl-pseudoisocytidine, pyrrolo-cytidine, pyrrolo-pseudoisocytidine, 2-thio-cytidine, 2-thio-5-methyl-cytidine, 4-thio-pseudoisocytidine, 4-thio-1-methyl-pseudoisocytidine, 4-thio-1-methyl-1-deaza-pseudoisocytidine, 1-methyl-1-deaza-pseudoisocytidine, zebularine, 5-aza-zebularine, 5-methyl-zebularine, 5-aza-2-thio-zebularine, 2-thio-zebularine, 2-methoxy-cytidine, 2-methoxy-5-methyl-cytidine, and 4-methoxy-pseudoisocytidine.

In some aspects, at least one uridine in an isolated molecule disclosed herein (e.g., a candidate nucleic acid molecule or an optimized nucleic acid molecule) has been replaced with pseudouridine, 5-methoxyuridine, 2-thiouridine, 4-thiouridine, N1-methylpseudouridine, or 5-aza-uridine.

In some aspects, at least one uridine in an isolated molecule disclosed herein (e.g., a candidate nucleic acid molecule or an optimized nucleic acid molecule) has been replaced with 2-thio-5-aza-uridine, 4-thio-pseudouridine, 2-thio-pseudouridine, 5-hydroxyuridine, 4-methoxy-pseudouridine, or 4-methoxy-2-thio-pseudouridine.

In some aspects, at least one uridine in an isolated molecule disclosed herein (e.g., a candidate nucleic acid molecule or an optimized nucleic acid molecule) has been replaced with 3-methyluridine, 5-carboxymethyl-uridine, 1-carboxymethyl-pseudouridine, 5-propynyl-uridine, 1-propynyl-pseudouridine, or 2-methoxy-4-thio-uridine.

In some aspects, at least one uridine in an isolated molecule disclosed herein (e.g., a candidate nucleic acid molecule or an optimized nucleic acid molecule) has been replaced with 5-taurinomethyluridine, 1-taurinomethyl-pseudouridine, 5-taurinomethyl-2-thio-uridine, 1-taurinomethyl-4-thio-uridine, 5-methyl-uridine, or 2-methoxyuridine.

In some aspects, at least one uridine in an isolated molecule disclosed herein (e.g., a candidate nucleic acid molecule or an optimized nucleic acid molecule) has been replaced with 1-methyl-pseudouridine, 4-thio-1-methyl-pseudouridine, 2-thio-1-methyl-pseudouridine, 1-methyl-1-deaza-pseudouridine, 2-thio-1-methyl-1-deaza-pseudouridine, or 2-thio-dihydrouridine.

In some aspects, at least one adenosine in an isolated molecule disclosed herein (e.g., a candidate nucleic acid molecule or an optimized nucleic acid molecule) has been replaced with 2-aminopurine, 2,6-diaminopurine, 7-deaza-adenine, 7-deaza-8-aza-adenine, 7-deaza-2-aminopurine, or 7-deaza-8-aza-2-aminopurine.

In some aspects, at least one adenosine in an isolated molecule disclosed herein (e.g., a candidate nucleic acid molecule or an optimized nucleic acid molecule) has been replaced with 2-aminopurine, 2,6-diaminopurine, 7-deaza-adenine, 7-deaza-8-aza-adenine, 7-deaza-2-aminopurine, or 7-deaza-8-aza-2-aminopurine.

In some aspects, at least one adenosine in an isolated molecule disclosed herein (e.g., a candidate nucleic acid molecule or an optimized nucleic acid molecule) has been replaced with 7-deaza-2,6-diaminopurine, 7-deaza-8-aza-2,6-diaminopurine, 1-methyladenosine, N6-methyladenosine, N6-isopentenyladenosine, or N6-(cis-hydroxyisopentenyl)adenosine.

In some aspects, at least one adenosine in an isolated molecule disclosed herein (e.g., a candidate nucleic acid molecule or an optimized nucleic acid molecule) has been replaced with 2-aminopurine, 2,6-diaminopurine, 7-deaza-adenine, 7-deaza-8-aza-adenine, 7-deaza-2-aminopurine, or 7-deaza-8-aza-2-aminopurine.

In some aspects, at least one adenosine in an isolated molecule disclosed herein (e.g., a candidate nucleic acid molecule or an optimized nucleic acid molecule) has been replaced with 2-methylthio-N6-(cis-hydroxyisopentenyl) adenosine, N6-glycinylcarbamoyladenosine, N6-threonylcarbamoyladenosine, 2-methylthio-N6-threonyl carbamoyladenosine, N6,N6-dimethyladenosine, or 7-methyladenine.

In some aspects, at least one guanosine in an isolated molecule disclosed herein (e.g., a candidate nucleic acid molecule or an optimized nucleic acid molecule) has been replaced with inosine, 1-methyl-inosine, wyosine, wybutosine, 7-deaza-guanosine, 7-deaza-8-aza-guanosine, or 6-thio-guanosine.

In some aspects, at least one guanosine in an isolated molecule disclosed herein (e.g., a candidate nucleic acid molecule or an optimized nucleic acid molecule) has been replaced with 6-thio-7-deaza-guanosine, 6-thio-7-deaza-8-aza-guanosine, 7-methyl-guanosine, 6-thio-7-methyl-guanosine, 7-methylinosine, or 6-methoxy-guanosine.

In some aspects, at least one guanosine in an isolated molecule disclosed herein (e.g., a candidate nucleic acid molecule or an optimized nucleic acid molecule) has been replaced with 1-methylguanosine, N2-methylguanosine, N2,N2-dimethylguanosine, 8-oxo-guanosine, 7-methyl-8-oxo-guanosine, or 1-methyl-6-thio-guanosine.

In some aspects, at least one cytidine in an isolated molecule disclosed herein (e.g., a candidate nucleic acid molecule or an optimized nucleic acid molecule) has been replaced with 5-methylcytidine, 5-aza-cytidine, pseudoisocytidine, 3-methyl-cytidine, N4-acetylcytidine, or 5-formylcytidine.

In some aspects, at least one cytidine in an isolated molecule disclosed herein (e.g., a candidate nucleic acid molecule or an optimized nucleic acid molecule) has been replaced with N4-methylcytidine, 5-hydroxymethylcytidine, 1-methyl-pseudoisocytidine, pyrrolo-cytidine, pyrrolo-pseudoisocytidine, or 2-thio-cytidine.

In some aspects, at least one cytidine in an isolated molecule disclosed herein (e.g., a candidate nucleic acid molecule or an optimized nucleic acid molecule) has been replaced with 2-thio-5-methyl-cytidine, 4-thio-pseudoisocytidine, 4-thio-1-methyl-pseudoisocytidine, 4-thio-1-methyl-1-deaza-pseudoisocytidine, 1-methyl-1-deaza-pseudoisocytidine, or zebularine.

In some aspects, at least one cytidine in an isolated molecule disclosed herein (e.g., a candidate nucleic acid molecule or an optimized nucleic acid molecule) has been replaced with 5-aza-zebularine, 5-methyl-zebularine, 5-aza-2-thio-zebularine, 2-thio-zebularine, 2-methoxy-cytidine, or 2-methoxy-5-methyl-cytidine.

In some aspects, 100% of the uridine nucleosides in an isolated molecule disclosed herein (e.g., a candidate nucleic acid molecule or an optimized nucleic acid molecule) have been replaced with a nucleoside selected from the group consisting of pseudouridine, 5-methoxyuridine, 2-thiouridine, 4-thiouridine, N1-methylpseudouridine, 5-aza-uridine, 2-thio-5-aza-uridine, 4-thio-pseudouridine, 2-thio-pseudouridine, 5-hydroxyuridine, 4-methoxy-pseudouridine, 4-methoxy-2-thio-pseudouridine, 3-methyluridine, 5-carboxymethyl-uridine, 1-carboxymethyl-pseudouridine, 5-propynyl-uridine, 1-propynyl-pseudouridine, 2-methoxy-4-thio-uridine, 5-taurinomethyluridine, 1-taurinomethyl-pseudouridine, 5-taurinomethyl-2-thio-uridine, 1-taurinomethyl-4-thio-uridine, 5-methyl-uridine, 2-methoxyuridine, 1-methyl-pseudouridine, 4-thio-1-methyl-pseudouridine, 2-thio-1-methyl-pseudouridine, 1-methyl-1-deaza-pseudouridine, 2-thio-1-methyl-1-deaza-pseudouridine, or 2-thio-dihydrouridine.

In some aspects, 100% of the adenosine nucleosides in an isolated molecule disclosed herein (e.g., a candidate nucleic acid molecule or an optimized nucleic acid molecule) have been replaced with a nucleoside selected from the group consisting of 2-aminopurine, 2,6-diaminopurine, 7-deaza-adenine, 7-deaza-8-aza-adenine, 7-deaza-2-aminopurine, 7-deaza-8-aza-2-aminopurine, 7-deaza-2,6-diaminopurine, 7-deaza-8-aza-2,6-diaminopurine, 1-methyladenosine, N6-methyladenosine, N6-isopentenyladenosine, N6-(cis-hydroxyisopentenyl)adenosine, 2-methylthio-N6-(cis-hydroxyisopentenyl)adenosine, N6-glycinylcarbamoyladenosine, N6-threonylcarbamoyladenosine, 2-methylthio-N6-threonyl carbamoyladenosine, N6,N6-dimethyladenosine, or 7-methyladenine.

In some aspects, 100% of the guanosine nucleosides in an isolated molecule disclosed herein (e.g., a candidate nucleic acid molecule or an optimized nucleic acid molecule) have been replaced with a nucleoside selected from the group consisting of inosine, 1-methyl-inosine, wyosine, wybutosine, 7-deaza-guanosine, 7-deaza-8-aza-guanosine, 6-thio-guanosine, 6-thio-7-deaza-guanosine, 6-thio-7-deaza-8-aza-guanosine, 7-methyl-guanosine, 6-thio-7-methyl-guanosine, 7-methylinosine, 6-methoxy-guanosine, 1-methylguanosine, N2-methylguanosine, N2,N2-dimethylguanosine, 8-oxo-guanosine, 7-methyl-8-oxo-guanosine, or 1-methyl-6-thio-guanosine.

In some aspects, 100% of the uridine nucleosides in an isolated molecule disclosed herein (e.g., a candidate nucleic acid molecule or an optimized nucleic acid molecule) have been replaced with a nucleoside selected from the group consisting of 5-methylcytidine, 5-aza-cytidine, pseudoisocytidine, 3-methyl-cytidine, N4-acetylcytidine, 5-formylcytidine, N4-methylcytidine, 5-hydroxymethylcytidine, 1-methyl-pseudoisocytidine, pyrrolo-cytidine, pyrrolo-pseudoisocytidine, 2-thio-cytidine, 2-thio-5-methyl-cytidine, 4-thio-pseudoisocytidine, 4-thio-1-methyl-pseudoisocytidine, 4-thio-1-methyl-1-deaza-pseudoisocytidine, 1-methyl-1-deaza-pseudoisocytidine, zebularine, 5-aza-zebularine, 5-methyl-zebularine, 5-aza-2-thio-zebularine, 2-thio-zebularine, 2-methoxy-cytidine, 2-methoxy-5-methyl-cytidine, 4-methoxy-pseudoisocytidine, or 4-methoxy-1-methyl-pseudoisocytidine.

In some aspects, at least 25%, at least 50%, at least 75% or at least 100% of uridines in an isolated molecule disclosed herein (e.g., a candidate nucleic acid molecule or an optimized nucleic acid molecule) have been replaced with pseudouridine.

In some aspects, at least 25%, at least 50%, at least 75% or at least 100% of uridines in an isolated molecule disclosed herein (e.g., a candidate nucleic acid molecule or an optimized nucleic acid molecule) have been replaced with 2-thiouridine.

In some aspects, at least 25%, at least 50%, at least 75% or at least 100% of uridines in an isolated molecule disclosed herein (e.g., a candidate nucleic acid molecule or an optimized nucleic acid molecule) have been replaced with 4-thiouridine.

In some aspects, at least 25%, at least 50%, at least 75% or at least 100% of uridines in an isolated molecule disclosed herein (e.g., a candidate nucleic acid molecule or an optimized nucleic acid molecule) have been replaced with 5-methoxyuridine.

In some aspects, at least 25%, at least 50%, at least 75% or at least 100% of uridines in an isolated molecule disclosed herein (e.g., a candidate nucleic acid molecule or an optimized nucleic acid molecule) have been replaced with 4-methoxy-2-thio-pseudouridine.

In some aspects, at least 25%, at least 50%, at least 75% or at least 100% of uridines in an isolated molecule disclosed herein (e.g., a candidate nucleic acid molecule or an optimized nucleic acid molecule) have been replaced with 4-methoxy-pseudouridine.

In some aspects, at least 25%, at least 50%, at least 75% or at least 100% of uridines in an isolated molecule disclosed herein (e.g., a candidate nucleic acid molecule or an optimized nucleic acid molecule) have been replaced with 5-hydroxyuridine.

In some aspects, at least 25%, at least 50%, at least 75% or at least 100% of uridines in an isolated molecule disclosed herein (e.g., a candidate nucleic acid molecule or an optimized nucleic acid molecule) have been replaced with 2-thio-pseudouridine.

In some aspects, at least 25%, at least 50%, at least 75% or at least 100% of uridines in an isolated molecule disclosed herein (e.g., a candidate nucleic acid molecule or an optimized nucleic acid molecule) have been replaced with 2-thio-5-aza-uridine.

In some aspects, at least 25%, at least 50%, at least 75% or at least 100% of uridines in an isolated molecule disclosed herein (e.g., a candidate nucleic acid molecule or an optimized nucleic acid molecule) have been replaced with 1-carboxymethyl-pseudouridine.

In some aspects, at least 25%, at least 50%, at least 75% or at least 100% of uridines in an isolated molecule disclosed herein (e.g., a candidate nucleic acid molecule or an optimized nucleic acid molecule) have been replaced N1-methylpseudouridine.

In some aspects, at least 25%, at least 50%, at least 75% or at least 100% of cytidines in an isolated molecule disclosed herein (e.g., a candidate nucleic acid molecule or an optimized nucleic acid molecule) have been replaced 5-methylcytidine or 3-methyl-cytidine.

In some aspects, the optimized nucleic acid sequence comprises only uridine substitutions. In some aspects, the optimized nucleic acid sequence comprises only cytidine substitutions. In some aspects, the optimized nucleic acid sequence comprises only guanosine substitutions. In some aspects, the optimized nucleic acid sequence comprises only adenosine substitutions.

In other aspects, the optimized nucleic acid sequence comprises only uridine substitutions and cytidine substitutions. In other aspects, the optimized nucleic acid sequence comprises only uridine substitutions and guanosine substitutions. In other aspects, the optimized nucleic acid comprises only uridine substitutions and adenosine substitutions.

In some aspects, 25%, 50%, 75%, or 100% of uridines in the candidate nucleic acid sequence are replaced by 4-thiouridine in the optimized nucleic acid sequence. In some specific aspects, the generation the optimized nucleic acid sequence further comprises the replacement of at least one cytidine with 5-methylcytidine. In some specific aspects, in addition to 4-thiouridine substitutions, 25%, 50%, 75%, or 100% of cytidines in the candidate nucleic acid sequence are replaced with 5-methylcytidine in the optimized nucleic acid sequence. In a specific aspect, 25% of uridines in the candidate nucleic acid sequence are replaced with 4-thiouridine in the optimized nucleic acid sequence and 25% of cytidines in the candidate nucleic acid sequence are replaced with 5-methylcytidine in the optimized nucleic acid sequence. In a specific aspect, 25% of uridines in the candidate nucleic acid sequence are replaced with 4-thiouridine in the optimized nucleic acid sequence and 50% of cytidines in the candidate nucleic acid sequence are replaced with 5-methylcytidine (m5C) in the optimized nucleic acid sequence. In a specific aspect, 25% of uridines in the candidate nucleic acid sequence are replaced with 4-thiouridine in the optimized nucleic acid sequence and 100% of cytidines in the candidate nucleic acid sequence are replaced with 5-methylcytidine (m5C) in the optimized nucleic acid sequence. In a specific aspect, 100% of uridines in the candidate nucleic acid sequence are replaced with 4-thiouridine in the optimized nucleic acid sequence, but no cytidines are replaced in the candidate nucleic acid sequence. In a specific aspect, 100%, of uridines in the candidate nucleic acid sequence are replaced with 4-thiouridine in the optimized nucleic acid sequence and 100% of cytidines in the candidate nucleic acid sequence are replaced with 5-methylcytidine (m5C) in the optimized nucleic acid sequence.

In other aspects, 25%, 50%, 75% or 100% of uridines in the candidate nucleic acid sequence are replaced with 2-thiouridine in the optimized nucleic acid sequence. In some specific aspects, the generation the optimized nucleic acid sequence further comprises the replacement of at least one cytidine with 5-methylcytidine. In some specific aspects, in addition to 2-thiouridine substitutions, 25%, 50%, 75%, or 100% of cytidines in the candidate nucleic acid sequence are replaced with 5-methylcytidine in the optimized nucleic acid sequence. In a specific aspect, 25% of uridines in the candidate nucleic acid sequence are replaced with 2-thiouridine in the optimized nucleic acid sequence and 25% of cytidines in the candidate nucleic acid sequence are replaced with 5-methylcytidine in the optimized nucleic acid sequence. In a specific aspect, 25% of uridines in the candidate nucleic acid sequence are replaced with 2-thiouridine in the optimized nucleic acid sequence and 50% of cytidines in the candidate nucleic acid sequence are replaced with 5-methylcytidine (m5C) in the optimized nucleic acid sequence. In a specific aspect, 25% of uridines in the candidate nucleic acid sequence are replaced with 2-thiouridine in the optimized nucleic acid sequence and 100% of cytidines in the candidate nucleic acid sequence are replaced with 5-methylcytidine (m5C) in the optimized nucleic acid sequence. In a specific aspect, 100% of uridines in the candidate nucleic acid sequence are replaced with 2-thiouridine in the optimized nucleic acid sequence, but no cytidines are replaced in the candidate nucleic acid sequence. In a specific aspect, 100% of uridines in the candidate nucleic acid sequence are replaced with 2-thiouridine in the optimized nucleic acid sequence and 100% of cytidines in the candidate nucleic acid sequence are replaced with 5-methylcytidine (m5C) in the optimized nucleic acid sequence.

In other aspects, 25%, 50%, 75% or 100% of uridines in the candidate nucleic acid sequence are replaced with pseudouridine in the optimized nucleic acid sequence. In some specific aspects, the generation the optimized nucleic acid sequence further comprises the replacement of at least one cytidine with 5-methylcytidine. In some specific aspects, in addition to pseudouridine substitutions, 25%, 50%, 75%, or 100% of cytidines in the candidate nucleic acid sequence are replaced with 5-methylcytidine in the optimized nucleic acid sequence. In a specific aspect, 25% of uridines in the candidate nucleic acid sequence are replaced with pseudouridine in the optimized nucleic acid sequence and 25% of cytidines in the candidate nucleic acid sequence are replaced with 5-methylcytidine in the optimized nucleic acid sequence. In a specific aspect, 25% of uridines in the candidate nucleic acid sequence are replaced with pseudouridine in the optimized nucleic acid sequence and 50% of cytidines in the candidate nucleic acid sequence are replaced with 5-methylcytidine (m5C) in the optimized nucleic acid sequence. In a specific aspect, 25% of uridines in the candidate nucleic acid sequence are replaced with pseudouridine in the optimized nucleic acid sequence and 100% of cytidines in the candidate nucleic acid sequence are replaced with 5-methylcytidine (m5C) in the optimized nucleic acid sequence. In a specific aspect, 100% of uridines in the candidate nucleic acid sequence are replaced with pseudouridine in the optimized nucleic acid sequence, but no cytidines are replaced in the candidate nucleic acid sequence. In a specific aspect, 100% of uridines in the candidate nucleic acid sequence are replaced with pseudouridine in the optimized nucleic acid sequence and 100% of cytidines in the candidate nucleic acid sequence are replaced with 5-methylcytidine (m5C) in the optimized nucleic acid sequence.

In other aspects, 25%, 50%, 75% or 100% of uridines in the candidate nucleic acid sequence are replaced with 5-methoxyuridine in the optimized nucleic acid sequence. In some specific aspects, the generation the optimized nucleic acid sequence further comprises the replacement of at least one cytidine with 5-methylcytidine. In some specific aspects, in addition to 5-methoxyuridine substitutions, 25%, 50%, 75%, or 100% of cytidines in the candidate nucleic acid sequence are replaced with 5-methylcytidine in the optimized nucleic acid sequence. In a specific aspect, 25% of uridines in the candidate nucleic acid sequence are replaced with 5-methoxyuridine in the optimized nucleic acid sequence and 25% of cytidines in the candidate nucleic acid sequence are replaced with 5-methylcytidine (m5C) in the optimized nucleic acid sequence. In a specific aspect, 25% of uridines in the candidate nucleic acid sequence are replaced with 5-methoxyuridine in the optimized nucleic acid sequence and 50% of cytidines in the candidate nucleic acid sequence are replaced with 5-methylcytidine in the optimized nucleic acid sequence. In another aspect, 25% of uridines in the candidate nucleic acid sequence are replaced with 5-methoxyuridine in the optimized nucleic acid sequence and 100% of cytidines in the candidate nucleic acid sequence are replaced with 5-methylcytidine in the optimized nucleic acid sequence. In a specific aspect, 100% of uridines in the candidate nucleic acid sequence are replaced with 5-methoxyuridine in the optimized nucleic acid sequence, but no cytidines are replaced in the candidate nucleic acid sequence. In another aspect, 100% of uridines in the candidate nucleic acid sequence are replaced with 5-methoxyuridine in the optimized nucleic acid sequence and 100% of cytidines in the candidate nucleic acid sequence are replaced with 5-methylcytidine (m5C) in the optimized nucleic acid sequence.

In other aspects, 25%, 50%, 75% or 100% of uridines in the candidate nucleic acid sequence are replaced with N1-methylpseudouridine in the optimized nucleic acid sequence. In some specific aspects, the generation the optimized nucleic acid sequence further comprises the replacement of at least one cytidine with 5-methylcytidine. In some specific aspects, in addition to N1-methylpseudouridine substitutions, 25%, 50%, 75%, or 100% of cytidines in the candidate nucleic acid sequence are replaced with 5-methylcytidine in the optimized nucleic acid sequence. In a specific aspect, 25% of uridines in the candidate nucleic acid sequence are replaced with N1-methylpseudouridine in the optimized nucleic acid sequence and 25% of cytidines in the candidate nucleic acid sequence are replaced with 5-methylcytidine in the optimized nucleic acid sequence. In a specific aspect, 25% of uridines in the candidate nucleic acid sequence are replaced with N1-methylpseudouridine in the optimized nucleic acid sequence and 50% of cytidines in the candidate nucleic acid sequence are replaced with 5-methylcytidine in the optimized nucleic acid sequence. In a specific aspect, 25% of uridines in the candidate nucleic acid sequence are replaced with N1-methylpseudouridine in the optimized nucleic acid sequence and 100% of cytidines in the candidate nucleic acid sequence are replaced with 5-methylcytidine in the optimized nucleic acid sequence. In another specific aspect, 100% of uridines in the candidate nucleic acid sequence are replaced with N1-methylpseudouridine in the optimized nucleic acid sequence, but no cytidines are replaced in the candidate nucleic acid sequence. In a specific aspect, 100% of uridines in the candidate nucleic acid sequence are replaced with N1-methylpseudouridine in the optimized nucleic acid sequence and 100% of cytidines in the candidate nucleic acid sequence are replaced with 5-methylcytidine in the optimized nucleic acid sequence.

In specific aspects, the present disclosure provides mRNA sequences (e.g., candidate nucleic acid sequences or nucleic acid sequences optimized according to the multiparametric nucleic acid optimization methods disclosed herein) wherein between 25% and 100% of uridines in the nucleic acid sequence are replaced with 5-methoxyuridine. In some aspects, at least about 25%, at least about 30%, at least about 35%, at least about 40%, at least about 45%, at least about 50%, at least about 55%, at least about 60%, at least about 65%, at least about 70%, at least about 75%, at least about 80%, at least about 85%, at least about 90%, at least about 91%, at least about 92%, at least about 93%, at least about 94%, at least about 95%, at least about 96%, at least about 97%, at least about 98%, at least about 99%, or 100% of uridines in the nucleic acid sequence are replaced with 5-methoxyuridine. In specific aspects, the nucleic acid sequence comprises about 25%, 26%, 27%, 28%, 29%, 30%, 31%, 42%, 33%, 34%, 35%, 36%, 37%, 38%, 39%, 40%, 41%, 42%, 43%, 44%, 45%, 46%, 47%, 48%, 49%, 50%,. 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99% or 100% of uridines in the nucleic acid sequence replaced with 5-methoxyuridine. In some aspects, no other nucleosides are replaced in the nucleic acid sequence, thus, in some aspects the nucleic acid sequence comprises 25%, 50%, 75%, or 100% of uridines in the nucleic acid sequence replaced with 5-methoxyuridine and no other nucleosides are replaced by either natural or non-natural nucleosides. In other aspects, other nucleosides are replaced in the nucleic acid sequence. In one specific aspects, cytidines are replaced with 5-methylcytidine. Thus, some aspects, the nucleic acid sequence comprises the 5-methoxyuridine substitution disclosed above, and further comprises about 1%, 2%, 3%, 4%, 5%, 6%, 7%, 8%, 9%, 10%, 11%, 12%, 13%, 14%, 15%, 16%, 17%, 18%, 19%, 20%, 21%, 22%, 23%, 24%, 25%, 26%, 27%, 28%, 29%, 30%, 31%, 32%, 33%, 34%, 35%, 36%, 37%, 38%, 39%, 40%, 41%, 42%, 43%, 44%, 45%, 46%, 47%, 48%, 49%, 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99% or 100% of cytidines in the nucleic acid sequence replaced with 5-methylcytidine. In some specific aspects, no other nucleobases are replaced in the nucleic acid sequence in addition to the 5-methoxyuridine and 5-methylcytidine disclosed above.

In specific aspects, the present disclosure provides mRNA sequences (e.g., candidate nucleic acid sequences or nucleic acid sequences optimized according to the multiparametric nucleic acid optimization methods disclosed herein) wherein between 25% and 100% of uridines in the nucleic acid sequence are replaced with 4-thiouridine. In some aspects, at least about 25%, at least about 30%, at least about 35%, at least about 40%, at least about 45%, at least about 50%, at least about 55%, at least about 60%, at least about 65%, at least about 70%, at least about 75%, at least about 80%, at least about 85%, at least about 90%, at least about 91%, at least about 92%, at least about 93%, at least about 94%, at least about 95%, at least about 96%, at least about 97%, at least about 98%, at least about 99%, or 100% of uridines in the nucleic acid sequence are replaced with 4-thiouridine. In specific aspects, the nucleic acid sequence comprises about 25%, 26%, 27%, 28%, 29%, 30%, 31%, 42%, 33%, 34%, 35%, 36%, 37%, 38%, 39%, 40%, 41%, 42%, 43%, 44%, 45%, 46%, 47%, 48%, 49%, 50%,. 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99% or 100% of uridines in the nucleic acid sequence replaced with 4-thiouridine. In some aspects, no other nucleosides are replaced in the nucleic acid sequence, thus, in some aspects the nucleic acid sequence comprises 25%, 50%, 75%, or 100% of uridines in the nucleic acid sequence replaced with 4-thiouridine and no other nucleosides are replaced by either natural or non-natural nucleosides. In other aspects, other nucleosides are replaced in the nucleic acid sequence. In one specific aspects, cytidines are replaced with 5-methylcytidine. Thus, some aspects, the nucleic acid sequence comprises the 4-thiouridine substitution disclosed above, and further comprises about 1%, 2%, 3%, 4%, 5%, 6%, 7%, 8%, 9%, 10%, 11%, 12%, 13%, 14%, 15%, 16%, 17%, 18%, 19%, 20%, 21%, 22%, 23%, 24%, 25%, 26%, 27%, 28%, 29%, 30%, 31%, 32%, 33%, 34%, 35%, 36%, 37%, 38%, 39%, 40%, 41%, 42%, 43%, 44%, 45%, 46%, 47%, 48%, 49%, 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99% or 100% of cytidines in the nucleic acid sequence replaced with 5-methylcytidine. In some specific aspects, no other nucleobases are replaced in the nucleic acid sequence in addition to the 4-thiouridine and 5-methylcytidine disclosed above.

In specific aspects, the present disclosure provides mRNA sequences (e.g., candidate nucleic acid sequences or nucleic acid sequences optimized according to the multiparametric nucleic acid optimization methods disclosed herein) wherein between 25% and 100% of uridines in the nucleic acid sequence are replaced with 2-thiouridine. In some aspects, at least about 25%, at least about 30%, at least about 35%, at least about 40%, at least about 45%, at least about 50%, at least about 55%, at least about 60%, at least about 65%, at least about 70%, at least about 75%, at least about 80%, at least about 85%, at least about 90%, at least about 91%, at least about 92%, at least about 93%, at least about 94%, at least about 95%, at least about 96%, at least about 97%, at least about 98%, at least about 99%, or 100% of uridines in the nucleic acid sequence are replaced with 2-thiouridine. In specific aspects, the nucleic acid sequence comprises about 25%, 26%, 27%, 28%, 29%, 30%, 31%, 42%, 33%, 34%, 35%, 36%, 37%, 38%, 39%, 40%, 41%, 42%, 43%, 44%, 45%, 46%, 47%, 48%, 49%, 50%,. 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99% or 100% of uridines in the nucleic acid sequence replaced with 2-thiouridine. In some aspects, no other nucleosides are replaced in the nucleic acid sequence, thus, in some aspects the nucleic acid sequence comprises 25%, 50%, 75%, or 100% of uridines in the nucleic acid sequence replaced with 2-thiouridine and no other nucleosides are replaced by either natural or non-natural nucleosides. In other aspects, other nucleosides are replaced in the nucleic acid sequence. In one specific aspects, cytidines are replaced with 5-methylcytidine. Thus, some aspects, the nucleic acid sequence comprises the 2-thiouridine substitution disclosed above, and further comprises about 1%, 2%, 3%, 4%, 5%, 6%, 7%, 8%, 9%, 10%, 11%, 12%, 13%, 14%, 15%, 16%, 17%, 18%, 19%, 20%, 21%, 22%, 23%, 24%, 25%, 26%, 27%, 28%, 29%, 30%, 31%, 32%, 33%, 34%, 35%, 36%, 37%, 38%, 39%, 40%, 41%, 42%, 43%, 44%, 45%, 46%, 47%, 48%, 49%, 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99% or 100% of cytidines in the nucleic acid sequence replaced with 5-methylcytidine. In some specific aspects, no other nucleobases are replaced in the nucleic acid sequence in addition to the 2-thiouridine and 5-methylcytidine disclosed above.

In specific aspects, the present disclosure provides mRNA sequences (e.g., candidate nucleic acid sequences or nucleic acid sequences optimized according to the multiparametric nucleic acid optimization methods disclosed herein) wherein between 25% and 100% of uridines in the nucleic acid sequence are replaced with pseudouridine. In some aspects, at least about 25%, at least about 30%, at least about 35%, at least about 40%, at least about 45%, at least about 50%, at least about 55%, at least about 60%, at least about 65%, at least about 70%, at least about 75%, at least about 80%, at least about 85%, at least about 90%, at least about 91%, at least about 92%, at least about 93%, at least about 94%, at least about 95%, at least about 96%, at least about 97%, at least about 98%, at least about 99%, or 100% of uridines in the nucleic acid sequence are replaced with pseudouridine. In specific aspects, the nucleic acid sequence comprises about 25%, 26%, 27%, 28%, 29%, 30%, 31%, 42%, 33%, 34%, 35%, 36%, 37%, 38%, 39%, 40%, 41%, 42%, 43%, 44%, 45%, 46%, 47%, 48%, 49%, 50%,. 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99% or 100% of uridines in the nucleic acid sequence replaced with pseudouridine. In some aspects, no other nucleosides are replaced in the nucleic acid sequence, thus, in some aspects the nucleic acid sequence comprises 25%, 50%, 75%, or 100% of uridines in the nucleic acid sequence replaced with pseudouridine and no other nucleosides are replaced by either natural or non-natural nucleosides. In other aspects, other nucleosides are replaced in the nucleic acid sequence. In one specific aspects, cytidines are replaced with 5-methylcytidine. Thus, some aspects, the nucleic acid sequence comprises the pseudouridine substitution disclosed above, and further comprises about 1%, 2%, 3%, 4%, 5%, 6%, 7%, 8%, 9%, 10%, 11%, 12%, 13%, 14%, 15%, 16%, 17%, 18%, 19%, 20%, 21%, 22%, 23%, 24%, 25%, 26%, 27%, 28%, 29%, 30%, 31%, 32%, 33%, 34%, 35%, 36%, 37%, 38%, 39%, 40%, 41%, 42%, 43%, 44%, 45%, 46%, 47%, 48%, 49%, 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99% or 100% of cytidines in the nucleic acid sequence replaced with 5-methylcytidine. In some specific aspects, no other nucleobases are replaced in the nucleic acid sequence in addition to the pseudouridine and 5-methylcytidine disclosed above.

In specific aspects, the present disclosure provides mRNA sequences (e.g., candidate nucleic acid sequences or nucleic acid sequences optimized according to the multiparametric nucleic acid optimization methods disclosed herein) wherein between 25% and 100% of uridines in the nucleic acid sequence are replaced with N1-methylpseudouridine. In some aspects, at least about 25%, at least about 30%, at least about 35%, at least about 40%, at least about 45%, at least about 50%, at least about 55%, at least about 60%, at least about 65%, at least about 70%, at least about 75%, at least about 80%, at least about 85%, at least about 90%, at least about 91%, at least about 92%, at least about 93%, at least about 94%, at least about 95%, at least about 96%, at least about 97%, at least about 98%, at least about 99%, or 100% of uridines in the nucleic acid sequence are replaced with N1-methylpseudouridine. In specific aspects, the nucleic acid sequence comprises about 25%, 26%, 27%, 28%, 29%, 30%, 31%, 42%, 33%, 34%, 35%, 36%, 37%, 38%, 39%, 40%, 41%, 42%, 43%, 44%, 45%, 46%, 47%, 48%, 49%, 50%,. 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99% or 100% of uridines in the nucleic acid sequence replaced with N1-methylpseudouridine. In some aspects, no other nucleosides are replaced in the nucleic acid sequence, thus, in some aspects the nucleic acid sequence comprises 25%, 50%, 75%, or 100% of uridines in the nucleic acid sequence replaced with N1-methylpseudouridine and no other nucleosides are replaced by either natural or non-natural nucleosides. In other aspects, other nucleosides are replaced in the nucleic acid sequence. In one specific aspects, cytidines are replaced with 5-methylcytidine. Thus, some aspects, the nucleic acid sequence comprises the 5-methoxyuridine substitution disclosed above, and further comprises about 1%, 2%, 3%, 4%, 5%, 6%, 7%, 8%, 9%, 10%, 11%, 12%, 13%, 14%, 15%, 16%, 17%, 18%, 19%, 20%, 21%, 22%, 23%, 24%, 25%, 26%, 27%, 28%, 29%, 30%, 31%, 32%, 33%, 34%, 35%, 36%, 37%, 38%, 39%, 40%, 41%, 42%, 43%, 44%, 45%, 46%, 47%, 48%, 49%, 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99% or 100% of cytidines in the nucleic acid sequence replaced with 5-methylcytidine. In some specific aspects, no other nucleobases are replaced in the nucleic acid sequence in addition to the N1-methylpseudouridine and 5-methylcytidine disclosed above.

### V. Computational Model Construction, Computer Implemented Methods and Computer Readable Media

The present disclosure provides computer implemented multiparametric methods and systems for optimizing a nucleic acid sequence (e.g., an RNA or DNA sequence), for example, for translation efficacy (e.g., the translation efficacy of a therapeutic synthetic mRNA after administration to a subject in need thereof). These methods are in turn based on the application of discrete optimization methods based on the application, for example, of objective, probabilistic, multivariate statistical models. These models can comprise one or more than one modules implementing in a computer system the optimization methods disclosed herein.

In some aspects, the present disclosure provides a computer implemented multiparametric codon optimization method comprising:
(a) inputting at least one candidate nucleic acid sequence;
(b) applying a multiparametric codon optimization method according to any one of the embodiments disclosed herein to the candidate nucleic acid sequence; and,
(c) outputting at least one optimized nucleic acid sequence.

In some aspects, at least one optimized nucleic acid sequence output in step (c) is used as an inputting sequence in step (a). In some aspects, the method is executed recursively for at least 2, 3, 4, 5, 6, 7, 8, 9 or 10 cycles. In other aspects, the method is executed recursively for at least 10, at least 20, at least 30, at least 40, at least 50, at least 60, at least 70, at least 80, at least 90, or at least 100 cycles. In other aspects, the method is executed recursively for at least 200, at least 300, at least 400, at least 500, at least 600, at least 700, at least 800, at least 900, or at least 1000 cycles. In yet other aspects, the method is executed recursively for at least 2000, at least 3000, at least 4000, at least 5000, at least 6000, at least 7000, at least 8000, at least 9000, or at least 10000 cycles. In some aspects, the method further comprises submitting electronically the optimized nucleic acid sequence to an automated nucleic acid synthesizer. In some aspects, a library of candidate nucleic acid sequences is used as input in step (a). In some aspects, the output of step (c) is a library of optimized nucleic acid sequences.

In some aspects, the modeling comprises a plurality of values and each value in the plurality of values describes a relationship between a nucleic acid sequence property and an expression property; a plurality of nucleic acid sequence properties and an expression property; or a plurality of nucleic acid sequence properties and a plurality of expression properties. In some aspects, the modeling includes one or more refining steps, for example, computing a predicted score for a population of optimized nucleic acid sequences derived from the non-optimized nucleic acid sequence using the modeled sequence-expression relationship, wherein each optimized nucleic acid sequence in the population of optimized nucleic acid sequences includes a codon substitution at one or more codons in the non-optimized nucleic acid sequence, and then selecting the optimized nucleic sequence among the population of optimized nucleic acid sequences as a function of the predicted score assigned to each sequence in the set of optimized nucleic acid sequences.

In some aspects, the modeling comprises generating a set of optimized nucleic acid sequences comprising at least about 5, 10, 20, 30, 40, 50, 60, 70, 80, 90 or 100 optimized nucleic acid sequences. In other aspects, the modeling comprises generating a set of optimized nucleic acid sequences comprising at least 100, 200, 300, 400, 500, 600, 700, 800, 900, 1000, 1100, 1200, 1300, 1400, 1500, 1600, 1700, 1800, 1900 or 2000 optimized nucleic acid sequences. In yet other aspects, the modeling comprises generating a set of optimized nucleic acid sequences comprising 3000, 4000, 5000, 6000, 7000, 8000, 9000, 10000, 11000, 12000, 13000, 14000, 15000, 16000, 17000, 18000, 19000, or 20000 optimized nucleic acid sequences. In some aspects, the modeling comprises generating a set of at least 20000 optimized nucleic acid sequences.

In some aspects, the multiparametric methods disclosed herein comprise integrating modeling data corresponding to 2, 3, 4, 5, 6, 7, 8, 9, 10, 11, 12, 13, 14, 15, 16, 17, 18, 19, 20, 21, 22, 23, 24, or 25 parameters. In some aspects, all parameters are modeled using the same method (e.g., HMMs, SVMs, or neural networks). In some aspects, at least one parameter or subset of parameters is modeled using a modeling method different from the rest (e.g., one parameter may be modeled using an SVM whereas the rest of the parameters could be modeled using logistic regression). In some aspects, each parameter or group of parameters is assigned a certain weight.

Any suitable objective, probabilistic, multivariate statistical model known to one of skill in the art can be used to practice the methods and systems of the present disclosure. Non-limiting examples of the models that can be used to practice the methods of the present disclosure encompass supervised classification methods and include Fisher's Linear Discriminant Analysis, Logistic Regression, Naive Bayesian, K-nearest neighbor classifier, Artificial neural networks, and Classification trees. Machine learning methods suitable to practice the multiparametric nucleic acid optimization methods disclosed herein can include, for example, supervised learning methods (e.g., analytical learning, artificial neural networks, case-based reasoning, decision tree learning, inductive logic programming Gaussian process regression, gene expression programming, kernel estimators, support vector machines, random forests, ensembles of classifiers, etc.), unsupervised learning methods (e.g., neural networks with the self-organizing map (SOM) and adaptive resonance theory (ART)), semisupervised learning method (e.g., constrained clustering, PU learning), reinforced learning methods (e.g., Monte Carlo methods), transductive inference methods (e.g., transductive support vector machines, Bayesian Committee machines), or multi-task learning methods (e.g., clustered multi-task learning). In some aspects, the modeling comprises boosting or adaptive boosting.

In specific aspects, the present disclosure provides a computer implemented method comprising a multiparametric codon optimization method implemented is a swarm algorithm (see, e.g., U.S. Pat. No. 8,326,547). In some aspects, the swarm algorithm as a multi-swarm algorithm. In other aspects, the present disclosure provides a computer implemented method comprising a multiparametric codon optimization method implemented as a Bayesian optimization algorithm. In other aspects, the present disclosure provides a computer implemented method comprising a multiparametric codon optimization method implemented as a combinatorial optimization algorithm, In yet another aspect, the present disclosure provides a computer implemented method comprising a multiparametric codon optimization method implemented as a genetic algorithm. In specific aspects, the genetic algorithm is implemented in parallel. In some aspects, the genetic algorithm is a coarse-grained parallel genetic algorithm, whereas in other aspects the genetic algorithm is a fine-grained parallel genetic algorithm. In some aspects, the genetic algorithm comprises adaptive parameters.

Another aspect of the present disclosure provides a computer program product for use in conjunction with a computer system, the computer program product comprising a computer readable storage medium and a computer program mechanism embedded therein. The computer program mechanism can comprise instructions for carrying out any step of any method disclosed herein that does not involve expressing a protein or measuring an abundance of a protein. Still another aspect of the invention provides a computer system comprising a central processing unit and a memory, coupled to the central processing unit, the memory storing the aforementioned computer program product.

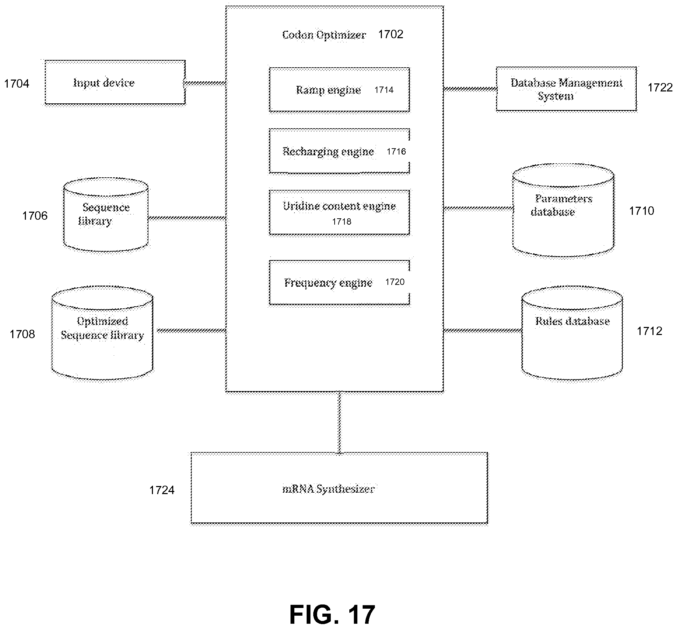

FIG. 17 is a block diagram of a codon optimization system 1700 according to an embodiment of the present invention. Codon optimization system 1700 includes a codon optimizer 1702, one or more input devices 1704, and one or more databases. The one or more databases may include, for example and without limitation, a sequence library 1706, an optimized sequence library 1708, a parameters database 1710, and a rules database 1712.

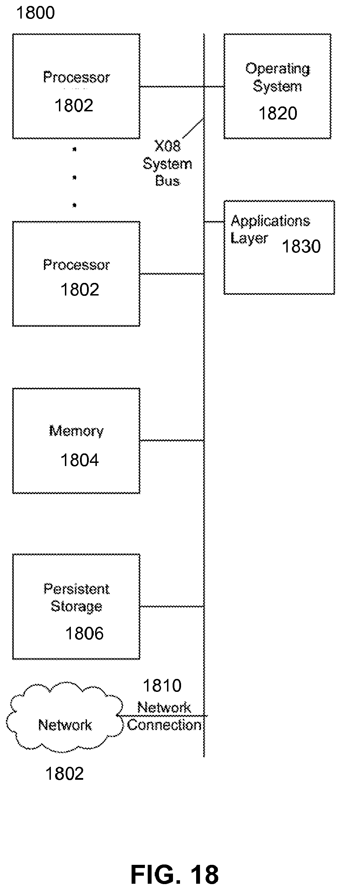

Codon optimizer 1702 executes a multiparametric method for nucleic acid optimization as disclosed above. Due to the complexity of the calculations involved, codon optimizer 1702 may be implemented on a computer specially programmed to conduct the complex optimization process. An example computing device is illustrated in FIG. 18. FIG. 18 illustrates a computing device 1800 having hardware elements that are electrically coupled via bus. Computing device 1800 accesses a network 1802 over a network connection 1810 that provides computing device 1800 with telecommunications capabilities. Computing device 1800 uses an operating system 1820 as software that manages hardware resources and coordinates the interface between hardware and software.

In an embodiment, computing device 1800 contains a combination of hardware, software, and firmware constituent parts that allow it to run an applications layer 1830. Computing device 1800, in embodiments, may be organized around a system bus 1808, but any type of infrastructure that allows the hardware infrastructure elements of computing device 1800 to communicate with and interact with each other may also be used.

Processing tasks in the embodiment of FIG. 18 are carried out by one or more processors 1802. However, it should be noted that various types of processing technology may be used here, including programmable logic arrays (PLAs), application-specific integrated circuits (ASICs), multi-core processors, multiple processors, or distributed processors. Additional specialized processing resources such as graphic, multimedia, or mathematical processing capabilities may also be used to aid in certain processing tasks. These processing resources may be hardware, software, or an appropriate combination thereof. For example, one or more of processors 1802 may be a graphics-processing unit (GPU). In an embodiment, a GPU is a processor that is a specialized electronic circuit designed to rapidly process mathematically intensive applications on electronic devices. The GPU may have a highly parallel structure that is efficient for parallel processing of large blocks of data, such as mathematically intensive data. Alternatively or in addition, one or more of processors 1802 may be a special parallel processor without the graphics optimization, such parallel processors performing the mathematically intensive functions described herein. One or more of processors 1802 may include a processing accelerator (e.g., DSP or other special-purpose processor).

In order to manipulate data in accordance with embodiments described herein, processors 1802 access a memory 1804 via system bus 1808. Memory 1804 is nontransitory memory, such as random access memory (RAM). Memory 1804 may include one or more levels of cache. Memory 1804 has stored therein control logic (i.e., computer software) and/or data. For data that needs to be stored more permanently, processors 1802 access persistent storage 1806 via system bus 1808. Persistent storage 1806 may include, for example, a hard disk drive and/or a removable storage device or drive. A removable storage drive may be an optical storage device, a compact disc drive, flash memory, a floppy disk drive, a magnetic tape drive, tape backup device, and/or any other storage device/drive.

Processors 1802, memory 1804, and persistent storage 1806 cooperate with operating system 1820 to provide basic functionality for computing device 1800. Operating system 1820 provides support functionality for applications layer 1830.

Network connection 1810 enables computer device 1800 to communicate and interact with any combination of remote devices, remote networks, remote entities, etc. For example, network connection 1810 may allow computer device 1800 to communicate with remote devices over network 1802, which may be a wired and/or wireless network, and which may include any combination of LANs, WANs, the Internet, etc. Control logic and/or data may be transmitted to and from computer device 1800 via network connection 1810.

Applications layer 1830 may house various modules and components. For example, the applications and modules described above with respect to FIG. 17 may be included in applications layer 1830.

It should be noted that computer-readable medium embodiments may include any physical medium which is capable of encoding instructions that may subsequently be used by a processor to implement methods described herein. Example physical media may include floppy discs, optical discs (e.g. CDs, mini-CDs, DVDs, HD-DVD, Blu-ray), hard drives, punch cards, tape drives, flash memory, or memory chips. However, any other type of tangible, persistent storage that can serve in the role of providing instructions to a processor may be used to store the instructions in these embodiments.

Computing device 1800 may be coupled to a computer-readable storage media reader, either directly or via network 1802. The computer-readable storage media reader can be further coupled to computer-readable storage media, the combination comprehensively representing remote, local, fixed and/or removable storage devices plus storage media, memory, etc. for temporarily and/or more permanently containing computer-readable information, which can include storage device, memory and/or any other such accessible system resource.

Returning to FIG. 17, codon optimizer 1702 includes a ramp optimization engine 1714, a recharging optimization engine 1716, a uridine content optimization engine 1718, and a codon frequency optimization engine 1720. Each of engines 1714, 1716, 1718, and 1720 is implemented on one or more processors, such as processor(s) 1802 in FIG. 18. In an embodiment, each engine is implemented on its own processor. In another embodiment, multiple engines are implemented on one or more shared processors. Codon optimizer 1702 can also be implemented in a distributed computing environment where tasks are performed by remote processing devices that are linked through the communications network. Ramp optimization engine 1714 executes at least a portion of a multiparametric nucleic acid optimization method comprising the use of expression ramps, as described above. Uridine content optimization engine 1718 executes at least a uridine content optimization component of a multiparametric nucleic acid optimization method, as described above. Codon frequency optimization engine 1720 executes at least a portion of a multiparametric nucleic acid optimization method using modifications in the frequency of use of one or more codons relative to other synonymous codons in the optimized nucleic acid sequence as described above. Other optimization engines to execute other portions of a multiparametric nucleic acid optimization method may also be included as appropriate.

Input device 1704 provides input data to codon optimizer 1702. Input device 1704 can be any suitable interface between a user and codon optimizer 1702 as implemented in a computer system, for input and output of data and other information, and for operable interaction with the one or more processing units, such as processor(s) 1802 in FIG. 18. In one aspect, data to be input into the tool can be derived from one source. In one aspect, data to be input into the tool can be derived from more than one source. In some aspects, input device 1704 can alternatively or additionally provide direct input from measuring equipment. Data may be input numerically, as a mathematical expression, as a graph, or in other constructs as known to one skilled in the art. In some aspects, data can be automatically or manually entered from a nucleic acid sequence library. A device for providing input data may include, for example, a detector for detecting characteristics of the data element, e.g., such as a fluorescent plate reader, mass spectrometer, gene chip reader, etc.

Optimization system 1700 also includes a database management system 1722, though one of skill in the art will recognize that such a database management system is optional. User requests or queries can be formatted in an appropriate language understood by the database management system that processes the query to extract the relevant information from various databases, such as sequence library 1706, parameters database 1710, and rules database 1712.

Codon optimizer 1702 may be connected directly to the components shown, may be connected to those components via a communications network, or may be connected through intervening devices.

All or part of system 1700 may be implemented on a server accessible to a user through a client interface, where the server includes the hardware necessary for running computer program products (e.g., software) to access database data for processing user requests.

The output of codon optimizer 1702-the optimized sequence construct (e.g., an mRNA sequence)-may be stored in optimized sequence library 1708. One or more optimized sequences from optimized sequence library 1708 may be sent to an mRNA synthesizer 1724 to be chemically synthesized.

Some aspects described herein can be implemented so as to include a computer program product. A computer program product may include a computer readable medium having computer readable program code embodied in the medium for causing an application program to execute on a computer with a database. As used herein, a "computer program product" refers to an organized set of instructions in the form of natural or programming language statements that are contained on a physical media of any nature (e.g., written, electronic, magnetic, optical or otherwise) and that may be used with a computer or other automated data processing system. Such programming language statements, when executed by a computer or data processing system, cause the computer or data processing system to act in accordance with the particular content of the statements. When the programming language statements are implemented in software, the software can be stored in any computer readable memory such as in RAM, ROM, flash memory, a magnetic disk, a laser disk, or other storage medium, as is also known. Likewise, this software can be delivered to a user or computer device via any known delivery method including, for example, over a communication channel such as a telephone line, the Internet, a wireless connection, etc., or via a transportable medium, such as a computer readable disk, flash drive, etc.

Computer program products include without limitation: programs in source and object code and/or test or data libraries embedded in a computer readable medium. Furthermore, the computer program product that enables a computer system or data processing equipment device to act in pre-selected ways may be provided in a number of forms, including, but not limited to, original source code, assembly code, object code, machine language, encrypted or compressed versions of the foregoing and any and all equivalents.

In one aspect, a computer program product is provided to implement the multiparametric nucleic acid optimization methods disclosed herein, for example, to optimize the sequence of a certain gene via codon optimization to yield a nucleic acid sequence which in turn can be synthesized and expressed, wherein the expression levels of the optimized nucleic acid sequence are higher than the expression levels of the corresponding nucleic acid sequence prior to codon optimization.

It is also envisioned that some instructions may be transmitted as computer signals embodied in a carrier wave, as well as signals (e.g., electrical and optical) propagated through a transmission medium. Thus, the various types of information discussed above could be formatted in a structure, such as a data structure, and transmitted as an electrical signal through a transmission medium or stored on a computer readable medium.

The steps of the disclosed methods and systems are operational with numerous general or special purpose computer system environments or configurations. Examples of well-known computing systems, environments, and/or configuration that can be suitable for use with methods or systems disclosed herein include, but are not limited to, personal computers, server computers, hand-held or laptop devices, multiprocessor systems, microprocessor-based systems, set top boxes, programmable consumer electronics, network PCs, minicomputers, mainframe computers, distributed computing environments that include any of the above systems or devices, and the like.

In some aspects, the instructions for execution in the computer-readable medium are executed iteratively.

Any methods of the present disclosure and all their variants can be implemented in such computer-readable media and in such computer systems.

### VI. Characterization of Optimized Nucleic Acids

In some aspects of the present disclosure, the nucleic acids (e.g., mRNAs) optimized according to the multiparametric methods disclosed herein can be tested to determine whether at least one polynucleotide sequence property (e.g., stability when exposed to nucleases) or expression property has been improved with respect to the non-optimized nucleic acid sequence. The term "expression property" refers to a property of a polynucleotide *in vivo* (e.g., translation efficacy of a synthetic mRNA after administration to a subject in need thereof) or *in vitro* (e.g., translation efficacy of a synthetic mRNA tested in an *in vitro* model system). Expression properties include but are not limited to the amount of protein produced by a therapeutic mRNA after administration and the amount of soluble or otherwise functional protein produced. In some aspects, optimized nucleic acids designed according to the methods disclosed herein can be evaluated according to the viability of the cells expressing a protein encoded by an mRNA designed according to the disclosed methods.

In a particular aspect, a plurality of optimized nucleic acids (e.g., mRNAs) containing designed codon substitutions with respect to the non-optimized nucleic acid sequence is characterized functionally to measure a property of interest, for example an expression property in an *in vitro* model system, or *in vivo* in a target tissue or cell. Examples of expression properties include but are not limited to, expression of a polypeptide, expression of a polypeptide in soluble form, or expression of a polypeptide in biologically or chemically active form.

### a. Optimization of Nucleic Acid Intrinsic Properties

In some aspects of the present disclosure, the desired property to be optimized is an intrinsic property of the nucleic acid sequence (e.g., an mRNA) optimized according to the methods disclosed herein. For example, the nucleic acid sequence (e.g., an mRNA) can be optimized *in vivo* or *in vitro* for stability. In some aspects, the nucleic acid sequence can be optimized for expression in a particular target tissue or cell. In some aspects, the nucleic acid sequence is optimized to increase its plasma half by preventing its degradation by endo and exonucleases.

In other aspects, the nucleic acid sequence is optimized to increase its resistance to hydrolysis in solution, for example, to lengthen the time that the optimized nucleic acid (e.g., an mRNA) or a pharmaceutical composition comprising the optimized nucleic acid can be stored under aqueous conditions with minimal degradation. In other aspects, the nucleic acid sequence (e.g., an mRNA) can optimized to increase its resistance to hydrolysis in dry storage conditions, for example, to lengthen the time that the optimized nucleic acid can be stored after lyophilization with minimal degradation.

### b. Optimization of Protein Expression Properties

In some aspects of the present disclosure, the desired property to be optimized is the level of expression of a protein encoded by a nucleic acid sequence (e.g., an mRNA) optimized according to the methods disclosed herein. Protein expression levels can be measured using one or more expression systems. In some aspects, expression can be measured in cell culture systems, e.g., HeLa cells. In some aspects, expression can be measured using in vitro expression systems prepared from extracts of living cells, e.g., rabbit reticulocyte lysates, or in vitro expression systems prepared by assembly of purified individual components.

In some aspects, protein expression in solution form can be desirable, whereas in other cases protein expression in inclusion body form is desirable. Accordingly, in some aspects the multiparametric nucleic acid optimization methods disclosed herein can be used to optimize the levels of expressed proteins in soluble form. In other aspects, the multiparametric nucleic acid optimization methods disclosed herein can be used to optimize the levels of expressed proteins in inclusion body form.

Levels of protein expression and other properties such as levels of aggregation and the presence of truncation products (i.e., fragments due to proteolysis, hydrolysis, or defective translation) can be measured according to methods known in the art, for example, using electrophoresis (e.g., native or SDS-PAGE) or chromatographic methods (e.g., HPLC, size exclusion chromatography, etc.).

### c. Optimization of Target Tissue/Target Cell Viability

In some cases, the expression of heterologous proteins encoded by a therapeutic nucleic acid protein (e.g., an mRNA) can have deleterious effects in the target tissue or cell, reducing protein yield, or reducing the quality of the expressed product (e.g., due to the presence of protein fragments or precipitation of the expressed protein in inclusion bodies), or causing toxicity. Heterologous protein expression can also be deleterious to cells transfected with a nucleic acid for autologous or heterologous transplantation. Accordingly, in some aspects of the present disclosure the multiparametric nucleic acid optimization methods disclosed herein can be used to increase the viability of target cells expressing the protein encoded by the optimized nucleic acid. Changes in cell or tissue viability, toxicity, and other physiological reaction such as cytokine release can be measured according to methods known in the art.

### VII. Polynucleotides, vectors, pharmaceutical compositions

In certain aspects, the present disclosure encompasses polynucleotides optimized according to the multiparametric nucleic acid optimization methods disclosed herein. For example, the present disclosure provides a polynucleotide or set of polynucleotides comprising at least one nucleic acid sequence (e.g., an mRNA) optimized according to the multiparametric nucleic acid optimization methods disclosed herein that encodes a protein of interest (e.g., a therapeutic protein).

The polynucleotides of the present disclosure can be in the form of RNA or in the form of DNA. DNA includes cDNA, genomic DNA, and synthetic DNA; and can be doublestranded or single-stranded, and if single stranded can be the coding strand or non-coding (anti-sense) strand. In particular aspects, the polynucleotide is an mRNA. In some aspects, the mRNA is a synthetic mRNA. In some aspects, the synthetic mRNA comprises at least one non-natural nucleobase.

In certain aspects, the polynucleotides are isolated. In certain aspects, the polynucleotides are substantially pure. In certain aspects the polynucleotides comprise the coding sequence for the mature polypeptide fused in the same reading frame to a polynucleotide which aids, for example, in expression and secretion of a polypeptide from a host cell (e.g., a leader sequence which functions as a secretory sequence for controlling transport of a polypeptide from the cell). The polypeptide having a leader sequence is a preprotein and can have the leader sequence cleaved by the host cell to form the mature form of the polypeptide. The polynucleotides can also encode for a proprotein which is the mature protein plus additional 5' amino acid residues.

In some aspects, the nucleic acid sequence (e.g., an mRNA) optimized according to the multiparametric nucleic acid optimization methods disclosed herein encodes a variant of a protein of interest, for example, a fragment, analog, or derivatives of the protein of interest (e.g., a therapeutic protein).

The polynucleotide variants can contain alterations in the coding regions, non-coding regions, or both. In some aspects the polynucleotide variants contain alterations which produce silent substitutions, additions, or deletions, but do not alter the properties or activities of the encoded polypeptide. Polynucleotide variants can be produced for a variety of reasons, e.g., to optimize codon expression for a particular target tissue in a patient (change codons in the human mRNA to those preferred in a certain tissue or which will result in a translation profile particularly advantageous for the expression of the protein in the target tissue, for example, a translation rate that will result in a certain concentration of protein encoded by the mRNA in the target tissue). Vectors and cells comprising polynucleotides optimized according to the multiparametric nucleic acid optimization method described herein are also provided.

In some aspects a nucleic acids sequence (e.g., an mRNA) optimized according to the multiparametric nucleic acid optimization methods disclosed herein, and encoding a protein of interest, e.g., a therapeutic protein, can be constructed by chemical synthesis using an oligonucleotide synthesizer. Such oligonucleotides can be designed based on the amino acid sequence of the desired polypeptide and selecting those codons that are favored in the host cell or tissue in which the polypeptide of interest will be produced. Standard methods can be applied to synthesize an isolated polynucleotide sequence encoding an isolated polypeptide of interest. For example, a complete amino acid sequence can be used to construct a backtranslated gene. Further, a DNA oligomer containing a nucleotide sequence coding for the particular isolated polypeptide can be synthesized. For example, several small oligonucleotides coding for portions of the desired polypeptide can be synthesized and then ligated. The individual oligonucleotides typically contain 5' or 3' overhangs for complementary assembly.

Once assembled (by synthesis, site-directed mutagenesis or another method), the polynucleotide sequences (e.g., DNAs) encoding a particular isolated polypeptide of interest can be inserted into an expression vector and operatively linked to an expression control sequence appropriate for expression of the protein in a desired host. Proper assembly can be confirmed by nucleotide sequencing, restriction mapping, and expression of a biologically active polypeptide in a suitable host. As is well known in the art, in order to obtain high expression levels of a transfected gene in a target tissue or target cell, the gene must be operatively linked to transcriptional and translational expression control sequences that are functional in the chosen expression host.

In certain aspects, expression vectors are used to amplify and express nucleic acid sequences optimized according to the multiparametric nucleic acid optimization methods disclosed herein encoding a protein of interest. Expression vectors are replicable DNA constructs which have synthetic or cDNA-derived nucleic acids sequences optimized according to the multiparametric nucleic acid optimization methods disclosed herein encoding a protein of interest, operatively linked to suitable transcriptional or translational regulatory elements derived from mammalian, microbial, viral or insect genes.

A transcriptional unit generally comprises an assembly of (1) a genetic element or elements having a regulatory role in gene expression, for example, transcriptional promoters or enhancers, (2) a structural or coding sequence which is transcribed into mRNA and translated into protein, and (3) appropriate transcription and translation initiation and termination sequences, as described in detail below. Such regulatory elements can include an operator sequence to control transcription. The ability to replicate in a host, usually conferred by an origin of replication, and a selection gene to facilitate recognition of transformants can additionally be incorporated.

DNA regions are operatively linked when they are functionally related to each other. For example, DNA for a signal peptide (secretory leader) is operatively linked to DNA for a polypeptide if it is expressed as a precursor which participates in the secretion of the polypeptide; a promoter is operatively linked to a coding sequence if it controls the transcription of the sequence; or a ribosome binding site is operatively linked to a coding sequence if it is positioned so as to permit translation. Structural elements intended for use in yeast expression systems include a leader sequence enabling extracellular secretion of translated protein by a host cell. Alternatively, where recombinant protein is expressed without a leader or transport sequence, it can include an N-terminal methionine residue. This residue can optionally be subsequently cleaved from the expressed recombinant protein to provide a final product.

Various mammalian or insect cell culture systems can also be advantageously employed to express proteins encoded by nucleic acids sequences (e.g., mRNAs) optimized according to the multiparametric nucleic acid optimization methods disclosed herein. Expression of the recombinant proteins in mammalian cell model can be used to determine the level of functionality of the optimized nucleic acid, e.g., it translational efficacy, and therefore to evaluate whether the optimized nucleic is suitable for in vivo administration to a target tissue or cell in a subject in need thereof.

Examples of suitable mammalian model cell lines include HEK-293 and HEK-293T, the COS-7 lines of monkey kidney cells, described by Gluzman (Cell 23:175, 1981), and other cell lines including, for example, L cells, C127, 3T3, Chinese hamster ovary (CHO), NSO, HeLa and BHK cell lines. Mammalian expression vectors can comprise nontranscribed elements such as an origin of replication, a suitable promoter and enhancer linked to the gene to be expressed, and other 5' or 3' flanking nontranscribed sequences, and 5' or 3' nontranslated sequences, such as necessary ribosome binding sites, a polyadenylation site, splice donor and acceptor sites, and transcriptional termination sequences. Baculovirus systems for production of heterologous proteins in insect cells are reviewed by Luckow and Summers, BioTechnology 6:47 (1988).

The present disclosure also provides a pharmaceutical composition comprising an optimized nucleic acid (e.g., an mRNA) prepared according to the multiparametric nucleic acid optimization methods disclosed herein, or a vector or set of vectors comprising an optimized nucleic acid prepared according to the multiparametric nucleic acid optimization methods disclosed herein, and a pharmaceutically acceptable vehicle or excipient.

### VIII. Embodiments

E1. A multiparametric method for optimizing a candidate nucleic acid sequence, the method comprising at least one optimization method selected from: (i) modifying at least one subsequence in the candidate nucleic acid sequence to generate a ramp subsequence; (ii) substituting at least one codon in the candidate nucleic acid sequence with an alternative codon to increase or decrease uridine content to generate a uridine-modified sequence; (iii) substituting at least one codon in the candidate nucleic acid sequence or the uridine-modified sequence with a fast recharging codon; (iv) substituting at least one codon in the candidate nucleic acid sequence with an alternative codon having a higher codon frequency in the synonymous codon set; (v) substituting at least one natural nucleobase in the candidate nucleic acid sequence with an alternative synthetic nucleobase; (vi) substituting at least one internucleoside linkage in the candidate nucleic acid sequence with a non-natural internucleoside linkage; and, (vii) combinations thereof, wherein the resulting optimized nucleic acid sequence has at least one optimized property with respect to the candidate nucleic acid sequence.

E2. The multiparametric method according to embodiment E1, wherein the optimized nucleic acid sequence comprises at least one ramp subsequence.

E3. The multiparametric method according to embodiment E2, wherein a ramp subsequence comprises at least 1, 2, 3, 4, 5, 6, 7, 8, 9, 10, 11, 12, 13, 14, 15, 16, 17, 18, 19 or 20 consecutive codons.

E4. The multiparametric method according to embodiment E2, wherein the ramp subsequence is located at least 1, 2, 3, 4, 5, 6, 7, 8, 9, 10, 11, 12, 13, 14, 15, 16, 17, 18, 19 or 20 codons from the 5' end of the optimized nucleic acid sequence.

E5. The multiparametric method according to embodiment E2, wherein the ramp subsequence is a speed-up ramp subsequence.

E6. The multiparametric method according to embodiment E2, wherein the ramp subsequence is a speed-down ramp subsequence.

E7. The multiparametric method according to embodiment E2, wherein the optimized nucleic acid sequences comprises at least two ramp subsequences.

E8. The multiparametric method according to embodiment E7, wherein both ramp subsequences are speed-up ramp subsequences.

E9. The multiparametric method according to embodiment E7, wherein both ramp subsequences are speed-down ramp subsequences.

E10. The multiparametric method according to embodiment E7, wherein a ramp subsequence is a speed-up ramp subsequence and a ramp subsequence is a speed-down ramp subsequence.

E11. The multiparametric method according to embodiment E7, wherein two ramp subsequences are at least 10, 20, 30, 40, 50, 60, 70, 80, 90 or 100 codons apart in the optimized nucleic acid sequence.

E12. The multiparametric method according to embodiment E8, wherein the translation speed of the speed-up ramp subsequence is at least 10% higher than the translation speed of the corresponding subsequence in the candidate nucleic acid sequence.

E13. The multiparametric method according to embodiment E9, wherein the translation speed of the speed-down ramp subsequence is at least 10% lower than the translation speed of the corresponding subsequence in the candidate nucleic acid sequence.

E14. The multiparametric method according to embodiment E1, wherein the ramp subsequence is a homologous ramp subsequence.

E15. The multiparametric method according to embodiment E1, wherein the ramp subsequence is a heterologous ramp subsequence.

E16. The multiparametric method according to embodiment E1, wherein the ramp subsequence has a GC content (absolute or relative) at least about 5%, about 10%, about 15%, about 20%, about 25%, about 30%, about 35%, about 40%, about 45%, about 50%, about 55%, about 60%, about 65%, about 70%, about 75%, about 80%, about 85%, about 90%, about 95%, or about 100% higher or lower than the GC content (absolute of relative) of the corresponding subsequence in the candidate nucleic acid sequence.

E17. The multiparametric method according to embodiment E1, wherein the ramp subsequence has a uridine (U) content (absolute or relative) at least about 5%, about 10%, about 15%, about 20%, about 25%, about 30%, about 35%, about 40%, about 45%, about 50%, about 55%, about 60%, about 65%, about 70%, about 75%, about 80%, about 85%, about 90%, about 95%, or about 100% higher or lower than the uridine (U) content (absolute or relative) of the corresponding subsequence in the candidate nucleic acid sequence.

E18. The multiparametric method according to embodiment E1, wherein the protein sequence encoded by the ramp subsequence has an alpha-helical, beta-sheet, or random coil secondary structure.

E19. The multiparametric method according to embodiment E1, wherein the protein sequence encoded by the ramp subsequence comprises an amino acid sequence with alpha-helix and beta strand secondary structure; alpha-helix and random coil secondary structure;
beta strand and random coil secondary structure; or, alpha-helix, beta strand, and random coil secondary structure.

E20. The multiparametric method according to embodiment E1, wherein the codons in the optimized nucleic acid sequences are selected from an optimized codon set.

E21. The multiparametric method according to embodiment E20, wherein the optimized codon set is a limited codon set.

E22. The multiparametric method according to embodiment E21, wherein the limited codon set comprises 61, 60, 59, 58, 57, 56, 55, 54, 53, 52, 51, 50, 49, 48, 47, 46, 45, 44, 43, 42, 41, 40, 39, 38, 37, 36, 35, 34, 33, 32, 31, 30, 30, 29, 28, 27, 26, 25, 24, 23, 22, 21, or 20 codons.

E23. The multiparametric method according to embodiment E21, wherein at least one amino acid selected from the group consisting of Ala, Arg, Asn, Asp, Cys, Gln, Glu, Gly, His, Ile, Leu, Lys, Phe, Pro, Ser, Thr, Tyr, and Val is encoded by a single codon in the limited codon set.

E24. The multiparametric method according to embodiment E21, wherein the limited codon set consists of 20 codons, and wherein each codon encodes one of 20 amino acids.

E25. The multiparametric method according to embodiment E21, wherein the limited codon set comprises at least one codon selected from the group consisting of GCT, GCC, GCA, and GCG; at least a codon selected from the group consisting of CGT, CGC, CGA, CGG, AGA, and AGG; at least a codon selected from AAT or ACC; at least a codon selected from GAT or GAC; at least a codon selected from TGT or TGC; at least a codon selected from CAA or CAG; at least a codon selected from GAA or GAG; at least a codon selected from the group consisting of GGT, GGC, GGA, and GGG; at least a codon selected from CAT or CAC; at least a codon selected from the group consisting of ATT, ATC, and ATA; at least a codon selected from the group consisting of TTA, TTG, CTT, CTC, CTA, and CTG; at least a codon selected from AAA or AAG; an ATG codon; at least a codon selected from TTT or TTC; at least a codon selected from the group consisting of CCT, CCC, CCA, and CCG; at least a codon selected from the group consisting of TCT, TCC, TCA, TCG, AGT, and AGC; at least a codon selected from the group consisting of ACT, ACC, ACA, and ACG; a TGG codon; at least a codon selected from TAT or TAC; and, at least a codon selected from the group consisting of GTT, GTC, GTA, and GTG.

E26. The multiparametric method according to embodiment E21, wherein the limited codon set comprises at least one codon selected from the group consisting of GCU, GCC, GCA, and GCG; at least a codon selected from the group consisting of CGU, CGC, CGA, CGG, AGA, and AGG; at least a codon selected from AAU or ACC; at least a codon selected from GAU or GAC; at least a codon selected from UGU or UGC; at least a codon selected from CAA or CAG; at least a codon selected from GAA or GAG; at least a codon selected from the group consisting of GGU, GGC, GGA, and GGG; at least a codon selected from CAU or CAC; at least a codon selected from the group consisting of AUU, AUC, and AUA; at least a codon selected from the group consisting of UUA, UUG, CUU, CUC, CUA, and CUG; at least a codon selected from AAA or AAG; an AUG codon; at least a codon selected from UUU or UUC; at least a codon selected from the group consisting of CCU, CCC, CCA, and CCG; at least a codon selected from the group consisting of UCU, UCC, UCA, UCG, AGU, and AGC; at least a codon selected from the group consisting of ACU, ACC, ACA, and ACG; a UGG codon; at least a codon selected from UAU or UAC; and, at least a codon selected from the group consisting of GUU, GUC, GUA, and GUG.

E27. The multiparametric method according to embodiment E25, wherein the limited codon set is: (a) TTC, TTG, CTG, ATC, ATG, GTG, AGC, CCC, ACC, GCC, TAC, CAC, CAG, AAC, AAG, GAG, TGC, TGG, AGG, GGC; (b) TTT, CTA, ATA, ATG, GTA, TCG, CCG, ACG, GCG, TAT, CAT, CAA, AAT, AAA, GAT, GAA, TGT, TGG, CGT, GGT; (c) TTC, CTV, ATM, ATG, GTV, AGC, CCV, ACV, GCV, TAC, CAC, CAR, AAC, AAR, GAC, GAR, TGC, TGG, CGV, GGV; or, (d) TTC, CTV, ATM, ATG, GTV, AGC, CCV, ACV, GCV, TAC, CAC, CAR, AAC, AAR, GAC, GAR, TGC, TGG, AGR, GGV.

E28. The multiparametric method according to embodiment E26, wherein the limited codon set is: (a) UUC, UUG, CUG, AUC, AUG, GUG, AGC, CCC, ACC, GCC, UAC, CAC, CAG, AAC, AAG, GAG, UGC, UGG, AGG, GGC; (b) UUU, CUA, AUA, AUG, GUA, UCG, CCG, ACG, GCG, UAU, CAU, CAA, AAU, AAA, GAU, GAA, UGU, UGG, CGU, GGU; (c) UUC, CUV, AUM, AUG, GUV, AGC, CCV, ACV, GCV, UAC, CAC, CAR, AAC, AAR, GAC, GAR, UGC, UGG, CGV, GGV; or, (d) UUC, CUV, AUM, AUG, GUV, AGC, CCV, ACV, GCV, UAC, CAC, CAR, AAC, AAR, GAC, GAR, UGC, UGG, AGR, GGV.

E28. The multiparametric method according to embodiment E20, wherein the optimized codon set comprises at least one codon encoding an unnatural amino acid.

E29. The multiparametric method according to embodiment E20, wherein the optimized codon set comprises at least one codon consisting of more than 3 nucleobases.

E30. The multiparametric method according to embodiment E29, wherein the at least one codon consisting of more than 3 nucleobases consists of 4 or 5 nucleobases.

E31. The multiparametric method according to embodiment E20, wherein the optimized codon set comprises at least one codon comprising an unnatural nucleobase.

E32. The multiparametric method according to embodiment E1, wherein the uridine-modified sequence induces a lower Toll-Like Receptor (TLR) response when compared to the candidate nucleic acid sequence.

E33. The multiparametric method according to embodiment E32, wherein the lower TLR response is mediated by TLR3, TLR7, TLR8, or TLR9.

E34. The multiparametric method according to embodiment E33, wherein the lower TLR response is at least 10%, at least 20%, at least 30%, at least 40%, at least 50%, at least 60%, at least 70%, at least 80%, at least 90 or at least 100% lower than the TLR response caused by the candidate nucleic acid sequence.

E35. The multiparametric method according to embodiment E1, wherein the uridine content (absolute or relative content) of the uridine-modified sequence is higher than the uridine content of the candidate nucleic acid sequence.

E36. The multiparametric method according to embodiment E1, wherein the uridine content (absolute or relative content) of the uridine-modified sequence is lower than the uridine content of the candidate nucleic acid sequence.

E37. The multiparametric method according to embodiment E35, wherein the uridine-modified sequence contains at least 5%, 10%, 15%, 20%, 25%, 30%, 35%, 40%, 45% or 50% more uridine (absolute or relative) that the candidate nucleic acid sequence.

E38. The multiparametric method according to embodiment E36, wherein the uridine-modified sequence contains at least 5%, 10%, 15%, 20%, 25%, 30%, 35%, 40%, 45% or 50% less uridine (absolute or relative content) than the candidate nucleic acid sequence.

E39. The multiparametric method according to embodiment E1, wherein the uridine content (absolute or relative content) of the uridine-modified sequence is less than 50%, 49%, 48%, 47%, 46%, 45%, 44%, 43%, 42%, 41%, 40%, 39%, 38%, 37%, 36%, 35%, 34%, 33%, 32%, 31%, 30%, 29%, 28%, 27%, 26%, 25%, 24%, 23%, 22%, 21%, 20%, 19%, 18%, 17%, 16%, 15%, 14%, 13%, 12%, 11%, 10%, 9%, 8%, 7%, 6%, 5%, 4%, 3%, 2% or 1%.

E40. The multiparametric method according to embodiment E1, wherein the candidate nucleic acid sequence comprises at least one uridine cluster, wherein said uridine cluster is a subsequence of the candidate nucleic acid sequence wherein the percentage of total uridine nucleobases in said subsequence is above or below a predetermined threshold.

E41. The multiparametric method according embodiment E40, wherein the length of the subsequence is 10, 15, 20, 25, 30, 35, 40, 45, 50, 55, 60, 65, 70, 75, 80, 85, 90, 95, or 100 nucleobases.

E42. The multiparametric method according to embodiment E1, wherein the candidate nucleic acid sequence comprises at least one uridine cluster, wherein said uridine cluster is a subsequence of the candidate nucleic acid sequence wherein the percentage of uridine nucleobases in said subsequence as measured using a sliding window is above a predetermined threshold.

E43. The multiparametric method according to embodiment E42, wherein the length of the sliding window is 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, or 40 nucleobases.

E44. The multiparametric method according to any one of embodiments E40 to E43, wherein the threshold is 1%, 2%, 3%, 4%, 5%, 6%, 7%, 8%, 9%, 10%, 11%, 12%, 13%, 14%, 15%, 16%, 17%, 18%, 19%, 20%, 21%, 22%, 23%, 24% or 25% uridine content.

E45. The multiparametric method according to any one of embodiments E40 to E43, wherein the candidate nucleic acid sequence comprises at least two uridine clusters.

E46. The multiparametric method according to any one of embodiments E40 to E45, wherein the uridine-modified sequence contains less uridine-rich clusters than the candidate nucleic acid sequence.

E47. The multiparametric method according to any one of embodiments E40 to E45, wherein the uridine-modified sequence contains more uridine-rich clusters than the candidate nucleic acid sequence.

E48. The multiparametric method according to any one of embodiments E40 to E47, wherein the uridine-modified sequence contains uridine-rich clusters with are shorter in length than the corresponding uridine-rich clusters in the candidate nucleic acid sequence.

E49. The multiparametric method according to any one of embodiments E40 to E47, wherein the uridine-modified sequence contains uridine-rich clusters which are longer in length than the corresponding uridine-rich cluster in the candidate nucleic acid sequence.

E50. The multiparametric method according to embodiment E1, wherein the optimized nucleic acid sequence comprises an overall increase in Guanine/Cytosine (G/C) content (absolute or relative) relative to the G/C content (absolute or relative) of the candidate nucleic acid sequence.

E51. The multiparametric method according to embodiment E50, wherein the overall increase in G/C content (absolute or relative) is by at least about 5%, about 10%, about 15%, about 20%, about 25%, about 30%, about 35%, about 40%, about 45%, about 50%, about 55%, about 60%, about 65%, about 70% or about 75% relative to the G/C content (absolute or relative) of the candidate nucleic acid sequence.

E52. The multiparametric method according to embodiment E1, wherein the optimized nucleic acid sequence comprises an overall decrease in Guanine/Cytosine (G/C) content (absolute or relative) relative to the G/C content (absolute or relative) of the candidate nucleic acid sequence.

E53. The multiparametric method according to embodiment E50, wherein the overall decrease in G/C content (absolute or relative) is by at least 5%, 10%, 15%, 20%, 25%, 30%, 35%, 40%, 45% or 50% relative to the G/C content (absolute or relative) of the candidate nucleic acid sequence.

E54. The multiparametric method according to embodiment E1, wherein the optimized nucleic acid sequence comprises a local increase in Guanine/Cytosine (G/C) content (absolute or relative) in a subsequence (G/C modified subsequence) relative to the G/C content (absolute or relative) of the corresponding subsequence in the candidate nucleic acid sequence.

E55. The multiparametric method according to embodiment E50, wherein the local increase in G/C content (absolute or relative) is by at least 5%, 10%, 15%, 20%, 25%, 30%, 35%, 40%, 45% or 50%

E56. The multiparametric method according to embodiment E1, wherein the optimized nucleic acid sequence comprises a local decrease in Guanine/Cytosine (G/C) content (absolute or relative) in a subsequence relative to the G/C content (absolute or relative) of the corresponding subsequence of the candidate nucleic acid sequence.

E57. The multiparametric method according to embodiment E50, wherein the local decrease in G/C content (absolute or relative) is by at least 5%, 10%, 15%, 20%, 25%, 30%, 35%, 40%, 45% or 50%.

E58. The multiparametric method according to any one of embodiments E54 to E57, wherein the length of the subsequence is at least 5, 10, 15, 20, 25, 30, 35, 40, 45, 50, 55, 60, 65, 70, 75, 80, 85, 90, 95, or 100 nucleobases.

E59. The multiparametric method according to any one of embodiments E54 to E58, wherein the subsequence is located within: (a) at least 5, 10, 15, 20, 25, 30, 35, 40, 45, 50, 55, 60, 65, 70, 75, 80, 85, 90, 95 or 100 nucleobases from the 5' end of the candidate nucleic acid sequence; or, (b) a distance from the 5' end of the candidate nucleic acid sequence which is at least about 5%, about 10%, about 15%, about 20%, about 25%, about 30%, about 35%, about 40%, about 45%, about 50%, about 55%, about 60%, about 65%, about 70%, about 75%, about 80%, about 85%, about 90%, or about 95% of the length of the candidate nucleic acid sequence.

E60. The multiparametric method according to any one of embodiments E54 to E58, wherein the subsequence is located within: (a) at least 5, 10, 15, 20, 25, 30, 35, 40, 45, 50, 55, 60, 65, 70, 75, 80, 85, 90, 95 or 100 nucleobases from the 3' end of the candidate nucleic acid sequence; or, (b) a distance from the 3' end of the candidate nucleic acid sequence which is at least about 5%, about 10%, about 15%, about 20%, about 25%, about 30%, about 35%, about 40%, about 45%, about 50%, about 55%, about 60%, about 65%, about 70%, about 75%, about 80%, about 85%, about 90%, or about 95% of the length of the candidate nucleic acid sequence.

E61. The multiparametric method according to any one of embodiments E54 to E58, wherein the optimized nucleic acid sequence comprises more than one G/C content-modified subsequence wherein the G/C content of each G/C content-modified subsequence is increased or decreased with respect to the G/C content in a corresponding subsequence of the candidate nucleic acid sequence.

E62. The multiparametric method according to embodiment E61, wherein the optimized nucleic acid sequence comprises at least 2, 3, 4, 5, 6, 7, 8, 9, 10, 11, 12, 13, 14, 15, 16, 17, 18, 19, 20, 21, 22, 23, 24 or 25 G/C content-modified subsequences.

E63. The multiparametric method according to embodiment E61, wherein the distance between two G/C content-modified subsequences is at least 5, 10, 15, 20, 25, 30, 35, 40, 45, 50, 55, 60, 65, 70, 75, 80, 85, 90, 95 or 100 nucleobases.

E64. The multiparametric method according to any one of embodiments E61 to E63, wherein the G/C content (absolute or relative) of each G/C content-modified subsequence in the optimized nucleic acid sequence is increased with respect to the G/C content (absolute or relative) in a corresponding subsequence of the candidate nucleic acid sequence.

E65. The multiparametric method according to any one of embodiment E61 to E63, wherein the G/C content (absolute or relative) of each G/C content-modified subsequence in the optimized nucleic acid sequence is decreased with respect to the G/C content (absolute or relative) in a corresponding subsequence of the candidate nucleic acid sequence.

E66. The multiparametric method according to embodiment E1, wherein at least about 5%, at least about 10%, at least about 15%, at least about 20%, at least about 25%, at least about 30%, at least about 35%, at least about 40%, at least about 45%, at least about 50%, at least about 55%, at least about 60%, at least about 65%, at least about 70%, at least about 75%, at least about 80%, at least about 85%, at least about 90%, at least about 95%, at least about 99%, or 100% of the codons in the candidate nucleic acid sequence are substituted with alternative codons, each alternative codon having a codon frequency higher than the codon frequency of the substituted codon in the synonymous codon set.

E67. The multiparametric method according to embodiment E1, wherein at least one codon in the candidate nucleic acid sequence is substituted with an alternative codon having a higher codon frequency than the codon frequency of the substituted codon in the synonymous codon set, and at least one codon in the candidate nucleic acid sequence is substituted with an alternative codon having a lower codon frequency than the codon frequency of the substituted codon in the synonymous codon set.

E68. The multiparametric method according to embodiment E67, wherein at least about 5%, at least about 10%, at least about 15%, at least about 20%, at least about 25%, at least about 30%, at least about 35%, at least about 40%, at least about 45%, at least about 50%, at least about 55%, at least about 60%, at least about 65%, at least about 70%, or at least about 75% of the codons in the candidate nucleic acid sequence are substituted with alternative codons, each alternative codon having a codon frequency lower than the codon frequency of the substituted codon in the synonymous codon set.

E69. The multiparametric method according to any one of embodiments E66 to E68, wherein at least one alternative codon having a higher codon frequency has the highest codon frequency in the synonymous codon set.

E70. The multiparametric method according to embodiment E69, wherein all alternative codons having a higher codon frequency have the highest codon frequency in the synonymous codon set.

E71. The multiparametric method according to any one of embodiments E67 or E68, wherein at least one alternative codon having a lower codon frequency has the lowest codon frequency in the synonymous codon set.

E72. The multiparametric method according to embodiment E71, wherein all alternative codons having a lower codon frequency have the lowest codon frequency in the synonymous codon set.

E73. The multiparametric method according to embodiment E1, wherein at least about 5%, at least about 10%, at least about 15%, at least about 20%, at least about 25%, at least about 30%, at least about 35%, at least about 40%, at least about 45%, at least about 50%, at least about 55%, at least about 60%, at least about 65%, at least about 70%, at least about 75%, at least about 80%, at least about 85%, at least about 90%, at least about 95%, at least about 99%, or 100% of the codons in the candidate nucleic acid sequence are substituted with alternative codons having faster recharging rates.

E74. The multiparametric method according to embodiment E1, wherein at least one codon in the candidate nucleic acid sequence is substituted with an alternative codon having a faster recharging rate, and at least one codon in the candidate nucleic acid sequence is substituted with an alternative codon having a slower recharging rate.

E75. The multiparametric method according to embodiment E74, wherein at least about 5%, at least about 10%, at least about 15%, at least about 20%, at least about 25%, at least about 30%, at least about 35%, at least about 40%, at least about 45%, at least about 50%, at least about 55%, at least about 60%, at least about 65%, at least about 70%, or at least about 75% of the codons in the candidate nucleic acid sequence are substituted with alternative codons, each codon having a having a slower recharging rate.

E76. The multiparametric method according to any one of embodiments E73 to E75, wherein at least one alternative codon having a faster recharging rate has the fastest recharging rate.

E77. The multiparametric method according to embodiment E70, wherein all alternative codons having a faster recharging rate have the fastest recharging rate.

E78. The multiparametric method according to any one of embodiments E74 or E75, wherein at least one alternative codon having a slower recharging rate has the slowest recharging rate.

E79. The multiparametric method according to embodiment E71, wherein all alternative codons having a slower recharging rate have the slowest recharging rate.

E80. The multiparametric method according embodiment E1, wherein the method comprises one optimization method selected from the group consisting of (i) modifying at least one subsequence in the candidate nucleic acid sequence to generate a ramp subsequence; (ii) substituting at least one codon in the candidate nucleic acid sequence with an alternative codon to increase or decrease uridine content to generate a uridine-modified sequence; (iii) substituting at least one codon in the candidate nucleic acid sequence or the uridine-modified sequence with a fast recharging codon; (iv) substituting at least one codon in the candidate nucleic acid sequence with an alternative codon having a higher codon frequency in the synonymous codon set; (v) substituting at least one natural nucleobase in the candidate nucleic acid sequence with an alternative synthetic nucleobase; and (vi) substituting at least one internucleoside linkage in the candidate nucleic acid sequence with a non-natural internucleoside linkage.

E81. The multiparametric method according embodiment E1, wherein the method comprises two optimization methods selected from the group consisting of (i) modifying at least one subsequence in the candidate nucleic acid sequence to generate a ramp subsequence; (ii) substituting at least one codon in the candidate nucleic acid sequence with an alternative codon to increase or decrease uridine content to generate a uridine-modified sequence; (iii) substituting at least one codon in the candidate nucleic acid sequence or the uridine-modified sequence with a fast recharging codon; (iv) substituting at least one codon in the candidate nucleic acid sequence with an alternative codon having a higher codon frequency in the synonymous codon set; (v) substituting at least one natural nucleobase in the candidate nucleic acid sequence with an alternative synthetic nucleobase; and (vi) substituting at least one internucleoside linkage in the candidate nucleic acid sequence with a non-natural internucleoside linkage.

E82. The multiparametric method according embodiment E1, wherein the method comprises three optimization methods selected from the group consisting of (i) modifying at least one subsequence in the candidate nucleic acid sequence to generate a ramp subsequence; (ii) substituting at least one codon in the candidate nucleic acid sequence with an alternative codon to increase or decrease uridine content to generate a uridine-modified sequence; (iii) substituting at least one codon in the candidate nucleic acid sequence or the uridine-modified sequence with a fast recharging codon; (iv) substituting at least one codon in the candidate nucleic acid sequence with an alternative codon having a higher codon frequency in the synonymous codon set; (v) substituting at least one natural nucleobase in the candidate nucleic acid sequence with an alternative synthetic nucleobase; and (vi) substituting at least one internucleoside linkage in the candidate nucleic acid sequence with a non-natural internucleoside linkage.

E83. The multiparametric method according embodiment E1, wherein the method comprises four optimization methods selected from the group consisting of (i) modifying at least one subsequence in the candidate nucleic acid sequence to generate a ramp subsequence; (ii) substituting at least one codon in the candidate nucleic acid sequence with an alternative codon to increase or decrease uridine content to generate a uridine-modified sequence; (iii) substituting at least one codon in the candidate nucleic acid sequence or the uridine-modified sequence with a fast recharging codon; (iv) substituting at least one codon in the candidate nucleic acid sequence with an alternative codon having a higher codon frequency in the synonymous codon set; (v) substituting at least one natural nucleobase in the candidate nucleic acid sequence with an alternative synthetic nucleobase; and (vi) substituting at least one internucleoside linkage in the candidate nucleic acid sequence with a non-natural internucleoside linkage.

E84. The multiparametric method according embodiment E1, wherein the method comprises five optimization methods selected from the group consisting of (i) modifying at least one subsequence in the candidate nucleic acid sequence to generate a ramp subsequence; (ii) substituting at least one codon in the candidate nucleic acid sequence with an alternative codon to increase or decrease uridine content to generate a uridine-modified sequence; (iii) substituting at least one codon in the candidate nucleic acid sequence or the uridine-modified sequence with a fast recharging codon; (iv) substituting at least one codon in the candidate nucleic acid sequence with an alternative codon having a higher codon frequency in the synonymous codon set; (v) substituting at least one natural nucleobase in the candidate nucleic acid sequence with an alternative synthetic nucleobase; and (vi) substituting at least one internucleoside linkage in the candidate nucleic acid sequence with a non-natural internucleoside linkage.

E85. The multiparametric method according embodiment E1, wherein the method comprises six optimization methods selected from the group consisting of (i) modifying at least one subsequence in the candidate nucleic acid sequence to generate a ramp subsequence; (ii) substituting at least one codon in the candidate nucleic acid sequence with an alternative codon to increase or decrease uridine content to generate a uridine-modified sequence; (iii) substituting at least one codon in the candidate nucleic acid sequence or the uridine-modified sequence with a fast recharging codon; (iv) substituting at least one codon in the candidate nucleic acid sequence with an alternative codon having a higher codon frequency in the synonymous codon set; (v) substituting at least one natural nucleobase in the candidate nucleic acid sequence with an alternative synthetic nucleobase; and (vi) substituting at least one internucleoside linkage in the candidate nucleic acid sequence with a non-natural internucleoside linkage.

E86. The multiparametric method according to any one of embodiments E1 to E85, wherein the method comprises 7, 8, 9, 10, 11, 12, 13, 14, 15, 16, 17, 18, 19 or 20 optimization methods.

E87. The multiparametric method according to any one of embodiments E1 to E86, wherein the method comprises more than 20 optimization methods.

E88. The multiparametric method according to any one of embodiments E1 to E87, wherein at least 5%, at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 85%, at least 90%, at least 95%, at least 96%, at least 97%, at least 98%, at least 99% or 100% of the codons in the candidate nucleic acid sequence are replaced.

E89. The multiparametric method according to any one of embodiments E1 to E88, wherein the optimization methods are executed sequentially.

E90. The multiparametric method according to any one of embodiments E1 to E88, wherein the optimization methods are executed concurrently.

E91. The multiparametric method according to any one of embodiments E1 to E88, wherein the optimization methods are executed recursively.

E92. A method for expressing a protein in a target tissue or cell or an in vitro translation system, the method comprising: (a) obtaining an optimized gene sequence for expression in a human in vivo systemically or in a target tissue or target cell, using a method according to any one of embodiments E1 to E91; (b) synthesizing a nucleic acid molecule comprising the optimized gene sequence; (c) introducing the nucleic acid molecule into the target tissue or cell or combining it with the in vitro translation system,

E93. The method according to any one of embodiments E1 to E91, wherein the at least one optimized property with respect to the candidate nucleic acid sequence is selected from (i) increase in transcription efficacy; (ii) increase in translation efficacy; (iii) increase in nucleic acid (DNA or RNA) in vivo half-life; (iv) increase in nucleic acid (DNA or RNA) in vitro half-life; (v) decrease in nucleic acid (DNA or RNA) in vivo half-life; (vi) decrease in nucleic acid (DNA or RNA) in vitro half-life; (vii) increase in expressed protein yield; (viii) increase in expressed protein quality; (ix) increase in nucleic acid (DNA or RNA) structural stability; (x) increase in viability of cells expressing the optimized nucleic acid; and, (xi) combinations thereof.

E94. A computer implemented multiparametric codon optimization method comprising: (a) inputting at least one candidate nucleic acid sequence; (b) applying a multiparametric codon optimization method according to any one of embodiments E1 to E91 to the candidate nucleic acid sequence; and, outputting at least one optimized nucleic acid sequence.

E95. The computer implemented method according to embodiment E94, wherein at least one optimized nucleic acid sequence outputted in step (c) is used an inputting sequence in step (a).

E96. The computer implemented method according to embodiment E94, wherein said method is executed recursively for at least 2, 3, 4, 5, 6, 7, 8, 9 or 10 cycles.

E97. The computer implemented method according to embodiment E94, wherein said method is executed recursively for at least 10, at least 20, at least 30, at least 40, at least 50, at least 60, at least 70, at least 80, at least 90, or at least 100 cycles.

E98. The computer implemented method according to embodiment E94, wherein said method is executed recursively for at least 200, at least 300, at least 400, at least 500, at least 600, at least 700, at least 800, at least 900, or at least 1000 cycles.

E99. The computer implemented method according to embodiment E94, wherein said method is executed recursively for at least 2000, at least 3000, at least 4000, at least 5000, at least 6000, at least 7000, at least 8000, at least 9000, or at least 10000 cycles.

E100. The computer implemented method according to embodiment E94, further comprising submitting electronically the optimized nucleic acid sequence to an automated nucleic acid synthesizer.

E101. The computer implemented method according to embodiment E94, wherein a library of candidate nucleic acid sequences is used as input in step (a).

E102. The computer implemented method according to embodiment E94, wherein the output of step (c) is a library of optimized nucleic acid sequences.

E103. The computer implemented method according to embodiment E94, wherein the multiparametric codon optimization method of step (b) is implemented as a swarm algorithm.

E104. The computer implemented method according to embodiment E94, wherein the multiparametric codon optimization method of step (b) is implemented as a multi-swarm algorithm.

E105. The computer implemented method according to embodiment E94, wherein the multiparametric codon optimization method of step (b) is implemented as a Bayesian optimization algorithm.

E106. The computer implemented method according to embodiment E94, wherein the multiparametric codon optimization method of step (b) is implemented as a combinatorial optimization algorithm.

E107. The computer implemented method according to embodiment E94, wherein the multiparametric codon optimization method of step (b) is implemented as a genetic algorithm.

E108. The computer implemented method according to embodiment E107, wherein the genetic algorithm is implemented in parallel.

E109. The computer implemented method according to embodiment E108, wherein the parallel implementation of the genetic algorithms is a coarse-grained parallel genetic algorithm.

E110. The computer implemented method according to embodiment E108, wherein the parallel implementation of the genetic algorithms is a fine-grained parallel genetic algorithm.

E111. The computer implemented method according to embodiment E107, wherein the genetic algorithm comprises adaptive parameters.

E112. An isolated nucleic acid molecule encoding a protein optimized according to the method of any one of embodiments E1 to E91, or a complement thereof.

E113. The isolated nucleic acid molecule according to embodiment E115, wherein said nucleic acid molecule is a DNA.

E114. The isolated nucleic acid molecule according to embodiment E115, wherein said nucleic acid molecule is an RNA.

E115. The isolated nucleic acid molecule according to embodiment E117, wherein the RNA is mRNA.

E116. The isolated nucleic acid molecule according to embodiment E115, wherein the mRNA is synthetic.

E117. The isolated nucleic acid molecule according to embodiment E112, wherein said nucleic acid molecule comprises at least one nucleotide analogue.

E118. The isolated nucleic acid molecule according to embodiment E117, wherein at least one nucleotide analogue is selected from the group consisting of a 2'-O-methoxyethyl-RNA (2'-MOE-RNA) monomer, a 2'-fluoro-DNA monomer, a 2'-O-alkyl-RNA monomer, a 2'-amino-DNA monomer, a locked nucleic acid (LNA) monomer, a cEt monomer, a cMOE monomer, a 5'-Me-LNA monomer, a 2'-(3-hydroxy)propyl-RNA monomer, an arabino nucleic acid (ANA) monomer, a 2'-fluoro-ANA monomer, an anhydrohexitol nucleic acid (HNA) monomer, an intercalating nucleic acid (INA) monomer, and a combination of two or more of said nucleotide analogues.

E119. The isolated nucleic acid molecule according to embodiment E115, wherein said nucleic acid molecule comprises at least one backbone modification.

E120. The isolated nucleic acid molecule according to embodiment E119, wherein at least one backbone modification is a phosphorothioate internucleotide linkage.

E121. The isolated nucleic acid molecule according to embodiment E120, wherein of the internucleotide linkages are phosphorothioate internucleotide linkages.

E122. The isolated nucleic acid molecule according to embodiment E115, wherein said nucleic acid molecule comprises at least one nucleoside selected from the group consisting of 2- pseudouridine, 5-methoxyuridine, 2-thiouridine, 4-thiouridine, N1-methylpseudouridine, 5-aza-uridine, 2-thio-5-aza-uridine, 4-thio-pseudouridine, 2-thio-pseudouridine, 5-hydroxyuridine, 4-methoxy-pseudouridine, 4-methoxy-2-thio-pseudouridine, 3-methyluridine, 5-carboxymethyl-uridine, 1-carboxymethyl-pseudouridine, 5-propynyl-uridine, 1-propynyl-pseudouridine, 2-methoxy-4-thio-uridine, 5-taurinomethyluridine, 1-taurinomethyl-pseudouridine, 5-taurinomethyl-2-thio-uridine, 1-taurinomethyl-4-thio-uridine, 5-methyl-uridine, 2-methoxyuridine, 1-methyl-pseudouridine, 4-thio-1-methyl-pseudouridine, 2-thio-1-methyl-pseudouridine, 1-methyl-1-deaza-pseudouridine, 2-thio-1-methyl-1-deaza-pseudouridine, and 2-thio-dihydrouridine.

E123. The isolated nucleic acid molecule according to embodiment E115, wherein said nucleic acid molecule comprises at least one nucleoside selected from the group consisting of 2-aminopurine, 2,6-diaminopurine, 7-deaza-adenine, 7-deaza-8-aza-adenine, 7-deaza-2-aminopurine, 7-deaza-8-aza-2-aminopurine, 7-deaza-2,6-diaminopurine, 7-deaza-8-aza-2,6-diaminopurine, 1-methyladenosine, N6-methyladenosine, N6-isopentenyladenosine, N6-(cis-hydroxyisopentenyl)adenosine, 2-methylthio-N6-(cis-hydroxyisopentenyl)adenosine, N6-glycinylcarbamoyladenosine, N6-threonylcarbamoyladenosine, 2-methylthio-N6-threonyl carbamoyladenosine, N6,N6-dimethyladenosine, and 7-methyladenine.

E124. The isolated nucleic acid molecule according to embodiment E115, wherein said nucleic acid molecule comprises at least one nucleoside selected from the group consisting of inosine, 1-methyl-inosine, wyosine, wybutosine, 7-deaza-guanosine, 7-deaza-8-aza-guanosine, 6-thio-guanosine, 6-thio-7-deaza-guanosine, 6-thio-7-deaza-8-aza-guanosine, 7-methyl-guanosine, 6-thio-7-methyl-guanosine, 7-methylinosine, 6-methoxy-guanosine, 1-methylguanosine, N2-methylguanosine, N2,N2-dimethylguanosine, 8-oxo-guanosine, 7-methyl-8-oxo-guanosine, and 1-methyl-6-thio-guanosine.

E125. The isolated nucleic acid molecule according to embodiment E115, wherein said nucleic acid molecule comprises at least one nucleoside selected from the group consisting of 5-methylcytidine, 5-aza-cytidine, pseudoisocytidine, 3-methyl-cytidine, N4-acetylcytidine, 5-formylcytidine, N4-methylcytidine, 5-hydroxymethylcytidine, 1-methyl-pseudoisocytidine, pyrrolo-cytidine, pyrrolo-pseudoisocytidine, 2-thio-cytidine, 2-thio-5-methyl-cytidine, 4-thio-pseudoisocytidine, 4-thio-1-methyl-pseudoisocytidine, 4-thio-1-methyl-1-deaza-pseudoisocytidine, 1-methyl-1-deaza-pseudoisocytidine, zebularine, 5-aza-zebularine, 5-methyl-zebularine, 5-aza-2-thio-zebularine, 2-thio-zebularine, 2-methoxy-cytidine, 2-methoxy-5-methyl-cytidine, and 4-methoxy-pseudoisocytidine.

E126. The isolated nucleic acid molecule according to embodiment E115, wherein at least one uridine has been replaced with pseudouridine, 5-methoxyuridine, 2-thiouridine, 4-thiouridine, N1-methylpseudouridine, or 5-aza-uridine.

E127. The isolated nucleic acid molecule according to embodiment E115, wherein at least one uridine has been replaced with 2-thio-5-aza-uridine, 4-thio-pseudouridine, 2-thio-pseudouridine, 5-hydroxyuridine, 4-methoxy-pseudouridine, or 4-methoxy-2-thio-pseudouridine.

E128. The isolated nucleic acid molecule according to embodiment E115, wherein at least one uridine has been replaced with 3-methyluridine, 5-carboxymethyl-uridine, 1-carboxymethyl-pseudouridine, 5-propynyl-uridine, 1-propynyl-pseudouridine, or 2-methoxy-4-thio-uridine.

E129. The isolated nucleic acid molecule according to embodiment E115, wherein at least one uridine has been replaced with 5-taurinomethyluridine, 1-taurinomethyl-pseudouridine, 5-taurinomethyl-2-thio-uridine, 1-taurinomethyl-4-thio-uridine, 5-methyl-uridine, or 2-methoxyuridine.

E130. The isolated nucleic acid molecule according to embodiment E115, wherein at least one uridine has been replaced with 1-methyl-pseudouridine, 4-thio-1-methyl-pseudouridine, 2-thio-1-methyl-pseudouridine, 1-methyl-1-deaza-pseudouridine, 2-thio-1-methyl-1-deaza-pseudouridine, or 2-thio-dihydrouridine.

E131. The isolated nucleic acid molecule according to embodiment E115, wherein at least one adenosine has been replaced with 2-aminopurine, 2,6-diaminopurine, 7-deaza-adenine, 7-deaza-8-aza-adenine, 7-deaza-2-aminopurine, or 7-deaza-8-aza-2-aminopurine.

E132. The isolated nucleic acid molecule according to embodiment E115, wherein at least one adenosine has been replaced with 7-deaza-2,6-diaminopurine, 7-deaza-8-aza-2,6-diaminopurine, 1-methyladenosine, N6-methyladenosine, N6-isopentenyladenosine, or N6-(cis-hydroxyisopentenyl)adenosine.

E133. The isolated nucleic acid molecule according to embodiment E115, wherein at least one adenosine has been replaced with 2-methylthio-N6-(cis-hydroxyisopentenyl) adenosine, N6-glycinylcarbamoyladenosine, N6-threonylcarbamoyladenosine, 2-methylthio-N6-threonyl carbamoyladenosine, N6,N6-dimethyladenosine, or 7-methyladenine.

E134. The isolated nucleic acid molecule according to embodiment E115, wherein at least one guanosine has been replaced with inosine, 1-methyl-inosine, wyosine, wybutosine, 7-deaza-guanosine, 7-deaza-8-aza-guanosine, or 6-thio-guanosine.

E135. The isolated nucleic acid molecule according to embodiment E115, wherein at least one guanosine has been replaced with 6-thio-7-deaza-guanosine, 6-thio-7-deaza-8-aza-guanosine, 7-methyl-guanosine, 6-thio-7-methyl-guanosine, 7-methylinosine, or 6-methoxy-guanosine.

E136. The isolated nucleic acid molecule according to embodiment E115, wherein at least one guanosine has been replaced with 1-methylguanosine, N2-methylguanosine, N2,N2-dimethylguanosine, 8-oxo-guanosine, 7-methyl-8-oxo-guanosine, or 1-methyl-6-thio-guanosine.

E137. The isolated nucleic acid molecule according to embodiment E115, wherein at least one cytidine has been replaced with 5-methylcytidine, 5-aza-cytidine, pseudoisocytidine, 3-methyl-cytidine, N4-acetylcytidine, or 5-formylcytidine.

E138. The isolated nucleic acid molecule according to embodiment E115, wherein at least one cytidine has been replaced with N4-methylcytidine, 5-hydroxymethylcytidine, 1-methyl-pseudoisocytidine, pyrrolo-cytidine, pyrrolo-pseudoisocytidine, or 2-thio-cytidine.

E139. The isolated nucleic acid molecule according to embodiment E115, wherein at least one cytidine has been replaced with 2-thio-5-methyl-cytidine, 4-thio-pseudoisocytidine, 4-thio-1-methyl-pseudoisocytidine, 4-thio-1-methyl-1-deaza-pseudoisocytidine, 1-methyl-1-deaza-pseudoisocytidine, or zebularine.

E140. The isolated nucleic acid molecule according to embodiment E115, wherein at least one cytidine has been replaced with 5-aza-zebularine, 5-methyl-zebularine, 5-aza-2-thio-zebularine, 2-thio-zebularine, 2-methoxy-cytidine, or 2-methoxy-5-methyl-cytidine.

E141. The isolated nucleic acid molecule according to embodiment E115, wherein 100% of the uridine nucleosides in the isolated nucleic acid molecule have been replaced with a nucleoside selected from the group consisting of pseudouridine, 5-methoxyuridine, 2-thiouridine, 4-thiouridine, N1-methylpseudouridine, 5-aza-uridine, 2-thio-5-aza-uridine, 4-thio-pseudouridine, 2-thio-pseudouridine, 5-hydroxyuridine, 4-methoxy-pseudouridine, 4-methoxy-2-thio-pseudouridine, 3-methyluridine, 5-carboxymethyl-uridine, 1-carboxymethyl-pseudouridine, 5-propynyl-uridine, 1-propynyl-pseudouridine, 2-methoxy-4-thio-uridine, 5-taurinomethyluridine, 1-taurinomethyl-pseudouridine, 5-taurinomethyl-2-thio-uridine, 1-taurinomethyl-4-thio-uridine, 5-methyl-uridine, 2-methoxyuridine, 1-methyl-pseudouridine, 4-thio-1-methyl-pseudouridine, 2-thio-1-methyl-pseudouridine, 1-methyl-1-deaza-pseudouridine, 2-thio-1-methyl-1-deaza-pseudouridine, and 2-thio-dihydrouridine.

E142. The isolated nucleic acid molecule according to embodiment E115, wherein 100% of the adenosine nucleosides in the isolated nucleic acid molecule have been replaced with a nucleoside selected from the group consisting of 2-aminopurine, 2,6-diaminopurine, 7-deaza-adenine, 7-deaza-8-aza-adenine, 7-deaza-2-aminopurine, 7-deaza-8-aza-2-aminopurine, 7-deaza-2,6-diaminopurine, 7-deaza-8-aza-2,6-diaminopurine, 1-methyladenosine, N6-methyladenosine, N6-isopentenyladenosine, N6-(cis-hydroxyisopentenyl)adenosine, 2-methylthio-N6-(cis-hydroxyisopentenyl)adenosine, N6-glycinylcarbamoyladenosine, N6-threonylcarbamoyladenosine, 2-methylthio-N6-threonyl carbamoyladenosine, N6,N6-dimethyladenosine, and 7-methyladenine.

E143. The isolated nucleic acid molecule according to embodiment E115, wherein 100% of guanosine nucleosides in the isolated nucleic acid molecule (e.g., an mRNA) have been replaced with a nucleoside selected from the group consisting of inosine, 1-methyl-inosine, wyosine, wybutosine, 7-deaza-guanosine, 7-deaza-8-aza-guanosine, 6-thio-guanosine, 6-thio-7-deaza-guanosine, 6-thio-7-deaza-8-aza-guanosine, 7-methyl-guanosine, 6-thio-7-methyl-guanosine, 7-methylinosine, 6-methoxy-guanosine, 1-methylguanosine, N2-methylguanosine, N2,N2-dimethylguanosine, 8-oxo-guanosine, 7-methyl-8-oxo-guanosine, and 1-methyl-6-thio-guanosine.

E144. The isolated nucleic acid molecule according to embodiment E115, wherein 100% of cytidine nucleosides in the isolated nucleic acid molecule (e.g., an mRNA) have been replaced with a nucleoside selected from the group consisting of 5-methylcytidine, 5-aza-cytidine, pseudoisocytidine, 3-methyl-cytidine, N4-acetylcytidine, 5-formylcytidine, N4-methylcytidine, 5-hydroxymethylcytidine, 1-methyl-pseudoisocytidine, pyrrolo-cytidine, pyrrolo-pseudoisocytidine, 2-thio-cytidine, 2-thio-5-methyl-cytidine, 4-thio-pseudoisocytidine, 4-thio-1-methyl-pseudoisocytidine, 4-thio-1-methyl-1-deaza-pseudoisocytidine, 1-methyl-1-deaza-pseudoisocytidine, zebularine, 5-aza-zebularine, 5-methyl-zebularine, 5-aza-2-thio-zebularine, 2-thio-zebularine, 2-methoxy-cytidine, 2-methoxy-5-methyl-cytidine, 4-methoxy-pseudoisocytidine, and 4-methoxy-1-methyl-pseudoisocytidine.

E145. The isolated nucleic acid molecule according to embodiment E115, wherein at least 25%, at least 50%, at least 75% or at least 100% of uridines have been replaced with pseudouridine

E146. The isolated nucleic acid molecule according to embodiment E115, wherein at least 25%, at least 50%, at least 75% or at least 100% of uridines have been replaced with 2-thiouridine.

E147. The isolated nucleic acid molecule according to embodiment E115, wherein at least 25%, at least 50%, at least 75% or at least 100% of uridines have been replaced with 4-thiouridine.

E148. The isolated nucleic acid molecule according to embodiment E115, wherein at least 25%, at least 50%, at least 75% or at least 100% of uridines have been replaced with 5-methoxyuridine .

E149. The isolated nucleic acid molecule according to embodiment E115, wherein at least 25%, at least 50%, at least 75% or at least 100% of uridines have been replaced with 4-methoxy-2-thio-pseudouridine.

E150. The isolated nucleic acid molecule according to embodiment E115, wherein at least 25%, at least 50%, at least 75% or at least 100% of uridines have been replaced with 4-methoxy-pseudouridine.

E151. The isolated nucleic acid molecule according to embodiment E115, wherein at least 25%, at least 50%, at least 75% or at least 100% of uridines have been replaced with 4-methoxy-pseudouridine.

E152. The isolated nucleic acid molecule according to embodiment E115, wherein at least 25%, at least 50%, at least 75% or at least 100% of uridines have been replaced with 5-hydroxyuridine.

E153. The isolated nucleic acid molecule according to embodiment E115, wherein at least 25%, at least 50%, at least 75% or at least 100% of uridines have been replaced with 2-thio-pseudouridine.

E154. The isolated nucleic acid molecule according to embodiment E115, wherein at least 25%, at least 50%, at least 75% or at least 100% of uridines have been replaced with 2-thio-5-aza-uridine.

E155. The isolated nucleic acid molecule according to embodiment E115, wherein at least 25%, at least 50%, at least 75% or at least 100% of uridines have been replaced with 1-carboxymethyl-pseudouridine.

E156. The isolated nucleic acid molecule according to embodiment E115, wherein at least 25%, at least 50%, at least 75% or at least 100% of uridines have been replaced N1-methylpseudouridine.

E157. The isolated nucleic acid molecule according to any one of embodiments E126 to E130, wherein at least 25%, at least 50%, at least 75% or at least 100% of cytidines have been replaced with replaced with 5-methylcytidine or 3-methyl-cytidine.

E158. The isolated nucleic acid molecule according to embodiment E115, wherein: 25% of uridines have been replaced with 4-thiouridine; 50% of uridines have been replaced with 4-thiouridine; 100% of uridines have been replaced with 4-thiouridine; 25% of uridines have been replaced with 2-thiouridine (s2U) and 25% of cytidines have been replaced with 5-methylcytidine (m5C); 50% of uridines have been replaced with 2-thiouridine (s2U); 100% of uridines have been replaced with pseudouridine (Ψ); 100% of uridines have been replaced with pseudouridine (Ψ) and 100% of cytidines have been replaced with 5-methylcytidine (5mC); 25% of uridines have been replaced with 5-methoxyuridine (5moU) and 50% of cytidines have been replaced with 5-methylcytidine (5mC); 25% of uridines have been replaced with 5-methoxyuridine (5moU) and 100% of cytidines have been replaced with 5-methylcytidine (5mC); 100% of uridines have been replaced with 5-methoxyuridine (5moU); 100% of uridines have been replaced with 5-methoxyuridine (5moU) and 100% of cytidines have been replaced with 5-methylcytidine (5mC); 100% of uridines have been replaced with N1-methylpseudouridine (1mΨ); or, 100% of uridines have been replaced with N1-methylpseudouridine (1mΨ) and of cytidines have been replaced with 100% 5-methylcytidine (5mC).

E159. A vector or set of vectors comprising the optimized nucleic acid molecule or set of optimized nucleic acid molecules prepared according to the method of any one of embodiments E1 to E91, or a complement thereof.

E160. A method for producing a protein encoded by an optimized nucleic acid molecule prepared according to the method of any one of embodiments E1 to E91, wherein the expression is conducted using an in vitro translation system.

E161. A pharmaceutical composition comprising an optimized nucleic acid prepared according to the method of any one of embodiments E1 to E91, or a vector or set of vectors according to embodiment E159, and a pharmaceutically acceptable vehicle or excipient.

All patents and publications referred to herein are expressly incorporated by reference in their entireties.

Aspects of the present disclosure can be further defined by reference to the following non-limiting examples, which describe in detail preparation of certain antibodies of the present disclosure and methods for using antibodies of the present disclosure. It will be apparent to those skilled in the art that many modifications, both to materials and methods, can be practiced without departing from the scope of the present disclosure.

### Examples

### Materials and Methods

### I. Manufacture of polynucleotides

According to the present disclosure, the manufacture of polynucleotides (e.g., mRNAs) and or parts or regions thereof can be accomplished utilizing the methods taught in PCT Publ. Nos. WO2015/058069, WO2015/051214, and U.S. Pat. Appl. Publ. No. US2015/0050354, and U.S. Prov. Appl. No. 61/800,049 filed March 15, 2013 entitled "Manufacturing Methods for Production of RNA Transcripts", the contents of which is incorporated herein by reference in its entirety. Purification methods include those taught in PCT Publ. Nos. WO2015/058069, WO2015/051214, and U.S. Pat. Appl. Publ. No. US2015/0050354, and U.S. Prov. Appl. No's 61/799,872 filed March 15, 2013 entitled "Methods of removing DNA fragments in mRNA production"; and, 61/794,842 filed March 15, 2013, entitled "Ribonucleic acid purification", each of which is incorporated herein by reference in its entirety. Characterization of the polynucleotides disclosed herein can be accomplished using a procedure selected from the group consisting of polynucleotide mapping, reverse transcriptase sequencing, charge distribution analysis, and detection of RNA impurities, wherein characterizing comprises determining the RNA transcript sequence, determining the purity of the RNA transcript, or determining the charge heterogeneity of the RNA transcript. Such methods are taught in, for example, PCT Publ. Nos. WO2015/058069, WO2015/051214, and U.S. Pat. Appl. Publ. No. US2015/0050354, and U.S. Prov. Appl. Nos. 61/798,945 filed March 15, 2013 entitled "Characterization of mRNA molecules"; 61/799,905 filed March 15, 2013 entitled "Analysis of mRNA Heterogeneity and Stability" and 61/800,110 filed March 15, 2013 entitled "Ion Exchange Purification of mRNA" the contents of each of which is incorporated herein by reference in its entirety.

### II. PCR for cDNA Production

PCR procedures for the preparation of cDNA are performed using 2x KAPA HIFI^{™} HotStart ReadyMix by Kapa Biosystems (Woburn, MA). This system includes 2x KAPA ReadyMix12.5 µl; Forward Primer (10 µM) 0.75 µl; Reverse Primer (10 µM) 0.75 µl; Template cDNA -100 ng; and dH20 diluted to 25.0 µl. The reaction conditions are at 95° C for 5 min. and 25 cycles of 98° C for 20 sec, then 58° C for 15 sec, then 72° C for 45 sec, then 72° C for 5 min. then 4° C to termination.

The reverse primer of the instant invention incorporates a poly-T120 for a poly-A120 in the mRNA. Other reverse primers with longer or shorter poly(T) tracts can be used to adjust the length of the poly(A) tail in the polynucleotide mRNA. The reaction is cleaned up using Invitrogen's PURELINK^{™} PCR Micro Kit (Carlsbad, CA) per manufacturer's instructions (up to 5 µg). Larger reactions will require a cleanup using a product with a larger capacity. Following the cleanup, the cDNA is quantified using the NANODROP^{™} and analyzed by agarose gel electrophoresis to confirm the cDNA is the expected size. The cDNA is then submitted for sequencing analysis before proceeding to the in vitro transcription reaction.

### III. In vitro Transcription (IVT)

### A. Synthesis of mRNA Constructs in Preparation for IVT

*i. Restriction Digest of Plasmid DNA:* DNA plasmid is digested by incubation at 37°C for 2 hr in a 50 µL reaction containing DNA plasmid (50 ng/µL), BSA (1X), 1X NEBuffer 4 (50mM potassium acetate, 20mM Tris-acetate, 10mM magnesium acetate, 1mM DTT, pH 7.9), and XbaI (400 U/mL) (New England Biolabs). The restriction digest is analyzed by 1% agarose gel and used directly for PCR.

*ii. DNA Template Amplification:* The desired DNA template is amplified by PCR in 100 µL reactions using linearized plasmid (20 ng), dNTPs (0.2 µM each), forward primer (0.2 µM), reverse primer (0.2 µM), 1X Q5 reaction buffer, and Q5 high-fidelity DNA polymerase (20 U/mL) (New England Biolabs). All components are kept on ice until added to the thermocycler. The reaction conditions are at 95° C for 4 min. and 30 cycles of 98° C for 15 sec, then 72° C for 45 sec, then 72° C for 20 sec per kb, then 72° C for 5 min. then 4° C to termination. The PCR product is analyzed by capillary electrophoresis (CE) (Agilent 2100 Bioanalyzer) and desalted by ultrafiltration (Amicon).

### B. IVT Reaction

In vitro transcription (IVT) reactions are performed in 50 uL containing template DNA (25 ng/µL), NTPs (7.6 mM each), 1X T7 IVT buffer, RNase Inhibitor (1 U/µL), Pyrophosphatase (1 U/µL), and T7 RNA polymerase (7 U/µL) (NEB). In general, 24 50uL reactions per construct are used. Modified mRNA may be generated using 5-methyl-CTP and 1-methyl-pseudoUTP or any chosen modified triphosphate. IVT reactions are incubated at 37 °C for 4 hr, after which 2.5 µL of DNase I (2000 U/mL) (NEB) is added and the reaction allowed to incubated for another 45 min. The reactions are combined and purified using MEGAclear spin columns (Ambion) and eluted in 250 µL water. The IVT product is analyzed by CE (Agilent 2100 Bioanalyzer).

### IV. Enzymatic Capping

Capping of a polynucleotide is performed as follows where the mixture includes: IVT RNA 60 µg-180µg and dH₂0 up to 72 µl. The mixture is incubated at 65° C for 5 minutes to denature RNA, and then is transferred immediately to ice.

The protocol then involves the mixing of 10x Capping Buffer (0.5 M Tris-HCl (pH 8.0), 60 mM KCl, 12.5 mM MgCl₂) (10.0 µl); 20 mM GTP (5.0 µl); 20 mM S-Adenosyl Methionine (2.5 µl); RNase Inhibitor (100 U); 2'-O-Methyltransferase (400U); Vaccinia capping enzyme (Guanylyl transferase) (40 U); dH₂0 (Up to 28 µl); and incubation at 37° C for 30 minutes for 60 µg RNA or up to 2 hours for 180 µg of RNA.

The polynucleotide is then purified using Ambion's MEGACLEAR^{™} Kit (Austin, TX) following the manufacturer's instructions. Following the cleanup, the RNA is quantified using the NANODROP^{™} (ThermoFisher, Waltham, MA) and analyzed by agarose gel electrophoresis to confirm the RNA is the proper size and that no degradation of the RNA has occurred. The RNA product may also be sequenced by running a reverse-transcription-PCR to generate the cDNA for sequencing.

### V. PolyA Tailing Reaction

Without a poly-T in the cDNA, a poly-A tailing reaction must be performed before cleaning the final product. This is done by mixing Capped IVT RNA (100 µl); RNase Inhibitor (20 U); 10x Tailing Buffer (0.5 M Tris-HCl (pH 8.0), 2.5 M NaCl, 100 mM MgCl₂)(12.0 µl); 20 mM ATP (6.0 µl); Poly-A Polymerase (20 U); dH₂0 up to 123.5 µl and incubation at 37° C for 30 min. If the poly-A tail is already in the transcript, then the tailing reaction may be skipped and proceed directly to cleanup with Ambion's MEGACLEAR^{™} kit (Austin, TX) (up to 500 µg). Poly-A Polymerase is preferably a recombinant enzyme expressed in yeast. It should be understood that the processivity or integrity of the polyA tailing reaction may not always result in an exact size polyA tail. Hence polyA tails of approximately between 40-200 nucleotides, e.g, about 40, 50, 60, 70, 80, 90, 91, 92, 93, 94, 95, 96, 97, 98, 99, 100, 101, 102, 103, 104, 105, 106, 107, 108, 109, 110, 150-165, 155, 156, 157, 158, 159, 160, 161, 162, 163, 164 or 165 are within the scope of the invention.

### VI. Natural 5' Caps and 5' Cap Analogues

5'-capping of polynucleotides can be completed concomitantly during the *in vitro-*transcription reaction using the following chemical RNA cap analogs to generate the 5'-guanosine cap structure according to manufacturer protocols: 3'-O-Me-m7G(5')ppp(5') G [the ARCA cap];G(5')ppp(5')A; G(5')ppp(5')G; m7G(5')ppp(5')A; m7G(5')ppp(5')G (New England BioLabs, Ipswich, MA). 5'-capping of modified RNA can be completed posttranscriptionally using a Vaccinia Virus Capping Enzyme to generate the "Cap 0" structure: m7G(5')ppp(5')G (New England BioLabs, Ipswich, MA). Cap 1 structure can be generated using both Vaccinia Virus Capping Enzyme and a 2'-O methyl-transferase to generate: m7G(5')ppp(5')G-2'-O-methyl. Cap 2 structure may be generated from the Cap 1 structure followed by the 2'-O-methylation of the 5'-antepenultimate nucleotide using a 2'-O methyltransferase. Cap 3 structure may be generated from the Cap 2 structure followed by the 2'-O-methylation of the 5'-preantepenultimate nucleotide using a 2'-O methyl-transferase. Enzymes are preferably derived from a recombinant source. When transfected into mammalian cells, the modified mRNAs have a stability of between 12-18 hours or more than 18 hours, e.g., 24, 36, 48, 60, 72 or greater than 72 hours.

### VII. Capping Assays:

*A. Protein Expression Assay:* Polynucleotides encoding a polypeptide, containing any of the caps taught herein can be transfected into cells at equal concentrations. 6, 12, 24 and 36 hours post-transfection the amount of protein secreted into the culture medium can be assayed by ELISA. Synthetic polynucleotides that secrete higher levels of protein into the medium would correspond to a synthetic polynucleotide with a higher translationally-competent Cap structure.

*B. Purity Analysis Synthesis:* Polynucleotides encoding a polypeptide, containing any of the caps taught herein can be compared for purity using denaturing Agarose-Urea gel electrophoresis or HPLC analysis. Polynucleotides with a single, consolidated band by electrophoresis correspond to the higher purity product compared to polynucleotides with multiple bands or streaking bands. Synthetic polynucleotides with a single HPLC peak would also correspond to a higher purity product. The capping reaction with a higher efficiency would provide a more pure polynucleotide population.

*C. Cytokine Analysis:* Polynucleotides encoding a polypeptide, containing any of the caps taught herein can be transfected into cells at multiple concentrations. 6, 12, 24 and 36 hours post-transfection the amount of pro-inflammatory cytokines such as TNF-alpha and IFN-beta secreted into the culture medium can be assayed by ELISA. Polynucleotides resulting in the secretion of higher levels of pro-inflammatory cytokines into the medium would correspond to polynucleotides containing an immune-activating cap structure.

*D. Capping Reaction Efficiency:* Polynucleotides encoding a polypeptide, containing any of the caps taught herein can be analyzed for capping reaction efficiency by LC-MS after nuclease treatment. Nuclease treatment of capped polynucleotides would yield a mixture of free nucleotides and the capped 5'-5-triphosphate cap structure detectable by LC-MS. The amount of capped product on the LC-MS spectra can be expressed as a percent of total polynucleotide from the reaction and would correspond to capping reaction efficiency. The cap structure with higher capping reaction efficiency would have a higher amount of capped product by LC-MS.

### VIII. Agarose Gel Electrophoresis of Modified RNA or RT PCR Products

Individual polynucleotides (200-400 ng in a 20 µl volume) or reverse transcribed PCR products (200-400 ng) are loaded into a well on a non-denaturing 1.2% Agarose E-Gel (Invitrogen, Carlsbad, CA) and run for 12-15 minutes according to the manufacturer protocol.

### IX. Nanodrop Modified RNA Quantification and UV Spectral Data

Modified polynucleotides in TE buffer (1 µl) are used for Nanodrop UV absorbance readings to quantitate the yield of each polynucleotide from an chemical synthesis or in vitro transcription reaction.

### X. Formulation of Modified mRNA Using Lipidoids

Polynucleotides are formulated for in vitro experiments by mixing the polynucleotides with the lipidoid at a set ratio prior to addition to cells. In vivo formulation may require the addition of extra ingredients to facilitate circulation throughout the body. To test the ability of these lipidoids to form particles suitable for in vivo work, a standard formulation process used for siRNA-lipidoid formulations may used as a starting point. After formation of the particle, a polynucleotide is added and allowed to integrate with the complex. The encapsulation efficiency is determined using a standard dye exclusion assays.

### XI. Method of Screening for Protein Expression

*A. Electrospray Ionization:* A biological sample which may contain proteins encoded by a polynucleotide administered to the subject is prepared and analyzed according to the manufacturer protocol for electrospray ionization (ESI) using 1, 2, 3 or 4 mass analyzers. A biologic sample may also be analyzed using a tandem ESI mass spectrometry system. Patterns of protein fragments, or whole proteins, are compared to known controls for a given protein and identity is determined by comparison.

*B. Matrix-Assisted Laser Desorption*/*Ionization:* A biological sample which may contain proteins encoded by one or more polynucleotides administered to the subject is prepared and analyzed according to the manufacturer protocol for matrix-assisted laser desorption/ionization (MALDI). Patterns of protein fragments, or whole proteins, are compared to known controls for a given protein and identity is determined by comparison.

*C. Liquid Chromatography-Mass spectrometry-Mass spectrometry:* A biological sample, which may contain proteins encoded by one or more polynucleotides, may be treated with a trypsin enzyme to digest the proteins contained within. The resulting peptides are analyzed by liquid chromatography-mass spectrometry-mass spectrometry (LC/MS/MS). The peptides are fragmented in the mass spectrometer to yield diagnostic patterns that can be matched to protein sequence databases via computer algorithms. The digested sample may be diluted to achieve 1 ng or less starting material for a given protein. Biological samples containing a simple buffer background (e.g. water or volatile salts) are amenable to direct in-solution digest; more complex backgrounds (e.g. detergent, non-volatile salts, glycerol) require an additional clean-up step to facilitate the sample analysis. Patterns of protein fragments, or whole proteins, are compared to known controls for a given protein and identity is determined by comparison.

### Example 1

### Ramp Design

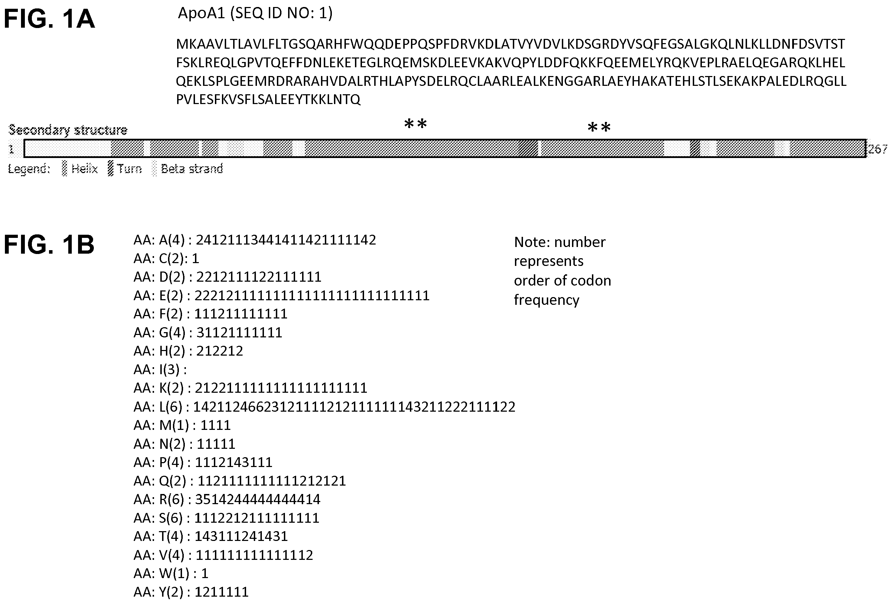

FIG. 1A shows the sequence and secondary structures of ApoA1 . The amino acid distribution shown in FIG. 1B shows that codons with the lower frequencies tend to cluster in the regions closer to the N-terminus, C-terminus, and central region of the two long alpha helical regions indicated by **. Such regions would be regions where translation rate would be slower (ramps). To test the role of G/C patterns and ramp composition, 10 biased codon sets (CO1 to CO10) were generated. Codon sets CO1, CO3, CO5, CO7 and CO9 were designed to introduce a GC rich ramp in the first 30 amino acids of the target protein (designate Target Protein 1). Codon sets CO2, CO4, CO6, CO8, and CO10 were created to introduce a GC poor ramp in the first 30 amino acids of the target protein. Codon sets CO1 and CO2 were composed of codons from rare sequences. Codon sets CO3 and CO4 were designed to introduce a high GC content in the sequence. Codon sets CO5 and CO6 were designed to introduce a low GC content in the sequence. Codon sets CO7 and CO8 were designed to introduce a high G content in the sequence. Codon sets CO9 and C10 were designed to introduce a high C content in the sequence (see FIG. 2B). Accordingly, a sequence encoded by codons from CO4 would have, as shown in FIG. 2C, a low GC ramp in the segment of the encoding sequence corresponding to the first 30 amino acids, whereas the rest of the sequence would have a high GC content.

Expression data (FIG. 2A) showed that high expression levels corresponded to constructs with low GC ramps located in the first 30 amino acids of each construct, independently of whether the rest of the construct was high GC, low GC, high G, or high C.

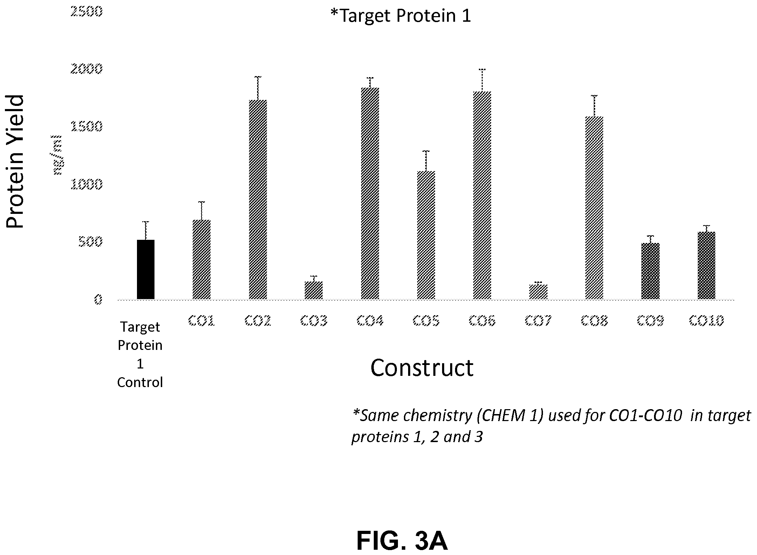

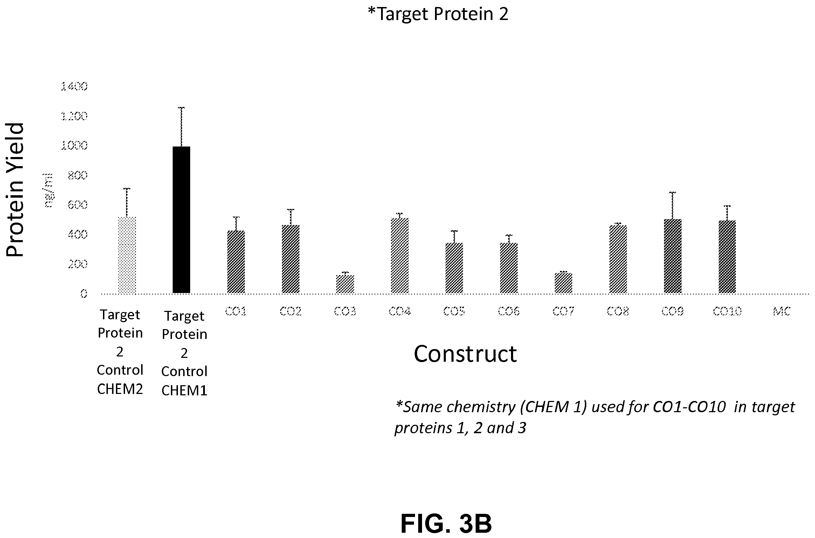

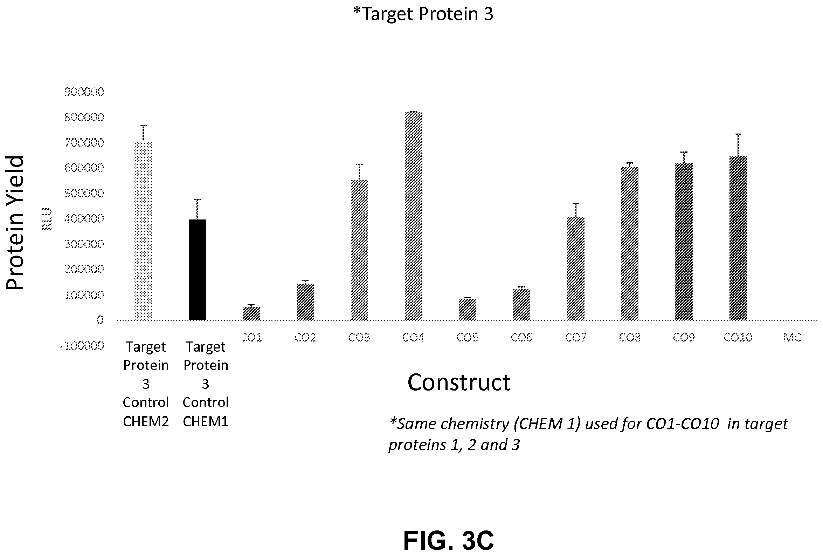

To determine whether the observation in wild type Target Protein 1 could be extrapolated to the same protein encoded by a chemically-modified mRNA, the encoding mRNA was chemically modified by replacing natural nucleobases with unnatural nucleobases according to the chemistry pattern described as Chem1. FIG. 3A shows that when the same target protein (Target Protein 1) was chemically modified according to Chem1, the results obtained were comparable to those obtained for the wild type protein. As shown in FIG. 3A, high levels of expression were obtained using CO2, CO4, CO6, and CO8, which have as a common characteristic the presence of a GC poor ramp in the first 30 amino acids. The experimental data shown in FIGS. 3B and 3C corresponds to constructs for another two target proteins (Target Protein 2 and Target Protein 3), using the same codon sets (CO1 to CO10) and the same chemistry used in FIG. 3A. Protein yield for the additional target proteins indicated also that high expression levels were achieved when using a GC poor ramp in the first 30 amino acids. In particular, high levels of expression were obtained in each case when CO4 was used.

The results also suggest that the effect of the selected codon set, even though CO4 is consistently the best codon set for the three tested targets, can vary among targets, suggesting that optimization rules may be tailored in a target-specific manner to achieve highest levels of expression.

The results also suggest that high expression levels can be obtained for the same target protein even if the level of sequence identity between mRNAs is relatively low. For example, as shown below, the level of sequence identity between the mRNA generated using CO4 (the best performer for Protein Target 1) and the mRNA generated using CO6 (the second best performer) was just 63%. The levels of sequence identity between the mRNAs generated using CO4 and CO5 was even lower, at 56%.

**TABLE 2: Identity table indicating percentage of sequence identity between the mRNA sequences generated for Target Protein 1 using codon sets CO1 to CO10.**

| | CO7 | CO8 | CO3 | CO4 | CO9 | CO10 | CO1 | CO2 | CO5 | CO6 |
|---|---|---|---|---|---|---|---|---|---|---|
| CO7 | 100 | 93 | 90 | 83 | 79 | 72 | 73 | 66 | 65 | 58 |
| CO8 | 93 | 100 | 83 | 90 | 72 | 79 | 66 | 73 | 58 | 65 |
| CO3 | 90 | 83 | 100 | 93 | 88 | 81 | 69 | 62 | 63 | 56 |
| CO4 | 83 | 90 | 93 | 100 | 81 | 88 | 62 | 69 | 56 | 63 |
| CO9 | 79 | 72 | 88 | 81 | 100 | 93 | 73 | 65 | 66 | 59 |
| CO10 | 72 | 79 | 81 | 88 | 93 | 100 | 65 | 73 | 59 | 66 |
| CO1 | 73 | 66 | 69 | 62 | 73 | 65 | 100 | 93 | 80 | 73 |
| CO2 | 66 | 73 | 62 | 69 | 65 | 73 | 93 | 100 | 73 | 80 |
| CO5 | 65 | 58 | 63 | 56 | 66 | 59 | 80 | 73 | 100 | 93 |
| CO6 | 58 | 65 | 56 | 63 | 59 | 66 | 73 | 80 | 93 | 100 |

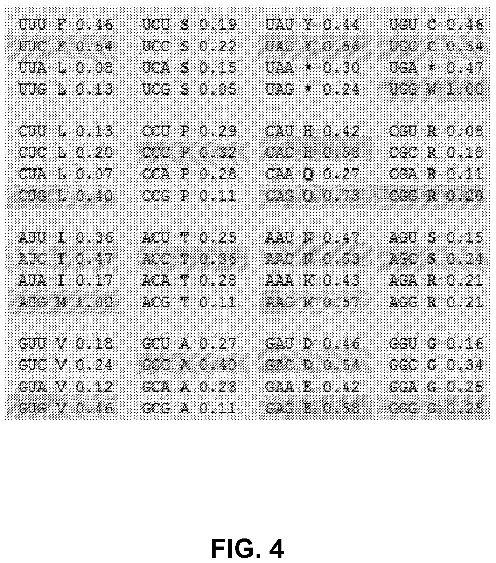

As shown in FIG. 4 there is a correlation between GC content and codon frequency. 19 out of 20 of the highest frequency codons also have highest GC-content codons, and 15 out of the 20 lowest frequency codons are also one of the lowest GC-content codons.

### Example 2

### Uridine Content Optimization

FIG. 5A shows the analysis of sequences encoding Protein Target 1 using a 20-mer sliding window to calculate the % of uridine over the length of the gene. The analysis was applied to two of the constructs generated and expressed in the previous example, CO3 and CO4, both of which were GC rich. The figure shows the theoretical maximum and theoretical minimum content for the two constructs. The graphic shows an almost perfect overlay between CO3, CO4, and the minimum uridine curve. The graphic also shows the ramp region in CO4. In the ramp region, the uridine content is close to the theoretical maximum uridine content. According to this data, reducing the GC content to the lowest possible values also results in the reduction of uridine to the lowest possible value.

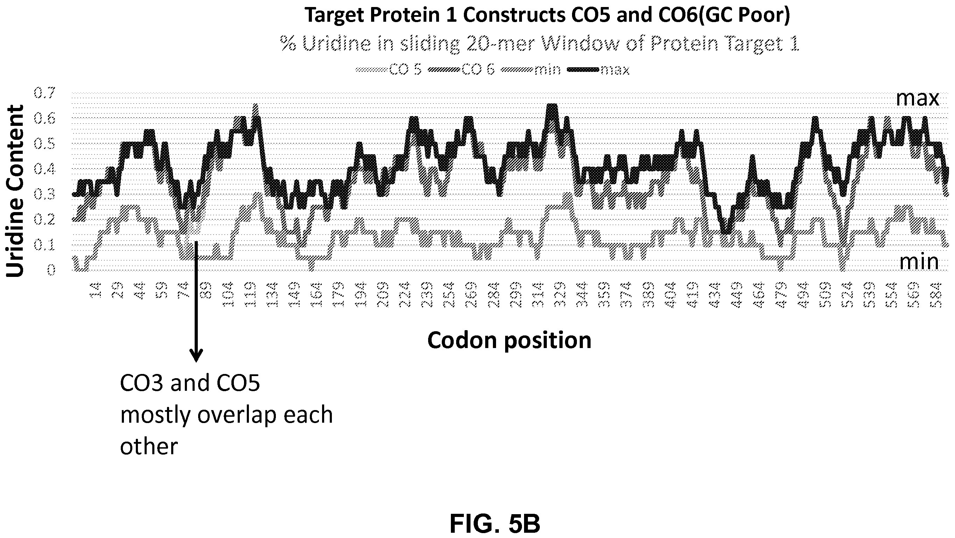

FIG. 5B presents another set of curves analyzing sequences encoding Protein Target 1 using a 20-mer sliding window to calculate the % of uridine over the length of the gene. In this case, the constructs were CO5 and CO6, both of which were GC poor. In this case, uridine content was close to the theoretical maximum. FIG. 5B further the overlay between low GC content and maximum theoretical uridine content which can be compared to the overlay between high GC content and minimum theoretical uridine content.

### Example 3

### Uridine Content and Ramp Optimization

In order to decouple ramp and uridine optimization contributions, a "uridine light" ramp approach was designed. According to this strategy, an orthogonal set of codon maps was created using machine learning that minimized uridine content and uridine clustering in the final product. Fifty of the codon maps were un-biased codon maps. Another fifty codon maps were uridine-biased codon maps.

Luciferase was used as the target protein in this set of experiments. Relative amino acid prevalence in luciferase is shown in FIG. 6A. The 100 codon maps generated were combined and used to generate 100 luciferase gene constructs. Codon bias from uridine selection in normal constructs and uridine biased constructs in shown in FIG. 6B.

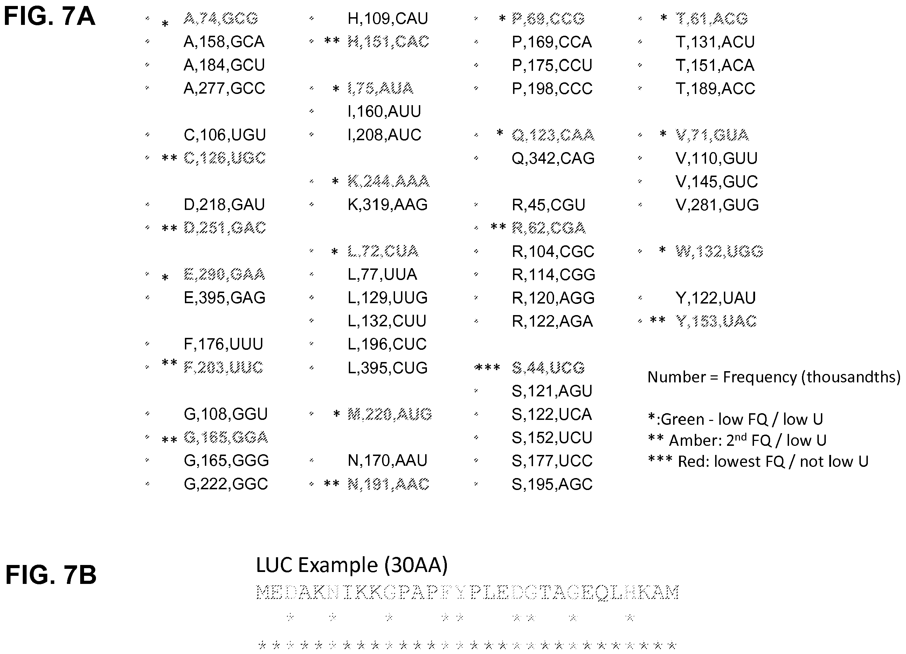

An exemplary uridine-biased codon map is shown in FIG. 7A, which also shows the distribution of amino acids encoded by those codons in the N-terminal 30 amino acids of luciferase (ramp region). Applying a 20-mer sliding window to the ramp region in luciferase to show % uridine highlights the differences between as HI-GC or LO-GC ramp (FIG. 8A) and a uridine-sensitive ramp ("uridine light" ramp) (FIG. 8B).

### Example 4

### In vitro Translation and In vivo Expression of Optimized Sequences

FIGS. 9 to 12 show the interplay between codon optimization, chemistry, and, optionally, target specificity of expression optimization. FIG. 9 shows in vitro translation data corresponding to expression of Target Protein 2 under control conditions (wild type) compared to the expression data for constructs generated using 4 novel codon sets (CO1, CO2, CO3 and CO4) and applying 4 different chemistries. Each chemistry represents a different set of unnatural nucleobase substitutions. Expression took place in HeLa cells. Similarly, FIG. 11 shows in vitro translation data corresponding to control expression of Target Protein 2 compared to the expression data for constructs generated using 6 novel codon sets (CO5, CO6, CO7, CO8, CO9 and CO10) and applying 4 different chemistries. Each chemistry represents a different set of unnatural nucleobase substitutions. Expression took place in HeLa cells.

FIGS. 11 and 12 correspond to in vivo activity of the constructs presented in FIG. 9 and FIG. 10, respectively, after intravenous administration of 0.05 mg/kg of each construct in MC3-LNP to mice.

Taken together, the data presented indicates that rule set for codon optimization depends on optimization-specific parameters such as uridine content, and on the specific chemistry used for target modification, and also can depend, in certain instances, on the specific target protein.

### Example 5

### In vivo Expression of Additional Optimized Sequences

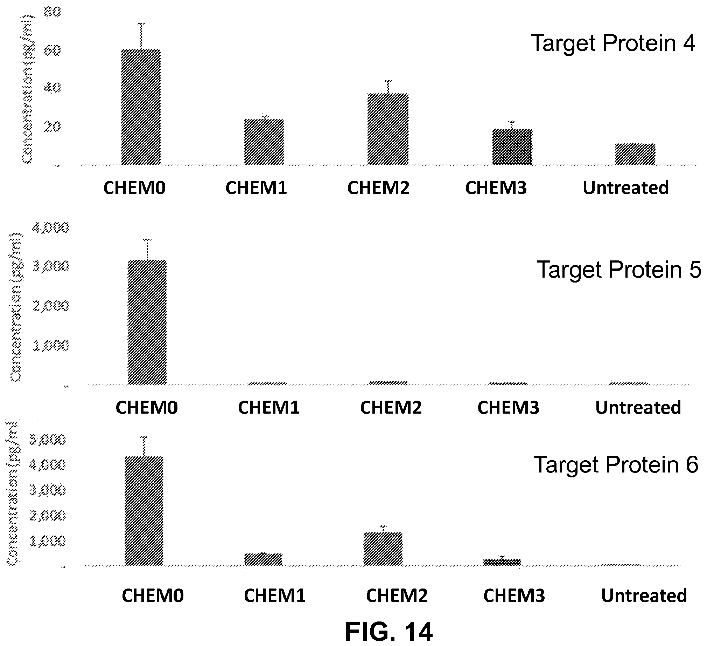

The optimization strategies outlined above were applied to Target Protein 2, but in addition to applying three different chemistries (Chem 1, Chem 2, and Chem 3), the constructs were also subject to bioinformatic optimization specific for mRNA chemistry composition and tissue of expression. Target Protein 2 mRNAs were administered intravenously to mice. All doses were formulated in LNP and dosed at 0.05 mg/kg (mRNA). The observed results (in vivo expression in mice) are shown in FIG. 14. The data presented shows that Chem3 chemistry outperformed the best Chem2 expression.

Similar experiments were conducted using another three target proteins (Target Protein 4, Target Protein 5, and Target Protein 6). See FIG. 15. Target protein mRNAs were administered intravenously to mice. All doses were formulated in MC3-LNP3 and dosed at 0.2 mg/kg (mRNA). Samples were collected 6 hours post dose. Immune profile analysis showed that the immune profile for Chem3 (even unpurified) was the most desirable, being comparable to the untreated control group *in vivo.*

It is to be appreciated that the Detailed Description section, and not the Summary and Abstract sections, is intended to be used to interpret the claims. The Summary and Abstract sections may set forth one or more but not all exemplary embodiments of the present invention as contemplated by the inventor(s), and thus, are not intended to limit the present invention and the appended claims in any way.

The present invention has been described above with the aid of functional building blocks illustrating the implementation of specified functions and relationships thereof. The boundaries of these functional building blocks have been arbitrarily defined herein for the convenience of the description. Alternate boundaries can be defined so long as the specified functions and relationships thereof are appropriately performed.

The foregoing description of the specific embodiments will so fully reveal the general nature of the invention that others can, by applying knowledge within the skill of the art, readily modify and/or adapt for various applications such specific embodiments, without undue experimentation, without departing from the general concept of the present invention. Therefore, such adaptations and modifications are intended to be within the meaning and range of equivalents of the disclosed embodiments, based on the teaching and guidance presented herein. It is to be understood that the phraseology or terminology herein is for the purpose of description and not of limitation, such that the terminology or phraseology of the present specification is to be interpreted by the skilled artisan in light of the teachings and guidance.

The breadth and scope of the present invention should not be limited by any of the above-described exemplary embodiments, but should be defined only in accordance with the following claims and their equivalents.

The present disclosure also includes the following items.
1. A multiparametric method for optimizing a candidate nucleic acid sequence, the method comprising at least one optimization method selected from:
   (i) modifying at least one subsequence in the candidate nucleic acid sequence to generate a ramp subsequence;
   (ii) substituting at least one codon in the candidate nucleic acid sequence with an alternative codon to increase or decrease uridine content to generate a uridine-modified sequence;
   (iii) substituting at least one codon in the candidate nucleic acid sequence or the uridine-modified sequence with a faster recharging codon;
   (iv) substituting at least one codon in the candidate nucleic acid sequence with an alternative codon having a higher codon frequency in the synonymous codon set;
   (v) substituting at least one natural nucleobase in the candidate nucleic acid sequence with an alternative synthetic nucleobase;
   (vi) substituting at least one internucleoside linkage in the candidate nucleic acid sequence with a non-natural internucleoside linkage; and,
   (vii) combinations thereof.
   wherein the resulting optimized nucleic acid sequence has at least one optimized property with respect to the candidate nucleic acid sequence.
2. The multiparametric method according to item 1, wherein the optimized nucleic acid sequence comprises at least one ramp subsequence.
3. The multiparametric method according to item 1, wherein the codons in the optimized nucleic acid sequence are selected from an optimized codon set which is a limited codon set.
4. The multiparametric method according to item 3, wherein the limited codon set consists of 20 codons, and wherein each codon encodes one of 20 amino acids.
5. The multiparametric method according to item 4, wherein the limited codon set is:
   (a) UUC, UUG, CUG, AUC, AUG, GUG, AGC, CCC, ACC, GCC, UAC, CAC, CAG, AAC, AAG, GAG, UGC, UGG, AGG, GGC;
   (b) UUU, CUA, AUA, AUG, GUA, UCG, CCG, ACG, GCG, UAU, CAU, CAA, AAU, AAA, GAU, GAA, UGU, UGG, CGU, GGU;
   (c) UUC, CUV, AUM, AUG, GUV, AGC, CCV, ACV, GCV, UAC, CAC, CAR, AAC, AAR, GAC, GAR, UGC, UGG, CGV, GGV; or,
   (d) UUC, CUV, AUM, AUG, GUV, AGC, CCV, ACV, GCV, UAC, CAC, CAR, AAC, AAR, GAC, GAR, UGC, UGG, AGR, GGV.
6. The multiparametric method according to item 1, wherein the uridine content (absolute or relative content) of the uridine-modified sequence is less than 50%, 49%, 48%, 47%, 46%, 45%, 44%, 43%, 42%, 41%, 40%, 39%, 38%, 37%, 36%, 35%, 34%, 33%, 32%, 31%, 30%, 29%, 28%, 27%, 26%, 25%, 24%, 23%, 22%, 21%, 20%, 19%, 18%, 17%, 16%, 15%, 14%, 13%, 12%, 11%, 10%, 9%, 8%, 7%, 6%, 5%, 4%, 3%, 2% or 1%.
7. The multiparametric method according to item 1, wherein the optimized nucleic acid sequence comprises an overall increase in Guanine/Cytosine (G/C) content (absolute or relative) relative to the G/C content (absolute or relative) of the candidate nucleic acid sequence.
8. The multiparametric method according to item 1, wherein at least about 5%, at least about 10%, at least about 15%, at least about 20%, at least about 25%, at least about 30%, at least about 35%, at least about 40%, at least about 45%, at least about 50%, at least about 55%, at least about 60%, at least about 65%, at least about 70%, at least about 75%, at least about 80%, at least about 85%, at least about 90%, at least about 95%, at least about 99%, or 100% of the codons in the candidate nucleic acid sequence are substituted with alternative codons, each alternative codon having a codon frequency higher than the codon frequency of the substituted codon in the synonymous codon set.
9. The multiparametric method according to item 1, wherein at least about 5%, at least about 10%, at least about 15%, at least about 20%, at least about 25%, at least about 30%, at least about 35%, at least about 40%, at least about 45%, at least about 50%, at least about 55%, at least about 60%, at least about 65%, at least about 70%, at least about 75%, at least about 80%, at least about 85%, at least about 90%, at least about 95%, at least about 99%, or 100% of the codons in the candidate nucleic acid sequence are substituted with faster recharging codons.
10. The multiparametric method according item 1, wherein the method comprises one, two, three, four, five, or six, optimization method selected from the group consisting of (i) modifying at least one subsequence in the candidate nucleic acid sequence to generate a ramp subsequence; (ii) substituting at least one codon in the candidate nucleic acid sequence with an alternative codon to increase or decrease uridine content to generate a uridine-modified sequence; (iii) substituting at least one codon in the candidate nucleic acid sequence or the uridine-modified sequence with a fast recharging codon; (iv) substituting at least one codon in the candidate nucleic acid sequence with an alternative codon having a higher codon frequency in the synonymous codon set; (v) substituting at least one natural nucleobase in the candidate nucleic acid sequence with an alternative synthetic nucleobase; and (vi) substituting at least one internucleoside linkage in the candidate nucleic acid sequence with a non-natural internucleoside linkage.
11. The multiparametric method according to item 1, wherein at least 5%, at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 85%, at least 90%, at least 95%, at least 96%, at least 97%, at least 98%, at least 99% or 100% of the codons in the candidate nucleic acid sequence are replaced.
12. A method for expressing a protein in a target tissue or cell or an *in vitro* translation system, the method comprising:
   (a) obtaining an optimized gene sequence for expression in a human *in vivo* systemically or in a target tissue or target cell, using a method according to item 1;
   (b) synthesizing a nucleic acid molecule comprising the optimized gene sequence;
   (c) introducing the nucleic acid molecule into the target tissue or cell or combining it with the *in vitro* translation system,
13. The method according to item 1, wherein the at least one optimized property with respect to the candidate nucleic acid sequence is selected from:
   (i) increase in transcription efficacy;
   (ii) increase in translation efficacy;
   (iii) increase in nucleic acid (DNA or RNA) in vivo half-life;
   (iv) increase in nucleic acid (DNA or RNA) in vitro half-life;
   (v) decrease in nucleic acid (DNA or RNA) in vivo half-life;
   (vi) decrease in nucleic acid (DNA or RNA) in vitro half-life
   (vii) increase in expressed protein yield;
   (viii) increase in expressed protein quality;
   (ix) increase in nucleic acid (DNA or RNA) structural stability;
   (x) increase in viability of cells expressing the optimized nucleic acid; and
   (xi) combinations thereof.
14. A computer implemented multiparametric codon optimization method comprising:
   (a) inputting at least one candidate nucleic acid sequence;
   (b) applying a multiparametric codon optimization method according to item 1 to the candidate nucleic acid sequence; and,
   (c) outputting at least one optimized nucleic acid sequence.
15. An isolated nucleic acid molecule encoding a protein optimized according to the method of item 1, or a complement thereof, wherein said nucleic acid molecule is a synthetic mRNA comprising at least one nucleoside selected from the group consisting of 2- pseudouridine, 5-methoxyuridine, 2-thiouridine, 4-thiouridine, N1-methylpseudouridine, 5-aza-uridine, 2-thio-5-aza-uridine, 4-thio-pseudouridine, 2-thio-pseudouridine, 5-hydroxyuridine, 4-methoxy-pseudouridine, 4-methoxy-2-thio-pseudouridine, 3-methyluridine, 5-carboxymethyl-uridine, 1-carboxymethyl-pseudouridine, 5-propynyl-uridine, 1-propynyl-pseudouridine, 2-methoxy-4-thio-uridine, 5-taurinomethyluridine, 1-taurinomethyl-pseudouridine, 5-taurinomethyl-2-thio-uridine, 1-taurinomethyl-4-thio-uridine, 5-methyl-uridine, 2-methoxyuridine, 1-methyl-pseudouridine, 4-thio-1-methyl-pseudouridine, 2-thio-1-methyl-pseudouridine, 1-methyl-1-deaza-pseudouridine, 2-thio-1-methyl-1-deaza-pseudouridine, and 2-thio-dihydrouridine.
16. An isolated nucleic acid molecule encoding a protein optimized according to the method of item 1, or a complement thereof, wherein said nucleic acid molecule is a synthetic mRNA comprising at least one nucleoside selected from the group consisting of 2-aminopurine, 2,6-diaminopurine, 7-deaza-adenine, 7-deaza-8-aza-adenine, 7-deaza-2-aminopurine, 7-deaza-8-aza-2-aminopurine, 7-deaza-2,6-diaminopurine, 7-deaza-8-aza-2,6-diaminopurine, 1-methyladenosine, N6-methyladenosine, N6-isopentenyladenosine, N6-(cis-hydroxyisopentenyl)adenosine, 2-methylthio-N6-(cis-hydroxyisopentenyl)adenosine, N6-glycinylcarbamoyladenosine, N6-threonylcarbamoyladenosine, 2-methylthio-N6-threonyl carbamoyladenosine, N6,N6-dimethyladenosine, and 7-methyladenine.
17. An isolated nucleic acid molecule encoding a protein optimized according to the method of item 1, or a complement thereof, wherein said nucleic acid molecule is a synthetic mRNA comprising at least one nucleoside selected from the group consisting of inosine, 1-methyl-inosine, wyosine, wybutosine, 7-deaza-guanosine, 7-deaza-8-aza-guanosine, 6-thio-guanosine, 6-thio-7-deaza-guanosine, 6-thio-7-deaza-8-aza-guanosine, 7-methyl-guanosine, 6-thio-7-methyl-guanosine, 7-methylinosine, 6-methoxy-guanosine, 1-methylguanosine, N2-methylguanosine, N2,N2-dimethylguanosine, 8-oxo-guanosine, 7-methyl-8-oxo-guanosine, and 1-methyl-6-thio-guanosine.
18. An isolated nucleic acid molecule encoding a protein optimized according to the method of item 1, or a complement thereof, wherein said nucleic acid molecule is a synthetic mRNA comprising at least one nucleoside selected from the group consisting of 5-methylcytidine, 5-aza-cytidine, pseudoisocytidine, 3-methyl-cytidine, N4-acetylcytidine, 5-formylcytidine, N4-methylcytidine, 5-hydroxymethylcytidine, 1-methyl-pseudoisocytidine, pyrrolo-cytidine, pyrrolo-pseudoisocytidine, 2-thio-cytidine, 2-thio-5-methyl-cytidine, 4-thio-pseudoisocytidine, 4-thio-1-methyl-pseudoisocytidine, 4-thio-1-methyl-1-deaza-pseudoisocytidine, 1-methyl-1-deaza-pseudoisocytidine, zebularine, 5-aza-zebularine, 5-methyl-zebularine, 5-aza-2-thio-zebularine, 2-thio-zebularine, 2-methoxy-cytidine, 2-methoxy-5-methyl-cytidine, and 4-methoxy-pseudoisocytidine.
19. The isolated nucleic acid molecule according to any one of items 15 to 18, wherein:
   (i) 25% of uridines have been replaced with 4-thiouridine;
   (ii) 50% of uridines have been replaced with 4-thiouridine;
   (iii) 100% of uridines have been replaced with 4-thiouridine;
   (iv) 25% of uridines have been replaced with 2-thiouridine (s2U) and 25% of cytidines have been replaced with 5-methylcytidine (m5C);
   (v) 50% of uridines have been replaced with 2-thiouridine (s2U);
   (vi) 100% of uridines have been replaced with pseudouridine (Ψ);
   (vii) 100% of uridines have been replaced with pseudouridine (Ψ) and 100% of cytidines have been replaced with 5-methylcytidine (5mC);
   (viii) 25% of uridines have been replaced with 5-methoxyuridine (5moU) and 50% of cytidines have been replaced with 5-methylcytidine (5mC);
   (ix) 25% of uridines have been replaced with 5-methoxyuridine (5moU) and 100% of cytidines have been replaced with 5-methylcytidine (5mC);
   (x) 100% of uridines have been replaced with 5-methoxyuridine (5moU);
   (xi) 100% of uridines have been replaced with 5-methoxyuridine (5moU) and 100% of cytidines have been replaced with 5-methylcytidine (5mC);
   (xii) 100% of uridines have been replaced with N1-methylpseudouridine (1mΨ); or,
   (xiii) 100% of uridines have been replaced with N1-methylpseudouridine (1mΨ) and of cytidines have been replaced with 100% 5-methylcytidine (5mC).

## Claims

1. A multiparametric method for optimizing a candidate nucleic acid sequence, the method comprising at least one optimization method selected from:
(i) modifying at least one subsequence in the candidate nucleic acid sequence to generate a ramp subsequence;
(ii) substituting at least one codon in the candidate nucleic acid sequence with an alternative codon to increase or decrease uridine content to generate a uridine-modified sequence;
(iii) substituting at least one codon in the candidate nucleic acid sequence or the uridine-modified sequence with a faster recharging codon;
(iv) substituting at least one codon in the candidate nucleic acid sequence with an alternative codon having a higher codon frequency in the synonymous codon set;
(v) substituting at least one natural nucleobase in the candidate nucleic acid sequence with an alternative synthetic nucleobase;
(vi) substituting at least one internucleoside linkage in the candidate nucleic acid sequence with a non-natural internucleoside linkage; and,
(vii) combinations thereof.
wherein the resulting optimized nucleic acid sequence has at least one optimized property with respect to the candidate nucleic acid sequence.

2. The multiparametric method according to claim 1, wherein the optimized nucleic acid sequence comprises at least one ramp subsequence.

3. The multiparametric method according to claim 1, wherein the codons in the optimized nucleic acid sequence are selected from an optimized codon set which is a limited codon set.

4. The multiparametric method according to claim 3, wherein the limited codon set consists of 20 codons, and wherein each codon encodes one of 20 amino acids.

5. The multiparametric method according to claim 4, wherein the limited codon set is:
(a) UUC, UUG, CUG, AUC, AUG, GUG, AGC, CCC, ACC, GCC, UAC, CAC, CAG, AAC, AAG, GAG, UGC, UGG, AGG, GGC;
(b) UUU, CUA, AUA, AUG, GUA, UCG, CCG, ACG, GCG, UAU, CAU, CAA, AAU, AAA, GAU, GAA, UGU, UGG, CGU, GGU;
(c) UUC, CUV, AUM, AUG, GUV, AGC, CCV, ACV, GCV, UAC, CAC, CAR, AAC, AAR, GAC, GAR, UGC, UGG, CGV, GGV; or,
(d) UUC, CUV, AUM, AUG, GUV, AGC, CCV, ACV, GCV, UAC, CAC, CAR, AAC, AAR, GAC, GAR, UGC, UGG, AGR, GGV.

6. The multiparametric method according to claim 1, wherein the uridine content (absolute or relative content) of the uridine-modified sequence is less than 50%, 49%, 48%, 47%, 46%, 45%, 44%, 43%, 42%, 41%, 40%, 39%, 38%, 37%, 36%, 35%, 34%, 33%, 32%, 31%, 30%, 29%, 28%, 27%, 26%, 25%, 24%, 23%, 22%, 21%, 20%, 19%, 18%, 17%, 16%, 15%, 14%, 13%, 12%, 11%, 10%, 9%, 8%, 7%, 6%, 5%, 4%, 3%, 2% or 1%.

7. The multiparametric method according to claim 1, wherein
(a) the optimized nucleic acid sequence comprises an overall increase in Guanine/Cytosine (G/C) content (absolute or relative) relative to the G/C content (absolute or relative) of the candidate nucleic acid sequence, or
(b) at least about 5%, at least about 10%, at least about 15%, at least about 20%, at least about 25%, at least about 30%, at least about 35%, at least about 40%, at least about 45%, at least about 50%, at least about 55%, at least about 60%, at least about 65%, at least about 70%, at least about 75%, at least about 80%, at least about 85%, at least about 90%, at least about 95%, at least about 99%, or 100% of the codons in the candidate nucleic acid sequence are substituted with alternative codons, each alternative codon having a codon frequency higher than the codon frequency of the substituted codon in the synonymous codon set; or
(c) at least about 5%, at least about 10%, at least about 15%, at least about 20%, at least about 25%, at least about 30%, at least about 35%, at least about 40%, at least about 45%, at least about 50%, at least about 55%, at least about 60%, at least about 65%, at least about 70%, at least about 75%, at least about 80%, at least about 85%, at least about 90%, at least about 95%, at least about 99%, or 100% of the codons in the candidate nucleic acid sequence are substituted with faster recharging codons.

8. The multiparametric method according claim 1, wherein the method comprises one, two, three, four, five, or six, optimization method selected from the group consisting of (i) modifying at least one subsequence in the candidate nucleic acid sequence to generate a ramp subsequence; (ii) substituting at least one codon in the candidate nucleic acid sequence with an alternative codon to increase or decrease uridine content to generate a uridine-modified sequence; (iii) substituting at least one codon in the candidate nucleic acid sequence or the uridine-modified sequence with a fast recharging codon; (iv) substituting at least one codon in the candidate nucleic acid sequence with an alternative codon having a higher codon frequency in the synonymous codon set; (v) substituting at least one natural nucleobase in the candidate nucleic acid sequence with an alternative synthetic nucleobase; and (vi) substituting at least one internucleoside linkage in the candidate nucleic acid sequence with a non-natural internucleoside linkage.

9. The multiparametric method according to claim 1, wherein at least 5%, at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 85%, at least 90%, at least 95%, at least 96%, at least 97%, at least 98%, at least 99% or 100% of the codons in the candidate nucleic acid sequence are replaced.

10. A method for expressing a protein in an *in vitro* translation system, the method comprising:
(a) obtaining an optimized gene sequence for expression in a human *in vivo* systemically or in a target tissue or target cell, using a method according to claim 1;
(b) synthesizing a nucleic acid molecule comprising the optimized gene sequence;
(c) combining the nucleic acid molecule with the *in vitro* translation system.

11. An optimized gene sequence obtained using a method according to claim 1, for use in a therapeutic method for expressing the sequence in a human *in vivo* systemically or in a target tissue or target cell, the method comprising:
(a) obtaining an optimized gene sequence for expression in a human *in vivo* systemically or in a target tissue or target cell, using a method according to claim 1;
(b) synthesizing a nucleic acid molecule comprising the optimized gene sequence;
(c) introducing the nucleic acid molecule into the human, the target tissue or target cell.

12. The method according to claim 1, wherein the at least one optimized property with respect to the candidate nucleic acid sequence is selected from:
(i) increase in transcription efficacy;
(ii) increase in translation efficacy;
(iii) increase in nucleic acid (DNA or RNA) in vivo half-life;
(iv) increase in nucleic acid (DNA or RNA) in vitro half-life;
(v) decrease in nucleic acid (DNA or RNA) in vivo half-life;
(vi) decrease in nucleic acid (DNA or RNA) in vitro half-life
(vii) increase in expressed protein yield;
(viii) increase in expressed protein quality;
(ix) increase in nucleic acid (DNA or RNA) structural stability;
(x) increase in viability of cells expressing the optimized nucleic acid; and
(xi) combinations thereof.

13. A computer implemented multiparametric codon optimization method comprising:
(a) inputting at least one candidate nucleic acid sequence;
(b) applying a multiparametric codon optimization method according to claim 1 to the candidate nucleic acid sequence; and,
(c) outputting at least one optimized nucleic acid sequence.

14. An isolated nucleic acid molecule encoding a protein optimized according to the method of claim 1, or a complement thereof, wherein said nucleic acid molecule is a synthetic mRNA comprising at least one nucleoside selected from the group consisting of
(a) 2- pseudouridine, 5-methoxyuridine, 2-thiouridine, 4-thiouridine, N1-methylpseudouridine, 5-aza-uridine, 2-thio-5-aza-uridine, 4-thio-pseudouridine, 2-thio-pseudouridine, 5-hydroxyuridine, 4-methoxy-pseudouridine, 4-methoxy-2-thio-pseudouridine, 3-methyluridine, 5-carboxymethyl-uridine, 1-carboxymethyl-pseudouridine, 5-propynyl-uridine, 1-propynyl-pseudouridine, 2-methoxy-4-thio-uridine, 5-taurinomethyluridine, 1-taurinomethyl-pseudouridine, 5-taurinomethyl-2-thio-uridine, 1-taurinomethyl-4-thio-uridine, 5-methyl-uridine, 2-methoxyuridine, 1-methyl-pseudouridine, 4-thio-1-methyl-pseudouridine, 2-thio-1-methyl-pseudouridine, 1-methyl-1-deaza-pseudouridine, 2-thio-1-methyl-1-deaza-pseudouridine, and 2-thio-dihydrouridine;
(b) 2-aminopurine, 2,6-diaminopurine, 7-deaza-adenine, 7-deaza-8-aza-adenine, 7-deaza-2-aminopurine, 7-deaza-8-aza-2-aminopurine, 7-deaza-2,6-diaminopurine, 7-deaza-8-aza-2,6-diaminopurine, 1-methyladenosine, N6-methyladenosine, N6-isopentenyladenosine, N6-(cis-hydroxyisopentenyl)adenosine, 2-methylthio-N6-(cis-hydroxyisopentenyl)adenosine, N6-glycinylcarbamoyladenosine, N6-threonylcarbamoyladenosine, 2-methylthio-N6-threonyl carbamoyladenosine, N6,N6-dimethyladenosine, and 7-methyladenine;
(c) inosine, 1-methyl-inosine, wyosine, wybutosine, 7-deaza-guanosine, 7-deaza-8-aza-guanosine, 6-thio-guanosine, 6-thio-7-deaza-guanosine, 6-thio-7-deaza-8-aza-guanosine, 7-methyl-guanosine, 6-thio-7-methyl-guanosine, 7-methylinosine, 6-methoxy-guanosine, 1-methylguanosine, N2-methylguanosine, N2,N2-dimethylguanosine, 8-oxo-guanosine, 7-methyl-8-oxo-guanosine, and 1-methyl-6-thio-guanosine;
(d) 5-methylcytidine, 5-aza-cytidine, pseudoisocytidine, 3-methyl-cytidine, N4-acetylcytidine, 5-formylcytidine, N4-methylcytidine, 5-hydroxymethylcytidine, 1-methyl-pseudoisocytidine, pyrrolo-cytidine, pyrrolo-pseudoisocytidine, 2-thio-cytidine, 2-thio-5-methyl-cytidine, 4-thio-pseudoisocytidine, 4-thio-1-methyl-pseudoisocytidine, 4-thio-1-methyl-1-deaza-pseudoisocytidine, 1-methyl-1-deaza-pseudoisocytidine, zebularine, 5-aza-zebularine, 5-methyl-zebularine, 5-aza-2-thio-zebularine, 2-thio-zebularine, 2-methoxy-cytidine, 2-methoxy-5-methyl-cytidine, and 4-methoxy-pseudoisocytidine.

15. The isolated nucleic acid molecule according to claim 14, wherein:
(i) 25% of uridines have been replaced with 4-thiouridine;
(ii) 50% of uridines have been replaced with 4-thiouridine;
(iii) 100% of uridines have been replaced with 4-thiouridine;
(iv) 25% of uridines have been replaced with 2-thiouridine (s2U) and 25% of cytidines have been replaced with 5-methylcytidine (m5C);
(v) 50% of uridines have been replaced with 2-thiouridine (s2U);
(vi) 100% of uridines have been replaced with pseudouridine (Ψ);
(vii) 100% of uridines have been replaced with pseudouridine (Ψ) and 100% of cytidines have been replaced with 5-methylcytidine (5mC);
(viii) 25% of uridines have been replaced with 5-methoxyuridine (5moU) and 50% of cytidines have been replaced with 5-methylcytidine (5mC);
(ix) 25% of uridines have been replaced with 5-methoxyuridine (5moU) and 100% of cytidines have been replaced with 5-methylcytidine (5mC);
(x) 100% of uridines have been replaced with 5-methoxyuridine (5moU);
(xi) 100% of uridines have been replaced with 5-methoxyuridine (5moU) and 100% of cytidines have been replaced with 5-methylcytidine (5mC);
(xii) 100% of uridines have been replaced with N1-methylpseudouridine (1mΨ); or,
(xiii) 100% of uridines have been replaced with N1-methylpseudouridine (1mΨ) and of cytidines have been replaced with 100% 5-methylcytidine (5mC).
